(19) Europäisches Patentamt
European Patent Office
Office européen des brevets

(11) **EP 4 596 567 A1**

(12) **EUROPEAN PATENT APPLICATION**
published in accordance with Art. 153(4) EPC

(43) Date of publication:
**06.08.2025 Bulletin 2025/32**

(21) Application number: **23885777.5**

(22) Date of filing: **31.10.2023**

(51) International Patent Classification (IPC):
***C07K 1/06*** *(2006.01)*

(52) Cooperative Patent Classification (CPC):
**C07K 1/06**

(86) International application number:
**PCT/JP2023/039324**

(87) International publication number:
**WO 2024/096023 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.11.2022 JP 2022175592**

(71) Applicant: **CHUGAI SEIYAKU KABUSHIKI KAISHA**
**Tokyo 115-8543 (JP)**

(72) Inventors:
- **SERIZAWA Hiroki**
  **Tokyo 115-8543 (JP)**
- **KOMIYA Shio**
  **Tokyo 115-8543 (JP)**

(74) Representative: **Vossius & Partner Patentanwälte Rechtsanwälte mbB**
**Siebertstrasse 3**
**81675 München (DE)**

# (54) METHOD FOR REMOVING DIBENZOFULVENE OR DIBENZOFULVENE DERIVATIVE

(57) The present inventors have found that dibenzofulvene or a dibenzofulvene derivative can be captured and removed without regeneration by using, as a capture agent, a sulfite ion or a bisulfite ion or a compound that produces these ions. The present inventors have also found a novel method for deprotecting a protecting group having a Fmoc skeleton and a novel method for producing an amino group-containing compound such as a peptide.

EP 4 596 567 A1

## Description

Technical Field

The present invention relates to a method for removing dibenzofulvene (DBF) or a dibenzofulvene derivative.

Background Art

Fmoc (9-fluorenylmethoxycarbonyl) groups are widely used in peptide synthesis as a protecting group for amino groups of amino acids and peptides. Peptide synthesis employs solid phase synthesis and liquid phase synthesis, and liquid phase synthesis is often used for mass production of pharmaceuticals or the like. The Fmoc group can be easily deprotected under basic conditions, and thus it is also used as a protective group of the amino group in liquid phase synthesis.

However, in the deprotecting step of the Fmoc group, dibenzofulvene or a compound in which an amine used for deprotecting is added to dibenzofulvene (hereinafter also referred to as "amine adduct") is produced as a by-product. Continuing peptide synthesis while the byproduct remains may cause a side reaction such as 9-fluorenylmethylation. Thus, dibenzofulvene or the amine adduct must be removed in the deprotecting step.

As the means for removing dibenzofulvene, Non Patent Literature 1 reports a means of converting dibenzofulvene into an amine adduct or a compound in which a thiol is added to dibenzofulvene (hereinafter also referred to as a "thiol adduct") using an amine or a thiol as a capture agent, and removing it by trituration, and Patent Literature 1 reports a means of removing dibenzofulvene by liquid separation using a hydrocarbon solvent and a polar organic solvent. Patent Literature 2 also describes a means of removing an amine adduct as a carbonate by contacting carbon dioxide with a reaction mixture containing the amine adduct. Furthermore, Patent Literatures 3 and 4 report a means of converting dibenzofulvene to a thiol adduct using thiocarboxylic acid, thiosulfonic acid, or thiophosphonic acid as a capture agent and then removing it by washing with a basic aqueous solution.

Citation List

Patent Literature

Patent Literature 1: International Publication No. WO 2009/014177
Patent Literature 2: International Publication No. WO 2010/016551
Patent Literature 3: International Publication No. WO 2013/089241
Patent Literature 4: Japanese Patent No. 7063409

Non Patent Literature

Non Patent Literature 1: J. E. Sheppeck II, et al. Tetrahedron Lett. 2000, No.41, vol.28, 5329-5333

Summary of Invention

Technical Problem

However, in the methods described in each of the above literatures, there have been possibilities that dibenzofulvene or a dibenzofulvene derivative are not sufficiently removed, or that captured dibenzofulvene or a dibenzofulvene derivative are not completely removed due to regeneration during removal. Accordingly, an object of the present invention is to provide a new method for removing dibenzofulvene or a dibenzofulvene derivative, the method being capable of capturing dibenzofulvene or a dibenzofulvene derivative and removing dibenzofulvene or a dibenzofulvene derivative without regeneration.

Solution to Problem

The present inventors have investigated a new method for removing dibenzofulvene or a dibenzofulvene derivative, and have found that dibenzofulvene or a dibenzofulvene derivative can be captured and removed without regeneration by using, as a capture agent, a sulfite ion or a bisulfite ion or a compound that produces these ions, and have completed the present invention. The present invention can be applied to a wide variety of synthetic processes, in

particular, the synthesis of amino group-containing compounds, for example, the synthesis of peptides. For example, in some Examples, the present invention also provides a novel method for deprotecting a protecting group having a Fmoc skeleton. In some specific examples, the present invention provides a novel method for producing an amino group-containing compound such as a peptide.

That is, the present invention includes the following.

[1] A method for removing dibenzofulvene or a dibenzofulvene derivative, comprising step (1) of mixing the following (i) and (ii):

(i) dibenzofulvene or a dibenzofulvene derivative;
(ii) a sulfite ion or a bisulfite ion or a compound that produces a sulfite ion or a bisulfite ion.

[1-1] A method for removing dibenzofulvene or a dibenzofulvene derivative, comprising the following step (1), (1’), or (1"):

(1) mixing the following (i) and (ii):

(i) dibenzofulvene or a dibenzofulvene derivative;
(ii) a sulfite ion or a bisulfite ion or a compound that produces a sulfite ion or a bisulfite ion,

(1’) mixing the following (i) and (ii):

(i) dibenzofulvene or a dibenzofulvene derivative;
(ii) at least one selected from the group consisting of bisulfite, disulfurous acid and a salt thereof, sulfurous acid and a salt thereof, dithionous acid and a salt thereof, and a solvate of these,

(1") forming (9H-fluoren-9-yl)methanesulfonic acid or a salt thereof or a (9H-fluoren-9-yl)methanesulfonic acid derivative or a salt thereof.

[2] A method for removing dibenzofulvene or a dibenzofulvene derivative, comprising step (1’) of mixing the following (i) and (ii):

(i) dibenzofulvene or a dibenzofulvene derivative;
(ii) at least one selected from the group consisting of bisulfite, disulfurous acid and a salt thereof, sulfurous acid and a salt thereof, dithionous acid and a salt thereof, and a solvate of these.

[3] A method for removing dibenzofulvene or a dibenzofulvene derivative, comprising step (1") of forming (9H-fluoren-9-yl)methanesulfonic acid or a salt thereof or a (9H-fluoren-9-yl)methanesulfonic acid derivative or a salt thereof.
[4] The method according to [1] or [2], wherein, in the step (1) or (1’), (9H-fluoren-9-yl)methanesulfonic acid or a salt thereof or a (9H-fluoren-9-yl)methanesulfonic acid derivative or a salt thereof is formed.
[4-1] A method for removing dibenzofulvene or a dibenzofulvene derivative from a mixture containing the dibenzofulvene or the dibenzofulvene derivative, the method comprising step (1‴) of mixing (i) dibenzofulvene or a dibenzofulvene derivative and (ii) a sulfite ion or a bisulfite ion or a compound that produces a sulfite ion or a bisulfite ion to form (9H-fluoren-9-yl)methanesulfonic acid or a salt thereof or a (9H-fluoren-9-yl)methanesulfonic acid derivative or a salt thereof.
[4-2] A method for removing dibenzofulvene or a dibenzofulvene derivative from a mixture containing the dibenzofulvene or the dibenzofulvene derivative, the method comprising step (1"") of mixing (i) dibenzofulvene or a dibenzofulvene derivative and (ii) at least one selected from the group consisting of bisulfite, disulfurous acid and a salt thereof, sulfurous acid and a salt thereof, dithionous acid and a salt thereof, and a solvate of these to form (9H-fluoren-9-yl)methanesulfonic acid or a salt thereof or a (9H-fluoren-9-yl)methanesulfonic acid derivative or a salt thereof.
[5] The method according to any of [1] to [4], wherein dibenzofulvene or a dibenzofulvene derivative is removed from a mixture containing the dibenzofulvene or the dibenzofulvene derivative.
[6] The method according to any of [1] to [5], comprising, before the step (1), (1’), (1"), (1"’) or (1″″), step (2) of mixing a deprotecting agent with a first amino group-containing compound in which an amino group is protected by a protecting group having a Fmoc skeleton.
[6-1] The method according to any of [1] to [5], comprising, before the step (1), (1’), (1"), (1‴) or (1″″), step (2) of mixing a

first amino group-containing compound in which an amino group is protected by a protecting group having a Fmoc skeleton with a deprotecting agent capable of deprotecting a protecting group having a Fmoc skeleton.
[7] The method according to [6], wherein dibenzofulvene or a dibenzofulvene derivative generated by the step (2) is removed from a mixture of the step (2).
[8] A method for removing dibenzofulvene or a dibenzofulvene derivative, comprising step (3) of mixing the following (i) to (iii):

(i) a first amino group-containing compound in which an amino group is protected by a protecting group having a Fmoc skeleton;
(ii) a deprotecting agent;
(iii) a sulfite ion or a bisulfite ion or a compound that produces a sulfite ion or a bisulfite ion.

[8-1] A method for removing dibenzofulvene or a dibenzofulvene derivative, comprising step (3) of mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) a sulfite ion or a bisulfite ion or a compound that produces a sulfite ion or a bisulfite ion.

[B1] A method for deprotecting a protecting group having a Fmoc skeleton, comprising step (3) of mixing (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) a sulfite ion or a bisulfite ion or a compound that produces a sulfite ion or a bisulfite ion.

[9] A method for removing dibenzofulvene or a dibenzofulvene derivative, comprising step (3’) of mixing the following (i) to (iii):

(i) a first amino group-containing compound in which an amino group is protected by a protecting group having a Fmoc skeleton;
(ii) a deprotecting agent;
(iii) at least one selected from the group consisting of bisulfite, disulfurous acid and a salt thereof, sulfurous acid and a salt thereof, dithionous acid and a salt thereof, and a solvate of these.

[9-1] A method for removing dibenzofulvene or a dibenzofulvene derivative, comprising step (3’) of mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) at least one selected from the group consisting of bisulfite, disulfurous acid and a salt thereof, sulfurous acid and a salt thereof, dithionous acid and a salt thereof, and a solvate of these.

[B1-1] A method for deprotecting a protecting group having a Fmoc skeleton, comprising step (3’) of mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) at least one selected from the group consisting of bisulfite, disulfurous acid and a salt thereof, sulfurous acid and a salt thereof, dithionous acid and a salt thereof, and a solvate of these.

[9-2] A method for removing dibenzofulvene or a dibenzofulvene derivative, comprising the following step (3) or (3’):

(3) mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) a sulfite ion or a bisulfite ion or a compound that produces a sulfite ion or a bisulfite ion;

(3') mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) at least one selected from the group consisting of bisulfite, disulfurous acid and a salt thereof, sulfurous acid and a salt thereof, dithionous acid and a salt thereof, and a solvate of these.

[B1-2] A method for deprotecting a protecting group having a Fmoc skeleton, comprising the following step (3) or (3'):

(3) mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) a sulfite ion or a bisulfite ion or a compound that produces a sulfite ion or a bisulfite ion;

(3') mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) at least one selected from the group consisting of bisulfite, disulfurous acid and a salt thereof, sulfurous acid and a salt thereof, dithionous acid and a salt thereof, and a solvate of these.

[10] The method according to [8], [9] or [B1], wherein, in the step (3) or (3'), (9H-fluoren-9-yl)methanesulfonic acid or a salt thereof or a (9H-fluoren-9-yl)methanesulfonic acid derivative or a salt thereof is formed.
[10-1] A method for removing dibenzofulvene or a dibenzofulvene derivative from a mixture containing the dibenzofulvene or the dibenzofulvene derivative, the method comprising step (3) of mixing the following (i) to (iii) to form (9H-fluoren-9-yl)methanesulfonic acid or a salt thereof or a (9H-fluoren-9-yl)methanesulfonic acid derivative or a salt thereof:

(i) a first amino group-containing compound in which an amino group is protected by a protecting group having a Fmoc skeleton,
(ii) a deprotecting agent capable of deprotecting a protecting group having a Fmoc skeleton,
(iii) a sulfite ion or a bisulfite ion or a compound that produces a sulfite ion or a bisulfite ion.

[10-2] A method for removing dibenzofulvene or a dibenzofulvene derivative from a mixture containing the dibenzofulvene or the dibenzofulvene derivative, the method comprising step (3') of mixing the following (i) to (iii) to form (9H-fluoren-9-yl)methanesulfonic acid or a salt thereof or a (9H-fluoren-9-yl)methanesulfonic acid derivative or a salt thereof:

(i) a first amino group-containing compound in which an amino group is protected by a protecting group having a Fmoc skeleton,
(ii) a deprotecting agent capable of deprotecting a protecting group having a Fmoc skeleton,
(iii) at least one selected from the group consisting of bisulfite, disulfurous acid and a salt thereof, sulfurous acid and a salt thereof, dithionous acid and a salt thereof, and a solvate of these.

[10-3] A method for removing dibenzofulvene or a dibenzofulvene derivative from a mixture containing the dibenzofulvene or the dibenzofulvene derivative, the method comprising the following step (3) or (3'):

(3) mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) a sulfite ion or a bisulfite ion or a compound that produces a sulfite ion or a bisulfite ion;

(3') mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) at least one selected from the group consisting of bisulfite, disulfurous acid and a salt thereof, sulfurous acid and a salt thereof, dithionous acid and a salt thereof, and a solvate of these.

[B1-3] A method for deprotecting a protecting group having a Fmoc skeleton, comprising step (3) of mixing the following (i) to (iii) to form (9H-fluoren-9-yl)methanesulfonic acid or a salt thereof or a (9H-fluoren-9-yl)methanesulfonic acid derivative or a salt thereof:

(i) a first amino group-containing compound in which an amino group is protected by a protecting group having a Fmoc skeleton,
(ii) a deprotecting agent capable of deprotecting a protecting group having a Fmoc skeleton,
(iii) a sulfite ion or a bisulfite ion or a compound that produces a sulfite ion or a bisulfite ion.

[B1-4] A method for deprotecting a protecting group having a Fmoc skeleton, comprising step (3') of mixing the following (i) to (iii) to form (9H-fluoren-9-yl)methanesulfonic acid or a salt thereof or a (9H-fluoren-9-yl)methanesulfonic acid derivative or a salt thereof:

(i) a first amino group-containing compound in which an amino group is protected by a protecting group having a Fmoc skeleton,
(ii) a deprotecting agent capable of deprotecting a protecting group having a Fmoc skeleton,
(iii) at least one selected from the group consisting of bisulfite, disulfurous acid and a salt thereof, sulfurous acid and a salt thereof, dithionous acid and a salt thereof, and a solvate of these.

[B1-5] A method for deprotecting a protecting group having a Fmoc skeleton, comprising the following step (3) or (3'):

(3) mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) a sulfite ion or a bisulfite ion or a compound that produces a sulfite ion or a bisulfite ion,

(3') mixing the following (i) to (iii):

(i) a first amino group-containing compound in which an amino group is protected by a protecting group having a Fmoc skeleton,
(ii) a deprotecting agent capable of deprotecting a protecting group having a Fmoc skeleton,
(iii) at least one selected from the group consisting of bisulfite, disulfurous acid and a salt thereof, sulfurous acid and a salt thereof, dithionous acid and a salt thereof, and a solvate of these.

[11] The method according to any of [8] to [10] or [B1], wherein dibenzofulvene or a dibenzofulvene derivative is removed from a mixture containing the dibenzofulvene or the dibenzofulvene derivative.
[12] The method according to any of [8] to [11] or [B1], wherein dibenzofulvene or a dibenzofulvene derivative generated by the step of mixing the deprotecting agent with the first amino group-containing compound in which an amino group is protected by a protecting group having a Fmoc skeleton is removed from a mixture of the step (3) or (3').
[12-1] The method according to any of [8] to [11] or [B1], wherein dibenzofulvene or a dibenzofulvene derivative generated by the step of mixing a first amino group-containing compound in which an amino group is protected by a protecting group having a Fmoc skeleton with a deprotecting agent capable of deprotecting a protecting group having

a Fmoc skeleton is removed from a mixture of the step (3) or (3').

[13] The method according to any of [1] to [12] or [B1], further comprising step (4) of washing a mixture after one or more of the steps (1), (1'), (1"), (1‴), (1″″), (3) or (3') with a washing solution.

[14] The method according to [13], wherein, in the step (4), (9H-fluoren-9-yl)methanesulfonic acid or a salt thereof or a (9H-fluoren-9-yl)methanesulfonic acid derivative or a salt thereof is removed.

[15] The method according to any of [1] to [14] or [B1], wherein the dibenzofulvene or the dibenzofulvene derivative is a compound represented by the following formula (1):

[Formula 1]

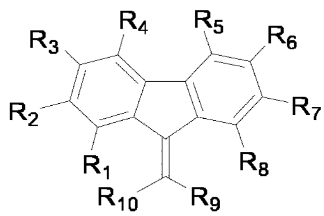

(1)

wherein,

$R_1$ to $R_8$ are independently selected from the group consisting of hydrogen, $C_1$-$C_8$ alkyl, $C_1$-$C_8$ fluoroalkyl, halogen, sulfo, and trimethylsilyl; and $R_9$ to $R_{10}$ are independently hydrogen or methyl.

[15-1] The method according to any of [1] to [14] or [B1], wherein the dibenzofulvene derivative is a compound represented by the following formula (1):

[Formula 2]

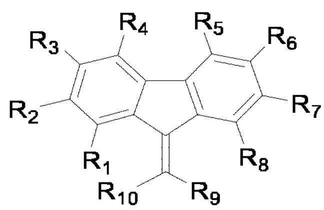

(1)

wherein,

$R_1$ to $R_8$ are independently selected from the group consisting of hydrogen, $C_1$-$C_8$ alkyl, $C_1$-$C_8$ fluoroalkyl, halogen, sulfo, and trimethylsilyl; $R_9$ to $R_{10}$ are independently hydrogen or methyl; and only any two of $R_1$ to $R_8$ are not hydrogen.

[16] The method according to any of [1] to [15] or [B1], wherein the dibenzofulvene or the dibenzofulvene derivative is dibenzofulvene.

[17] The method according to any of [1-1], [3] to [16] or [B1], wherein the (9H-fluoren-9-yl)methanesulfonic acid or a salt thereof or the (9H-fluoren-9-yl)methanesulfonic acid derivative or a salt thereof is a compound represented by the following formula (2) or a salt thereof, or a compound represented by the following formula (3):

[Formula 3]

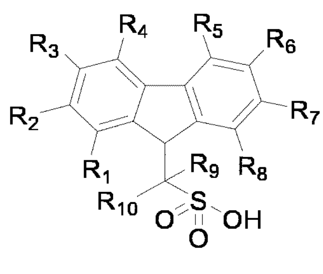

(

wherein,

$R_1$ to $R_8$ are independently selected from the group consisting of hydrogen, $C_1$-$C_8$ alkyl, $C_1$-$C_8$ fluoroalkyl, halogen,

sulfo, and trimethylsilyl; and $R_9$ to $R_{10}$ are independently hydrogen or methyl.

[Formula 4]

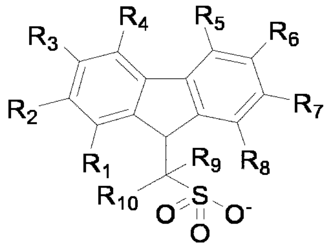

(3)

wherein,
$R_1$ to $R_8$ are independently selected from the group consisting of hydrogen, $C_1$-$C_8$ alkyl, $C_1$-$C_8$ fluoroalkyl, halogen, sulfo, and trimethylsilyl; and $R_9$ to $R_{10}$ are independently hydrogen or methyl.

[17-1] The method according to any of [1-1], [3] to [16], or [B1], wherein the (9H-fluoren-9-yl)methanesulfonic acid derivative or a salt thereof is a compound represented by the following formula (2) or a salt thereof, or a compound represented by the following formula (3):

[Formula 5]

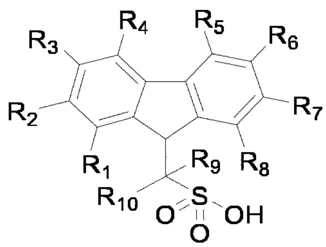

(2)

wherein,
$R_1$ to $R_8$ are independently selected from the group consisting of hydrogen, $C_1$-$C_8$ alkyl, $C_1$-$C_8$ fluoroalkyl, halogen, sulfo, and trimethylsilyl; $R_9$ to $R_{10}$ are independently hydrogen or methyl; and only any two of $R_1$ to $R_8$ are not hydrogen.

[Formula 6]

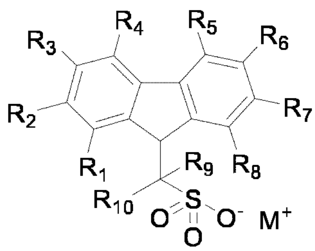

$M^+$: Counter Cation (3)

wherein,
$R_1$ to $R_8$ are independently selected from the group consisting of hydrogen, $C_1$-$C_8$ alkyl, $C_1$-$C_8$ fluoroalkyl, halogen, sulfo, and trimethylsilyl; $R_9$ to $R_{10}$ are independently hydrogen or methyl; and only any two of $R_1$ to $R_8$ are not hydrogen.

[18] The method according to any of [6] to [17] or [B1], wherein the protecting group having a Fmoc skeleton is a compound represented by the following formula (4):

[Formula 7]

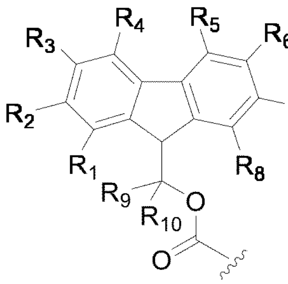

(4)

wherein,

$R_1$ to $R_8$ are independently selected from the group consisting of hydrogen, $C_1$-$C_8$ alkyl, $C_1$-$C_8$ fluoroalkyl, halogen, sulfo, and trimethylsilyl; and $R_9$ to $R_{10}$ are independently hydrogen or methyl. The wavy line represents a point of attachment to an amino group.

[18-1] The method according to any of [6] to [17] or [B1], wherein the protecting group having a Fmoc skeleton is a compound represented by the following formula (4):

[Formula 8]

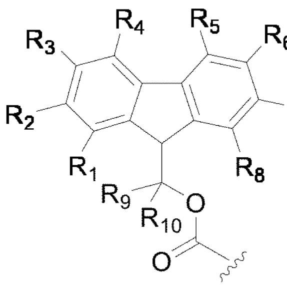

(4)

wherein,

$R_1$ to $R_8$ are independently selected from the group consisting of hydrogen, $C_1$-$C_8$ alkyl, $C_1$-$C_8$ fluoroalkyl, halogen, sulfo, and trimethylsilyl; $R_9$ to $R_{10}$ are independently hydrogen or methyl; and only any two of $R_1$ to $R_8$ are not hydrogen. The wavy line represents a point of attachment to an amino group.

[19] The method according to any of [6] to [17] or [B1], wherein the protective group having the Fmoc skeleton is a 9-fluorenylmethyloxycarbonyl (Fmoc) group, a 2,7-di-tert-butyl-Fmoc (Fmoc (2,7tb)) group, a 1-methyl-Fmoc (Fmoc (1Me)) group, a 2-fluoro-Fmoc (Fmoc (2F)) group, a 2,7-dibromo-Fmoc (Fmoc (2,7Br)) group, a 2-monoisooctyl-Fmoc (mio-Fmoc) group, a 2,7-diisooctyl-Fmoc (dio-Fmoc) group, a 2,7-(3,3,4,4,5,5,6,6,7,7,8,8,8-tridecafluorooctyl)-Fmoc (tdf-Fmoc) group, a 2,7-bis(trimethylsilyl)-Fmoc (Fmoc (2TMS)) group, a (2-sulfo-9H-fluoren-9-yl)methoxycarbonyl group (Fmoc (2so3h)), a [(1S)-1-(9H-fluoren-9-yl)ethoxy]carbonyl group (sm-Fmoc), or a [(1R)-1-(9H-fluoren-9-yl)ethoxy]carbonyl group (rm-Fmoc).

[20] The method according to any of [6] to [19] or [B1], wherein the protective group having the Fmoc skeleton is a 9-fluorenylmethyloxycarbonyl group.

[21] The method according to any of [6] to [20] or [B1], wherein the first amino group-containing compound is a peptide, an amino acid, or an amino acid amide.

[21-1] The method according to [21], wherein the first amino group-containing compound is a peptide or an amino acid.

[22] The method according to [21], wherein the first amino group-containing compound is a peptide.

[22-1] The method according to [21], wherein the first amino group-containing compound is an amino acid.

[23] The method according to any of [1] and [4] to [22], or [B1] wherein the sulfite ion or the bisulfite ion or the compound that produces a sulfite ion or a bisulfite ion captures dibenzofulvene or a dibenzofulvene derivative.

[24] The method according to any of [1] and [4] to [23], or [B1] wherein the sulfite ion or the bisulfite ion or the compound that produces a sulfite ion or a bisulfite ion is a sulfite ion or a bisulfite ion.

[25] The method according to any of [1] and [4] to [23] or [B1], wherein the sulfite ion or the bisulfite ion or the compound that produces a sulfite ion or a bisulfite ion is a compound that produces a sulfite ion or a bisulfite ion.

[26] The method according to [25], wherein the compound that produces a sulfite ion or a bisulfite ion is at least one selected from the group consisting of bisulfite, disulfurous acid and a salt thereof, sulfurous acid and a salt thereof, dithionous acid and a salt thereof, and a solvate of these.

[26-1] The method according to any of [1], [4] to [23] or [B1], wherein the sulfite ion or the bisulfite ion or the compound that produces a sulfite ion or a bisulfite ion is at least one selected from the group consisting of bisulfite, disulfurous acid and a salt thereof, sulfurous acid and a salt thereof, dithionous acid and a salt thereof, and a solvate of these.
[27] The method according to [25], wherein the compound that produces a sulfite ion or a bisulfite ion is at least one selected from the group consisting of bisulfite, disulfite, sulfite, dithionite, and a solvate of these.
[27-1] The method according to any of [1], [4] to [23] or [B1], wherein the sulfite ion or the bisulfite ion or the compound that produces a sulfite ion or a bisulfite ion is at least one selected from the group consisting of bisulfite, disulfite, sulfite, dithionite, and a solvate of these.
[28] The method according to [25], wherein the compound that produces a sulfite ion or a bisulfite ion is at least one selected from the group consisting of bisulfite, disulfite, sulfite, dithionite, and a solvate of these, and wherein the bisulfite, the disulfite, the sulfite, and the dithionite are a salt with at least one selected from the group consisting of alkali metal, alkaline earth metal, and ammonium.
[28-1] The method according to any of [1], [4] to [23] or [B1], wherein the sulfite ion or the bisulfite ion or the compound that produces a sulfite ion or a bisulfite ion is at least one selected from the group consisting of bisulfite, disulfite, sulfite, dithionite, and a solvate of these, and wherein the bisulfite, the disulfite, the sulfite, and the dithionite are a salt with at least one selected from the group consisting of alkali metal, alkaline earth metal, and ammonium.
[29] The method according to [25], wherein the compound that produces a sulfite ion or a bisulfite ion is at least one selected from the group consisting of bisulfite, disulfite, sulfite, dithionite, and a solvate of these, and wherein the bisulfite, the disulfite, the sulfite, and the dithionite are a salt with at least one selected from the group consisting of sodium, potassium, calcium, and ammonium.
[29-1] The method according to any of [1], [4] to [23] or [B1], wherein the sulfite ion or the bisulfite ion or the compound that produces a sulfite ion or a bisulfite ion is at least one selected from the group consisting of bisulfite, disulfite, sulfite, dithionite, and a solvate of these, and wherein the bisulfite, the disulfite, the sulfite, and the dithionite are a salt with at least one selected from the group consisting of sodium, potassium, calcium, and ammonium.
[30-1] The method according to [25], wherein the compound that produces a sulfite ion or a bisulfite ion is at least one selected from the group consisting of sodium bisulfite, sodium metabisulfite, potassium bisulfite, sodium dithionite, ammonium sulfite monohydrate, calcium sulfite hemihydrate, sodium sulfite, and potassium sulfite.
[30-2] The method according to any of [1], [4] to [23] or [B1], wherein the sulfite ion or the bisulfite ion or the compound that produces a sulfite ion or a bisulfite ion is at least one selected from the group consisting of sodium bisulfite, sodium metabisulfite, potassium bisulfite, sodium dithionite, ammonium sulfite monohydrate, calcium sulfite hemihydrate, sodium sulfite, and potassium sulfite.
[31] The method according to [25], wherein the compound that produces a sulfite ion or a bisulfite ion is at least one selected from the group consisting of sodium bisulfite, potassium bisulfite, and sodium dithionite.
[31-1] The method according to any of [1], [4] to [23] or [B1], wherein the sulfite ion or the bisulfite ion or the compound that produces a sulfite ion or a bisulfite ion is at least one selected from the group consisting of sodium bisulfite, potassium bisulfite, and sodium dithionite.
[32] The method according to any of [1] to [31] or [B1], wherein an additive is further mixed in the step (1), (1'), (1"), (1‴), (1⁗), (3), or (3').
[33] The method according to [32], wherein the additive is a first base.
[34] The method according to [33], wherein the first base is a tertiary amine.
[35] The method according to [34], wherein the tertiary amine is at least one selected from the group consisting of triethylamine, N,N-diisopropylethylamine, and 2,6-lutidine.
[36] The method according to any of [1] to [35], wherein water is further mixed in the step (1), (1'), (1"), (1‴), (1⁗), (3), or (3').
[36-1] The method according to any of [1] to [35], wherein water is not further mixed in the step (1), (1'), (1"), (1‴), (1⁗), (3), or (3').
[36-2] The method according to [36], wherein the water is not water contained in an organic solvent.
[37] The method according to any of [6] to [36] or [B1], wherein the deprotecting agent is a second base.
[38] The method according to [37], wherein the second base is at least one selected from the group consisting of an organic base having an amidine skeleton, a primary amine, a secondary amine, a tertiary amine, and an inorganic base. (Provided that none of the primary amine, the secondary amine, and the tertiary amine has an amidine skeleton in its molecule.)
[39] The method according to [38], wherein the second base is an organic base having an amidine skeleton.
[40] The method according to [39], wherein the organic base having the amidine skeleton is at least one selected from the group consisting of 1,8-diazabicyclo[5.4.0]undec-7-ene (DBU), 1,5-diazabicyclo[4.3.0]-5-nonen (DBN), and 7-methyl-1,5,7-triazabicyclo[4.4.0]dec-5-ene (MTBD).
[41] The method according to [39], wherein the organic base having the amidine skeleton is 1,8-diazabicyclo[5.4.0]undec-7-ene (DBU).

[42] The method according to [37], wherein the second base is a primary amine.
[43] The method according to [42], wherein the primary amine is propane-1-amine.
[44] The method according to [37], wherein the second base is a secondary amine.
[45] The method according to [44], wherein the secondary amine is at least one selected from the group consisting of morpholine, diethylamine, dicyclohexylamine, 1,1,1,3,3,3-hexamethyldisilazane, piperidine, pyrrolidine, and piperazine.
[46] The method according to [44], wherein the secondary amine is at least one selected from the group consisting of morpholine, diethylamine, dicyclohexylamine, and 1,1,1,3,3,3-hexamethyldisilazane.
[47] The method according to [37], wherein the second base is a tertiary amine.
[48] The method according to [47], wherein the tertiary amine is triethylamine.
[49] The method according to [37], wherein the second base is an inorganic base.
[50] The method according to [49], wherein the inorganic base is at least one selected from the group consisting of a carbonate and a metal alkoxide.
[51] The method according to [49], wherein the inorganic base is at least one selected from the group consisting of sodium carbonate and potassium tert-butoxide.
[52] The method according to any of [6] to [51] or [B1], wherein one or more of the steps (1), (1'), (1"), (1‴), (1⁗), (2), (3), or (3') is carried out in the presence of a solvent containing at least one selected from the group consisting of a nitrile-based solvent, an amide-based solvent, an sulfoxide-based solvent, an alcohol-based solvent, and an ether-based solvent.
[53] The method according to any of [6] to [51] or [B1], wherein one or more of the steps (1), (1'), (1"), (1‴), (1⁗), (2), (3), or (3') is carried out in the presence of a solvent containing at least one selected from the group consisting of acetonitrile, propionitrile, dimethylformamide, dimethylacetamide, N-methyl-2-pyrrolidone, 1,3-dimethyl-2-imidazolidinone, dimethyl sulfoxide, methanol, ethanol, n-propanol, 2-propanol, tetrahydrofuran, 2-methyltetrahydrofuran, diethyl ether, cyclopentylmethyl ether, and t-butylmethyl ether.
[54] The method according to any of [6] to [51] or [B1], wherein one or more of the steps (1), (1'), (1"), (1‴), (1⁗), and (2) is carried out in the presence of a solvent containing at least one selected from the group consisting of acetonitrile, dimethylacetamide, 2-methyltetrahydrofuran, and methanol.
[54-1] The method according to any of [6] to [51] or [B1], wherein one or more steps of the step (1), (1'), (1"), (1‴), (1⁗), and (2) is performed in the presence of a solvent containing acetonitrile.
[54-2] The method according to any of [6] to [51] or [B1], wherein one or more steps of the step (3) or (3') is carried out in the presence of a solvent containing at least one selected from the group consisting of acetonitrile, methanol, 1,3-dimethyl-2-imidazolidinone, and dimethyl sulfoxide.
[54-1] The method according to any of [6] to [51] or [B1], wherein one or more steps of the step (3) or (3') is carried out in the presence of a solvent containing 1,3-dimethyl-2-imidazolidinone.
[55] The method according to any of [13] to [54], wherein the washing solution has a pH of 10 to 14.
[56] The method according to any of [13] to [54], wherein the washing solution contains at least one selected from the group consisting of an aqueous ammonia solution, an aqueous carbonate solution, and an aqueous phosphate solution.
[57] The method according to any of [13] to [54], wherein the washing solution is an aqueous ammonia solution.
[58] A method for producing a first compound, comprising the method according to any of [1] to [57].

[A1] A method for producing a peptide compound, comprising the following steps of:

1) treating a protecting-group peptide protected by a protecting group having a Fmoc skeleton with a deprotecting agent capable of deprotecting a protecting group having a Fmoc skeleton in the presence of a sulfite ion or a bisulfite ion to obtain a deprotected peptide in which the protecting group is removed; and
2) optionally extending the deprotected peptide with one or more amino acids to obtain an extended peptide.

[A2] A method for producing a peptide compound, comprising the following steps of:

1) treating a protected peptide in which an amino group is protected by a protecting group having a Fmoc skeleton with a deprotecting agent capable of deprotecting a protecting group having a Fmoc skeleton to obtain a first mixture of (a) dibenzofulvene or a dibenzofulvene derivative and (b) a deprotected peptide in which the protecting group is removed;
2) treating the first mixture with a sulfite ion or a bisulfite ion to remove the dibenzofulvene or the dibenzofulvene derivative to obtain a second mixture containing the deprotected peptide; and
3) optionally extending the deprotected peptide with one or more amino acids to obtain an extended peptide.

[A3] The method according to [A1] or [A2], wherein the deprotected peptide or the extended peptide has an amino acid residue having one reaction site on the C-terminal side and an amino acid residue having another reaction site on the N-terminal side, and the method further comprises a step of binding the one reaction site with another reaction site to form a cyclic peptide compound.

[59] The production method according to [58], wherein the first compound is an amino acid or a peptide.
[60] The production method according to [58], wherein the first compound is (9H-fluoren-9-yl)methanesulfonic acid or a salt thereof or a (9H-fluoren-9-yl)methanesulfonic acid derivative or a salt thereof.
[61] The production method according to any of [58] to [60], wherein the method is carried out by liquid phase synthesis.
[62] The production method according to any of [58] to [60], wherein the method is carried out by solid phase synthesis.
[63] The production method according to any of [58] to [62], further comprising, before the step (2), (3) or (3'), a step of condensing a second amino group-containing compound in which an amino group is protected by a protecting group having a Fmoc skeleton and a third amino group-containing compound to obtain the first amino group-containing compound in which an amino group is protected by a protecting group having a Fmoc skeleton.
[64] The production method according to [63], wherein the second amino group-containing compound is an amino group-containing compound having a carboxyl group.
[65] The production method according to [63], wherein the second amino group-containing compound is an amino acid or a peptide.
[66] The production method according to [63], wherein the second amino group-containing compound is an amino acid.
[67] The production method according to any of [63] to [66], wherein the third amino group-containing compound is an amino group-containing compound that is not bound to a carrier for liquid phase peptide synthesis.
[68] The production method according to [67], wherein the carrier for liquid phase peptide synthesis is a compound that binds to the third amino group-containing compound directly or via a linker to make them soluble in an organic solvent and insoluble in water.
[68-1] The production method according to any of [63] to [66], wherein the third amino group-containing compound is an amino group-containing compound that is not bound to a carrier for liquid phase peptide synthesis, and the carrier for liquid phase peptide synthesis is a compound that binds to the third amino group-containing compound directly or via a linker to make the third amino group-containing compound more soluble in an organic solvent and more insoluble in water.
[69] The production method according to [67], wherein the third amino group-containing compound is a peptide, an amino acid, or an amino acid amide.
[70] The method according to any of [63] to [69] wherein the protective group having a Fmoc skeleton is a 9-fluorenylmethyloxycarbonyl group.
[71] The production method according to any of [58] to [70], comprising repeating the method according to any of [1] to [57] 2 times or more.
[71-1] Use of the following (a) and/or (b) for removing dibenzofulvene or a dibenzofulvene derivative:

(a) a sulfite ion or a bisulfite ion or a compound that produces a sulfite ion or a bisulfite ion,
(b) at least one compound selected from the group consisting of bisulfite, disulfurous acid and a salt thereof, sulfurous acid and a salt thereof, dithionous acid and a salt thereof, and a solvate of these.

[71-2] Combination use of the following (a) and/or (b) with a deprotecting agent capable of deprotecting a protecting group having a Fmoc skeleton, for deprotecting a protecting group having a Fmoc skeleton:

(a) a sulfite ion or a bisulfite ion or a compound that produces a sulfite ion or a bisulfite ion,
(b) at least one compound selected from the group consisting of bisulfite, disulfurous acid and a salt thereof, sulfurous acid and a salt thereof, dithionous acid and a salt thereof, and a solvate of these.

[72] A composition comprising: an amino group-containing compound; and (9H-fluoren-9-yl)methanesulfonic acid or a salt thereof or a (9H-fluoren-9-yl)methanesulfonic acid derivative or a salt thereof, wherein a value represented by the following formula A, calculated from a UVarea value at 210 nm by HPLC analysis is 1% or less:

area value of (9H-fluoren-9-yl)methanesulfonic acid or salt thereof or (9H-fluoren-9-yl)methanesulfonic acid derivative or salt thereof/(area value of amino group-containing compound + area value of (9H-fluoren-9-yl)methanesulfonic acid or salt thereof or (9H-fluoren-9-yl)methanesulfonic acid derivative or salt thereof) $\times$ 100 (%). (formula A):

[72-1] A composition comprising an amino group-containing compound, and (9H-fluoren-9-yl)methanesulfonic acid or a salt thereof or a (9H-fluoren-9-yl)methanesulfonic acid derivative or a salt thereof, wherein a content ratio of the (9H-fluoren-9-yl)methanesulfonic acid or a salt thereof or the (9H-fluoren-9-yl)methanesulfonic acid derivative or a salt thereof to the amino group-containing compound, and the (9H-fluoren-9-yl)methanesulfonic acid or a salt thereof or the (9H-fluoren-9-yl)methanesulfonic acid derivative or a salt thereof is 0.01 or less.

[73] The composition according to [72], wherein the (9H-fluoren-9-yl)methanesulfonic acid or a salt thereof or the (9H-fluoren-9-yl)methanesulfonic acid derivative or a salt thereof is (9H-fluoren-9-yl)methanesulfonic acid or a salt thereof.

[74] The composition according to [72] or [73], wherein the amino group-containing compound is a peptide, an amino acid, or an amino acid amide.

In the above numbering, the number cited in the dependent item includes numbers whose branch numbers are different, unless otherwise mentioned. For example, [72] cited in the dependent item shows that not only [72] but also [72-1] is included. The same applies to other numberings.

Advantageous Effects of Invention

According to the present invention, a new method for removing dibenzofulvene, the method being capable of capturing dibenzofulvene generated in a step of deprotecting a protecting group having a Fmoc skeleton, and removing dibenzofulvene without regeneration.

Description of Embodiments

(Terminology)

The "dibenzofulvene derivative" as used herein means a compound in which any substituent has been introduced at any position on the fluorene ring in dibenzofulvene. Examples of the dibenzofulvene derivative include a compound represented by the following formula:

[Formula 9]

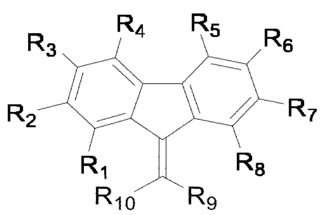

wherein,

$R_1$ to $R_8$ are independently selected from the group consisting of hydrogen, $C_1$-$C_8$ alkyl, $C_1$-$C_8$ fluoroalkyl, halogen, sulfo, and trimethylsilyl, but at least one of $R_1$ to $R_8$ is other than hydrogen; and $R_9$ to $R_{10}$ are independently hydrogen or methyl.

The dibenzofulvene derivative is preferably those in which only one, two, three, or four of $R_1$ to $R_8$ are not hydrogen, more preferably those in which only one or two of $R_1$ to $R_8$ are not hydrogen, and most preferably those in which only two of $R_1$ to $R_8$ are not hydrogen.

As used herein, the terms "(9H-fluoren-9-yl)methanesulfonic acid" and "(9H-fluoren-9-yl)methanesulfonic acid derivative" mean to compounds in which a group represented by "$-SO_3H$" is introduced at the end on exoolefin in dibenzofulvene and a dibenzofulvene derivative. Examples of the (9H-fluoren-9-yl)methanesulfonic acid and the (9H-fluoren-9-yl)methanesulfonic acid derivative include compounds represented by the following formula:

[Formula 10]

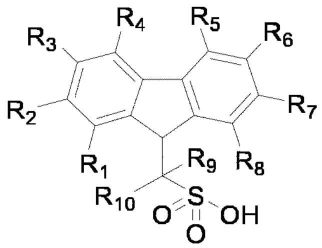

wherein,
$R_1$ to $R_8$ are independently selected from the group consisting of hydrogen, $C_1$-$C_8$ alkyl, $C_1$-$C_8$ fluoroalkyl, halogen, sulfo, and trimethylsilyl; and $R_9$ to $R_{10}$ are independently hydrogen or methyl.

The (9H-fluoren-9-yl)methanesulfonic acid derivative is preferably those in which only one, two, three, or four of $R_1$ to $R_8$ are not hydrogen, more preferably those in which only one or two of $R_1$ to $R_8$ are not hydrogen, and most preferably those in which only two of $R_1$ to $R_8$ are not hydrogen.

As used herein, the terms "salt of (9H-fluoren-9-yl)methanesulfonic acid" and "salt of (9H-fluoren-9-yl)methanesulfonic acid derivative" mean to a compound in which a group represented by "$-SO_3^-$" is introduced on exoolefin in dibenzofulvene and a dibenzofulvene derivative, or a salt of a compound in which a group represented by "$-SO_3H$" is introduced at the end on exoolefin in dibenzofulvene and a dibenzofulvene derivative. The salt of a compound described herein can be, for example, a salt with at least one selected from the group consisting of an alkali metal, an alkaline earth metal, and an ammonium ($NH_4^+$). Examples of the salt of (9H-fluoren-9-yl)methanesulfonic acid and the salt of (9H-fluoren-9-yl)methanesulfonic acid derivative include compounds represented by the following formula:

[Formula 11]

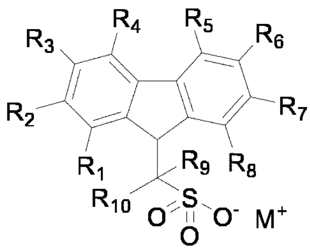

$M^+$: Counter Cation

wherein,
$R_1$ to $R_8$ are independently selected from the group consisting of hydrogen, $C_1$-$C_8$ alkyl, $C_1$-$C_8$ fluoroalkyl, halogen, sulfo, and trimethylsilyl; and $R_9$ to $R_{10}$ are independently hydrogen or methyl.

The salt of the (9H-fluoren-9-yl)methanesulfonic acid derivative is preferably those in which only one, two, three, or four of $R_1$ to $R_8$ are not hydrogen, more preferably those in which only one or two of $R_1$ to $R_8$ are not hydrogen, and most preferably those in which only two of $R_1$ to $R_8$ are not hydrogen.

The "protective group containing a Fmoc skeleton" as used herein means a group in which any substituent is introduced at any position on a Fmoc group or a skeleton constituting a Fmoc group. Specific examples of such a protective group having a Fmoc skeleton include protective groups represented by the following formula:

[Formula 12]

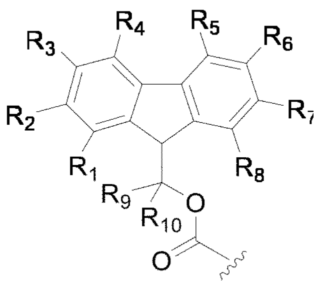

wherein,
$R_1$ to $R_8$ are independently selected from the group consisting of hydrogen, $C_1$-$C_8$ alkyl, $C_1$-$C_8$ fluoroalkyl, halogen, sulfo, and trimethylsilyl; $R_9$ to $R_{10}$ are independently hydrogen or methyl; and the wavy line represents a point of attachment to an amino group.

The protecting group having a Fmoc skeleton is preferably those in which only one, two, three, or four of $R_1$ to $R_8$ are not hydrogen, more preferably those in which only one or two of $R_1$ to $R_8$ are not hydrogen, and most preferably those in which only two of $R_1$ to $R_8$ are not hydrogen.

More specific examples of the protective group having a Fmoc skeleton include a 9-fluorenylmethyloxycarbonyl (Fmoc) group, a 2,7-di-tert-butyl-Fmoc (Fmoc (2,7tb)) group, a 1-methyl-Fmoc (Fmoc (1Me)) group, a 2-fluoro-Fmoc (Fmoc (2F)) group, a 2,7-dibromo-Fmoc (Fmoc (2,7Br)) group, a 2-monoisooctyl-Fmoc (mio-Fmoc) group, a 2,7-diisooctyl-Fmoc (dio-Fmoc) group, a 2,7-(3,3,4,4,5,5,6,6,7,7,8,8,8-tridecafluorooctyl)-Fmoc (tdf-Fmoc) group, a 2,7-bis(trimethylsilyl)-Fmoc (Fmoc (2TMS)) group, a (2-sulfo-9H-fluoren-9-yl)methyloxycarbonyl group (Fmoc (2so3h)), a [(1S)-1-(9H-fluoren-9-yl)ethoxy]carbonyl group (sm-Fmoc), and a [(1R)-1-(9H-fluoren-9-yl)ethoxy]carbonyl group (rm-Fmoc). Such a protective group having a Fmoc skeleton is preferably a Fmoc group. Such a protective group having a Fmoc skeleton can be introduced by a known method using a commercially available reagent or the like.

The phrase "remove a protecting group" as used herein is also referred to as "deprotect a protecting group".

As used herein, the phrase "capture dibenzofulvene or a dibenzofulvene derivative" means to make the dibenzofulvene or the dibenzofulvene derivative coexist with a sulfite ion or a bisulfite ion to form (9H-fluoren-9-yl) methanesulfonic acid or a salt thereof or a (9H-fluoren-9-yl)methanesulfonic acid derivative or a salt thereof.

As used herein, the term "sulfite ion" means an ion represented by "$SO_3^{2-}$", and the term "bisulfite ion" means an ion represented by "$HSO_3^-$". The term "compound that produces a sulfite ion or a bisulfite ion" as used herein is not particularly limited as long as the compound that produces a sulfite ion or a bisulfite ion by ionization in a solution (preferably in an aqueous solution). Examples thereof include bisulfite, disulfurous acid ($H_2S_2O_5$) and a salt thereof, sulfurous acid ($H_2SO_3$) and a salt thereof, dithionous acid ($H_2S_2O_4$) and a salt thereof, and a solvate of these. Examples of the bisulfite, the disulfite, the sulfite, and the dithionite include salts with at least one selected from the group consisting of alkali metals such as sodium and potassium, alkaline earth metals such as calcium, and ammonium. Specific examples of the bisulfite include sodium bisulfite and potassium bisulfite. Specific examples of the disulfite include sodium disulfite (sodium metabisulfite) and potassium disulfite (potassium metabisulfite). Specific examples of the sulfite include sodium sulfite, potassium sulfite, ammonium sulfite, and calcium sulfite. Specific examples of the dithionite include sodium dithionite and potassium dithionite.

The term "halogen" as used herein refers to, for example, F, Cl, Br, or I.

The "alkyl" as used herein is a monovalent group that is induced by the removal of any one hydrogen atom from aliphatic hydrocarbon and has a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear form but also a branched form. The alkyl is specifically alkyl having 1 to 20 carbon atoms ($C_1$-$C_{20}$; hereinafter, "$C_p$-$C_q$" means that the number of carbon atoms is p to q), and examples thereof include preferably $C_1$-$C_{10}$ alkyl, more preferably $C_1$-$C_8$ alkyl, further preferably $C_1$-$C_6$ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The "fluoroalkyl" as used herein means a group in which one or more hydrogen atoms of the "alkyl" defined above are replaced with a fluorine atom, and is preferably $C_1$-$C_8$ fluoroalkyl. Specific examples of the fluoroalkyl include monofluoromethyl, difluoromethyl, trifluoromethyl, 2,2-difluoroethyl, 2,2,2-trifluoroethyl, 3,3-difluoropropyl, 4,4-difluorobutyl, 5,5-difluoropentyl, and 3,3,4,4,5,5,6,6,7,7,8,8,8-tridecafluorooctyl.

The "sulfo" as used herein is a monovalent group represented by $-SO_3H$.

The "alkenyl" as used herein refers to a monovalent group having at least one double bond (two adjacent $SP^2$ carbon atoms). Depending on the placement of the double bond and substitutions (if present), the geometric form of the double bond can take entgegen (E) or tuzanmen (Z), cis or trans configurations. The alkenyl includes not only a linear form but also a branched form. Preferred examples of the alkenyl include $C_2$-$C_{10}$ alkenyl, more preferably $C_2$-$C_6$ alkenyl, and specific examples thereof include vinyl, allyl, 1-propenyl, 2-propenyl, 1-butenyl, 2-butenyl (including cis, trans), 3-butenyl, pentenyl, 3-methyl-2-butenyl, and hexenyl.

The "alkynyl" as used herein refers to a monovalent group having at least one triple bond (two adjacent SP carbon atoms). The alkynyl includes not only a linear form but also a branched form. Preferred examples of the alkynyl include $C_2$-$C_{10}$ alkynyl, more preferably $C_2$-$C_6$ alkynyl, and specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

The "aryl" as used herein means a monovalent aromatic hydrocarbon ring, and preferably includes $C_6$-$C_{10}$ aryl. Specific examples of the aryl include phenyl and naphthyl (e.g., 1-naphthyl, 2-naphthyl).

The "heteroaryl" as used herein means an aromatic cyclic monovalent group containing 1 to 5 heteroatoms in addition to a carbon atom. The ring may be a monocyclic ring or a ring fused with other rings, or may be partially saturated. The number of atoms constituting the ring is preferably 5 to 10 (5- to 10-membered heteroaryl), and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, indolizinyl, and imidazopyridyl.

The "aralkyl (arylalkyl)" as used herein means a group in which at least one hydrogen atom of the above-defined "alkyl" is replaced with the above-defined "aryl", and is preferably $C_7$-$C_{14}$ aralkyl, more preferably $C_7$-$C_{10}$ aralkyl. Specific examples of the aralkyl include benzyl, phenethyl, and 3-phenylpropyl.

The "heteroarylalkyl (heteroaralkyl)" as used herein means a group in which at least one hydrogen atom of the above-defined "alkyl" is replaced with the above-defined "heteroaryl", and is preferably 5- to 10-membered heteroaryl $C_1$-$C_6$ alkyl, more preferably 5- to 10-membered heteroaryl $C_1$-$C_2$ alkyl. Specific examples of the heteroarylalkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5-benzofuranyl) ethyl.

The "cycloalkyl" as used herein means a saturated or partially saturated cyclic monovalent aliphatic hydrocarbon group, and includes monocyclic rings, bicyclocyclic (e.g., fused, bridged, bicyclic spirocyclic) rings, and other polycyclic rings. Preferred examples of the cycloalkyl include $C_3$-$C_8$ cycloalkyl, and specific examples include cyclopropyl, cyclobutyl, cyclopentyl, cyclohexyl, cycloheptyl, cyclooctyl, bicyclo[2.2.1]heptyl, and spiro[3.3]heptyl.

The "alkoxy" as used herein means an oxy group to which the above-defined "alkyl" is attached, and preferably includes $C_1$-$C_6$ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The "fluoroalkoxy" as used herein means a group in which one or more hydrogen atoms of the above-defined "alkoxy" are replaced with a fluorine atom, and is preferably $C_1$-$C_8$ fluoroalkoxy. Specific examples of the fluoroalkoxy include monofluoromethoxy, difluoromethoxy, and trifluoromethoxy.

The "amino" as used herein means -NRR', wherein R and R' are each independently selected from the group consisting of hydrogen, alkyl, alkenyl, alkynyl, cycloalkyl, heterocyclyl, aryl, and heteroaryl, or R and R' mean a group formed by ring formation together with the nitrogen atom to which they are attached. Preferred examples of the amino include $-NH_2$, mono-$C_1$-$C_6$ alkylamino, di-$C_1$-$C_6$ alkylamino, and 4- to 8-membered cyclic amino.

The "aminocarbonyl" as used herein means a carbonyl group to which the above-defined "amino" is attached, and preferably includes $-CONH_2$, mono-$C_1$-$C_6$ alkylaminocarbonyl, di-$C_1$-$C_6$ alkylaminocarbonyl, and 4- to 8-membered cyclic aminocarbonyl. Specific examples of the aminocarbonyl include $-CONH_2$, dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3 .2.1]octan-8-ylcarbonyl.

The "alkylsulfonyl" as used herein means a sulfonyl group to which the above-defined "alkyl" is attached, and preferably includes $C_1$-$C_6$ alkylsulfonyl. Specific examples of the alkylsulfonyl include methylsulfonyl.

The "alkylsulfonylamino" as used herein means an amino group to which the above-defined "alkylsulfonyl" is attached, and preferably includes $C_1$-$C_6$ alkylsulfonylamino. Specific examples of the alkylsulfonylamino include methylsulfonylamino.

The "heterocyclyl" as used herein means a non-aromatic cyclic monovalent group containing 1 to 5 heteroatoms in addition to a carbon atom. The heterocyclyl may contain a double and/or triple bond in the ring, and the carbon atoms in the ring may be oxidized to form carbonyl, and the ring may be a monocyclic ring or a fused ring. The number of atoms constituting the ring is preferably 4 to 10 (4- to 10-membered heterocyclyl), more preferably 4 to 7 (4- to 7-membered heterocyclyl). Specific examples of the heterocyclyl include azetidinyl, oxiranyl, oxetanyl, azetidinyl, dihydrofuryl, tetrahydrofuryl, dihydropyranyl, tetrahydropyranyl, tetrahydropyridyl, tetrahydropyrimidyl, morpholinyl, thiomorpholinyl, pyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, azetidinyl, oxazolidone, benzodioxanyl, benzoxazolyl, dioxolanyl, dioxanyl, tetrahydropyrrolo[1,2-c]imidazole, thietanyl, 3,6-diazabicyclo[3.1.1]heptanyl, 2,5-diazabicyclo[2.2.1]heptanyl, 3-oxa-8-azabicyclo[3.2.1]octanyl, sultam, and 2-oxaspiro[3.3]heptyl.

The "solvate" as used herein refers to a molecular population that is formed by a compound together with a solvent. Examples of the solvate include hydrates, alcohol solvates (such as ethanol solvates, methanol solvates, 1-propanol solvates, 2-propanol solvates), and not only solvates formed with a single solvent such as dimethyl sulfoxide, but also solvates formed with a plurality of solvents per one molecule of the compound, or solvates formed with a plurality of types of solvents per one molecule of the compound. The solvate is a hydrate when the solvent is water. The solvate of the

compound according to the present invention is preferably a hydrate. Specific examples of such a hydrate include a hemi- to deca-hydrate, preferably a hemi- to penta-hydrate, and more preferably a hemi- to tri-hydrate.

The term "amino acid" as used herein includes a natural amino acid and a non-natural amino acid. The "amino acid" as used herein may mean an amino acid residue. The term "natural amino acid" as used herein refers to Gly, L-Ala, L-Ser, L-Thr, L-Val, L-Leu, L-Ile, L-Phe, L-Tyr, L-Trp, L-His, L-Glu, L-Asp, L-Gln, L-Asn, L-Cys, L-Met, L-Lys, L-Arg, or L-Pro. Examples of the non-natural amino acid include, but are not particularly limited to, β-amino acids, D-amino acids, N-substituted amino acids, α,α-disubstituted amino acids, amino acids having a side chain different from the natural one, and hydroxycarboxylic acid. As the amino acid herein, amino acids having any conformation are acceptable. The selection of a side chain of the amino acid is not particularly limited, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, a spiro-bonded cycloalkyl group, and the like. Each of the side chains may have a substituent. The substituent is also not limited, and one or two or more substituents may be freely selected independently from any substituents including, for example, a halogen atom, an O atom, an S atom, an N atom, a B atom, a Si atom, or a P atom. That is, examples of the side chain include an alkyl group, an alkoxy group, an alkoxyalkyl group, an alkenyl group, an alkynyl group, an aryl group, a heteroaryl group, an aralkyl group, or a cycloalkyl group which may be substituted, or oxo, aminocarbonyl, and a halogen atom. In a non-limiting embodiment, the amino acid as used herein may be a compound having a carboxyl group and an amino group in the same molecule (even in this case, proline, hydroxyproline, azetidine-2-carboxylic acid, and the like in which a nitrogen atom of the amino group and any atom of the side chain together forms a ring are also included in the amino acid).

The term "amino acid amide" as used herein means a compound in which at least one carboxyl group of a natural amino acid or a non-natural amino acid has been converted to an amide group.

The term "peptide" as used herein means a compound wherein two or more amino acids are linked by an amide bond. Peptides having an ester bond in a portion of the main chain, such as depsipeptide, are also included in the peptide herein. The number of residues contained in the peptide is preferably 2 to 29 residues, more preferably 3 to 20 residues, and further preferably 4 to 14 residues. The peptide and the peptide compound may be a linear peptide or a cyclic peptide.

The term "amino group-containing compound" as used herein means a compound having at least one primary amino group and/or secondary amino group. The term "an amino group-containing compound in which an amino group is protected by a protecting group" as used herein means a compound in which at least one primary amino group and/or secondary amino group contained in the amino group-containing compound is protected by a protecting group.

As used herein, "may be substituted" means that a group is optionally substituted by any substituent. Furthermore, a substituent may be added to each of the substituents. Such a substituent is not limited and can be one or two or more substituents each independently freely selected from any substituents including a halogen atom, an oxygen atom, a sulfur atom, a nitrogen atom, a boron atom, a silicon atom, or a phosphorus atom. Examples of the substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, and alkoxycarbonyl.

In the present specification, examples of the nitrile-based solvent include acetonitrile and propionitrile.

In the present specification, examples of the amide-based solvent include dimethylformamide, dimethylacetamide, and N-methyl-2-pyrrolidone, 1,3-dimethyl-2-imidazolidinone.

Examples of the sulfoxide-based solvent as used herein include dimethyl sulfoxide, diethyl sulfoxide, methyl ethyl sulfoxide, and methylphenyl sulfoxide.

In the present specification, examples of the alcohol-based solvent include methanol, ethanol, n-propanol, and 2-propanol.

In the present specification, examples of the ether-based solvent include diethyl ether, tetrahydrofuran, 2-methyltetrahydrofuran, 1,4-dioxane, 1,3-dioxolane, 1,2-dimethoxyethane, diisopropyl ether, cyclopentyl methyl ether, t-butyl methyl ether, 4-methyltetrahydropyran, diglyme, triglyme, and tetraglyme.

In the present specification, examples of the benzene-based solvent include benzene, toluene, xylene, fluorobenzene, chlorobenzene, 1,2-dichlorobenzene, bromobenzene, anisole, ethylbenzene, nitrobenzene, cumene, and benzotrifluoride.

In the present specification, examples of the ester-based solvent include methyl acetate, ethyl acetate, n-propyl acetate, isopropyl acetate, n-butyl acetate, and isobutyl acetate.

The term "one or more" as used herein means the number of 1 or 2 or more. When the term "one or more" is used in a context related to a substituent for a certain group, this term means a number from 1 to the maximum number of substituents accepted by the group. Specific examples of the term "one or more" include 1, 2, 3, 4, 5, 6, 7, 8, 9, 10, and/or greater numbers.

The meaning of the term "and/or" as used herein includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B and C.

(Removing method)

The method for removing dibenzofulvene or a dibenzofulvene derivative according to the present invention will be described. In an aspect, the dibenzofulvene or the dibenzofulvene derivative can be removed by any one of the following step (1), (1'), or (1"). As used herein, the phrase "remove dibenzofulvene or a dibenzofulvene derivative" includes converting the dibenzofulvene or the dibenzofulvene derivative to (9H-fluoren-9-yl)methanesulfonic acid or a salt thereof or a (9H-fluoren-9-yl)methanesulfonic acid derivative or a salt thereof to remove them. The method for removing dibenzofulvene or a dibenzofulvene derivative according to the present invention may be a method for removing dibenzofulvene or a dibenzofulvene derivative from a mixture containing the dibenzofulvene or the dibenzofulvene derivative.

The term "remove" as used herein means reducing an amount of a subject to 1% or less, 0.5% or less, 0.1% or less, or an undetectable amount, based on, for example, but not limited to, the total amount of the entire mixture containing the subject. The phrase "remove dibenzofulvene or a dibenzofulvene derivative" as used herein means reducing the value represented by the following Formula A, which is calculated, for example, by a UVarea value at 210 nm by HPLC analysis, to 1% or less, 0.5% or less, 0.1% or less, or an undetectable amount.

area value of (9H-fluoren-9-yl)methanesulfonic acid or salt thereof or (9H-fluoren-9-yl)methanesulfonic acid derivative or salt thereof/(area value of amino group-containing compound + area value of (9H-fluoren-9-yl)methanesulfonic acid or salt thereof or (9H-fluoren-9-yl)methanesulfonic acid derivative or salt thereof) $\times$ 100 (%). (formula A):

Steps (1) and (1')

Step (1) is a step of mixing (i) a dibenzofulvene or a dibenzofulvene derivative, and (ii) a sulfite ion or a bisulfite ion or a compound that produces a sulfite ion or a bisulfite ion. Step (1') is a step of mixing (i) a dibenzofulvene or a dibenzofulvene derivative, and (ii) at least one selected from the group consisting of bisulfite, disulfurous acid and a salt thereof, sulfurous acid and a salt thereof, dithionous acid and a salt thereof, and a solvate of these.

In the present invention, dibenzofulvene or a dibenzofulvene derivative can be removed by using a sulfite ion or a bisulfite ion or a compound generating these as a capture agent and converting the dibenzofulvene or the dibenzofulvene derivative to (9H-fluoren-9-yl)methanesulfonic acid or a salt thereof or a (9H-fluoren-9-yl)methanesulfonic acid derivative or a salt thereof.

In steps (1) and (1'), (i) and (ii) themselves may be mixed, or solutions obtained by dissolving (i) and/or (ii) in a solvent described below may be mixed. The term "mixing (i) and (ii)" as used herein includes any embodiments of adding (i) to (ii), adding (ii) to (i), and adding (i) and (ii) simultaneously. The term "mixing" as used herein does not require that a homogeneous mixture is obtained when mixing.

Steps (1) and (1') can be carried out in the presence or absence of a solvent, preferably at a temperature of -20°C to 80°C, more preferably at a temperature of 10°C to 80°C, and preferably for 0.1 to 48 hours, more preferably for 1 to 24 hours.

As (i), dibenzofulvene is preferably used. As (ii) in step (1'), bisulfite, disulfite, sulfite, dithionite, and a solvate of these are preferably used. Among these, salts of hydrogen sulfite, disulfurous acid, sulfurous acid, and dithionous acid with at least one selected from the group consisting of alkali metal, alkaline earth metal, and ammonium are preferably used; salts of hydrogen sulfite, disulfurous acid, sulfurous acid, and dithionous acid with at least one selected from the group consisting of sodium, potassium, calcium, and ammonium are more preferably used; sodium bisulfite, sodium metabisulfite, potassium bisulfite, sodium dithionite, ammonium sulfite monohydrate, calcium sulfite hemihydrate, sodium sulfite, and potassium sulfite are further preferably used; and sodium bisulfite, potassium bisulfite, and sodium dithionite are particularly preferably used.

The amount of (ii) used is not particularly limited, but for example, preferably 1 molar equivalent or more and 10 molar equivalents or less, more preferably 1 molar equivalent or more and 5 molar equivalents or less, per (i).

Examples of the solvent include at least one selected from the group consisting of a nitrile-based solvent, an amide-based solvent, an sulfoxide-based solvent, an alcohol-based solvent, and an ether-based solvent. Among them, a solvent containing at least one selected from the group consisting of acetonitrile, propionitrile, dimethylformamide, dimethylacetamide, N-methyl-2-pyrrolidone, 1,3-dimethyl-2-imidazolidinone, dimethyl sulfoxide, methanol, ethanol, n-propanol, 2-propanol, tetrahydrofuran, 2-methyltetrahydrofuran, diethyl ether, cyclopentylmethyl ether, and t-butylmethyl ether are preferably used; and a solvent containing at least one selected from the group consisting of acetonitrile, dimethylacetamide, methanol, and 2-methyltetrahydrofuran are more preferably used.

Steps (1) and (1') can be carried out in the presence of an additive. As the additive, a base can be used, for example, a tertiary amine such as triethylamine, N,N-diisopropylethylamine, and 2,6-lutidine is preferably used, and triethylamine is more preferably used.

The amount of the additive used is not particularly limited, but for example, preferably 1 molar equivalent or more and 20 molar equivalents or less, more preferably 1 molar equivalent or more and 10 molar equivalents or less, per (i).

Steps (1) and (1') can be carried out in the presence or absence of water, but preferably are carried out in the presence of water. The amount of water used is not particularly limited, but for example, preferably 1 molar equivalent or more and 300 molar equivalents or less, more preferably 1 molar equivalent or more and 100 molar equivalents or less, per (i). Preferably, the water is different from water contained in an organic solvent.

Steps (1"), (1‴) and (1⁗)

Step (1") is a step of forming (9H-fluoren-9-yl)methanesulfonic acid or a salt thereof or a (9H-fluoren-9-yl) methanesulfonic acid derivative or a salt thereof. The method of forming (9H-fluoren-9-yl)methanesulfonic acid or a salt thereof or a (9H-fluoren-9-yl)methanesulfonic acid derivative or a salt thereof is not particularly limited, but they can be formed, for example, by performing step (1) or (1') described above.

Step (1‴) is a step of reacting dibenzofulvene or a dibenzofulvene derivative with a sulfite ion or a bisulfite ion to form (9H-fluoren-9-yl)methanesulfonic acid or a salt thereof or a (9H-fluoren-9-yl)methanesulfonic acid derivative or a salt thereof.

Step (1⁗) is a step of mixing dibenzofulvene or a dibenzofulvene derivative, and, at least one selected from the group consisting of bisulfite, disulfurous acid and a salt thereof, sulfurous acid and a salt thereof, dithionous acid and a salt thereof, and a solvate of these to form (9H-fluoren-9-yl)methanesulfonic acid or a salt thereof or a (9H-fluoren-9-yl) methanesulfonic acid derivative or a salt thereof.

In an aspect, the following step (2) may be included before any of step (1), (1'), (1"), (1‴), or (1"").

Step (2)

Step (2) is a step of mixing, before step (1), (1'), (1"), (1‴), or (1"") described above, a deprotecting agent with a first amino group-containing compound in which an amino group is protected by a protecting group having a Fmoc skeleton.

The "protecting group having a Fmoc skeleton" in the first amino group-containing compound in which an amino group is protected by a protecting group having a Fmoc skeleton is preferably a 9-fluorenylmethyloxycarbonyl (Fmoc) group. The "first amino group-containing compound" in a first amino group-containing compound in which an amino group is protected by a protecting group having a Fmoc skeleton is preferably a peptide, an amino acid, or an amino acid amide, and more preferably a peptide. The peptide, the amino acid, or the amino acid amide herein includes a peptide, an amino acid, or an amino acid amide bound to a resin for solid phase synthesis, or a carrier for liquid phase peptide synthesis (e.g., a hydrophobic tag). Examples of the carrier for liquid phase peptide synthesis include compounds disclosed in Patent Literature 4, Patent Publication No. 5113118, Patent Publication No. 5929756, Patent Publication No. 6092513, Patent Publication No. 5768712, Patent Publication No. 5803674, Patent Publication No. 6116782, Patent Publication No. 6201076; Patent Publication No. 6283774; Patent Publication No. 6283775; Patent Publication No. 6322350; Patent Publication No. 6393857; Patent Publication No. 6531235; International Publication No. WO 2020/175472; and International Publication No. WO 2020/175473.

Step (2) can be carried out in the presence or absence of a solvent, preferably at a temperature of -20°C to 80°C, more preferably at a temperature of 10°C to 80°C, and preferably for 0.1 to 48 hours, more preferably for 0.5 to 24 hours.

Examples of the solvent include at least one selected from the group consisting of a nitrile-based solvent, an amide-based solvent, an sulfoxide-based solvent, an alcohol-based solvent, and an ether-based solvent. Among them, a solvent containing at least one selected from the group consisting of acetonitrile, propionitrile, dimethylformamide, dimethylacetamide, N-methyl-2-pyrrolidone, 1,3-dimethyl-2-imidazolidinone, dimethyl sulfoxide, methanol, ethanol, n-propanol, 2-propanol, tetrahydrofuran, 2-methyltetrahydrofuran, diethyl ether, cyclopentylmethyl ether, and t-butylmethyl ether are preferably used; and a solvent containing at least one selected from the group consisting of acetonitrile, dimethylacetamide, methanol, and 2-methyltetrahydrofuran are more preferably used.

As the deprotecting agent, a deprotecting agent capable of deprotecting a protecting group having a Fmoc skeleton is used. As a deprotecting agent, a base can be used, and examples thereof include an organic base having an amidine skeleton, a primary amine, a secondary amine, a tertiary amine, and an inorganic base. Examples of the organic base having an amidine skeleton include 1,8-diazabicyclo[5.4.0]undec-7-ene (DBU), 1,5-diazabicyclo[4.3.0]-5-nonen (DBN), and 7-methyl-1,5,7-triazabicyclo[4.4.0]dec-5-ene (MTBD). Among these, 1,8-diazabicyclo[5.4.0]undec-7-ene (DBU) is preferably used. Examples of the primary amine include propane-1-amine. Examples of the secondary amine include morpholine, diethylamine, dicyclohexylamine, 1,1,1,3,3,3-hexamethyldisilazane, piperidine, pyrrolidine, and piperazine. Among these, morpholine, diethylamine, dicyclohexylamine, and 1,1,1,3,3,3-hexamethyldisilazane are preferably used. Examples of the tertiary amine include triethylamine. Examples of the inorganic base include a carbonate such as sodium carbonate or potassium carbonate; a metal alkoxide such as a sodium tert-butoxide or potassium tert-butoxide. Among these, sodium carbonate and potassium tert-butoxide are preferably used.

The amount of the deprotecting agent is not particularly limited, but for example, preferably 0.5 molar equivalents or more and 10 molar equivalents or less, more preferably 1 molar equivalent or more and 5 molar equivalents or less, per the first amino group-containing compound in which an amino group is protected by a protecting group having a Fmoc skeleton.

In an aspect, the dibenzofulvene or the dibenzofulvene derivative can be removed by any one of the following step (3) or (3'). Preferably, the dibenzofulvene or the dibenzofulvene derivative can be removed from a mixture generated by the following step (3) or (3').

Steps (3) and (3')

Step (3) is a step of mixing (i) a first amino group-containing compound in which an amino group is protected by a protecting group having a Fmoc skeleton, (ii) a deprotecting agent, and (iii) a sulfite ion or a bisulfite ion or a compound that produces a sulfite ion or a bisulfite ion. Step (3') is a step of mixing (i) a first amino group-containing compound in which an amino group is protected by a protecting group having a Fmoc skeleton, (ii) a deprotecting agent, and (iii) at least one selected from the group consisting of bisulfite, disulfurous acid and a salt thereof, sulfurous acid and a salt thereof, dithionous acid and a salt thereof, and a solvate of these.

In steps (3) and (3'), (i), (ii) and (iii) themselves may be mixed, or solutions obtained by dissolving (i), (ii) and/or (iii) in a solvent described below may be mixed. The phrase "mixing (i) to (iii)" as used herein includes any embodiments of sequentially adding any component of (i) to (iii) to another component of (i) to (iii), and simultaneously adding (i) to (iii) to each other. Here, the "mixing" does not require that a homogeneous mixture is obtained when mixing.

The "protecting group having a Fmoc skeleton" in (i) is preferably a 9-fluorenylmethyloxycarbonyl (Fmoc) group. The "first amino group-containing compound" in (i) is preferably a peptide, an amino acid, or an amino acid amide, and more preferably a peptide or an amino acid. The peptide, the amino acid, or the amino acid amide herein includes a peptide, an amino acid, or an amino acid amide bound to a resin for solid phase synthesis, or a carrier for liquid phase peptide synthesis (e.g., a hydrophobic tag). Examples of the carrier for liquid phase peptide synthesis include compounds disclosed in Patent Literature 4, Patent Publication No. 5113118, Patent Publication No. 5929756, Patent Publication No. 6092513, Patent Publication No. 5768712, Patent Publication No. 5803674, Patent Publication No. 6116782, Patent Publication No. 6201076; Patent Publication No. 6283774; Patent Publication No. 6283775; Patent Publication No. 6322350; Patent Publication No. 6393857; Patent Publication No. 6531235; International Publication No. WO 2020/175472; and International Publication No. WO 2020/175473.

As the deprotecting agent (ii), a deprotecting agent capable of deprotecting a protecting group having a Fmoc skeleton is used. As (ii), a base can be used, and examples thereof include an organic base having an amidine skeleton, a primary amine, a secondary amine, a tertiary amine, and an inorganic base. Examples of the organic base having an amidine skeleton include 1,8-diazabicyclo[5.4.0]undec-7-ene (DBU), 1,5-diazabicyclo[4.3.0]-5-nonen (DBN), and 7-methyl-1,5,7-triazabicyclo[4.4.0]dec-5-ene (MTBD). Among these, 1,8-diazabicyclo[5.4.0]undec-7-ene (DBU) is preferably used. Examples of the primary amine include propane-1-amine. Examples of the secondary amines include morpholine, diethylamine, dicyclohexylamine, 1,1,1,3,3,3-hexamethyldisilazane, piperidine, pyrrolidine, and piperazine. Among these, morpholine, diethylamine, dicyclohexylamine, and 1,1,1,3,3,3-hexamethyldisilazane are preferably used. Examples of the tertiary amine include triethylamine. Examples of the inorganic base include a carbonate such as sodium carbonate or potassium carbonate; a metal alkoxide such as a sodium tert-butoxide or potassium tert-butoxide. Among these, sodium carbonate and potassium tert-butoxide are preferably used.

The amount of (ii) used is not particularly limited, but for example, preferably 0.5 molar equivalents or more and 10 molar equivalents or less, more preferably 1 molar equivalent or more and 5 molar equivalents or less, per (i).

As (iii) in step (3'), bisulfite, disulfite, sulfite, dithionite, and a solvate of these are preferably used. Among these, salts of hydrogen sulfite, disulfurous acid, sulfurous acid, and dithionous acid with at least one selected from the group consisting of alkali metal, alkaline earth metal, and ammonium are preferably used; salts of hydrogen sulfite, disulfurous acid, sulfurous acid, and dithionous acid with at least one selected from the group consisting of sodium, potassium, calcium, and ammonium are more preferably used; sodium bisulfite, sodium metabisulfite, potassium bisulfite, sodium dithionite, ammonium sulfite monohydrate, calcium sulfite hemihydrate, sodium sulfite, and potassium sulfite are further preferably used; and sodium bisulfite, potassium bisulfite, and sodium dithionite are particularly preferably used.

The amount of (iii) used is not particularly limited, but for example, preferably 1 molar equivalent or more and 10 molar equivalents or less, more preferably 1 molar equivalent or more and 5 molar equivalents or less, per (i).

Steps (3) and (3') can be carried out in the presence or absence of a solvent, preferably at a temperature of -20°C to 80°C, more preferably at a temperature of 10°C to 80°C, and preferably for 1 to 48 hours, more preferably for 1 to 24 hours.

Examples of the solvent include at least one selected from the group consisting of a nitrile-based solvent, an amide-based solvent, an sulfoxide-based solvent, an alcohol-based solvent, and an ether-based solvent. Among them, a

solvent containing at least one selected from the group consisting of acetonitrile, propionitrile, dimethylformamide, dimethylacetamide, N-methyl-2-pyrrolidone, 1,3-dimethyl-2-imidazolidinone, dimethyl sulfoxide, methanol, ethanol, n-propanol, 2-propanol, tetrahydrofuran, 2-methyltetrahydrofuran, diethyl ether, cyclopentylmethyl ether, and t-butylmethyl ether are preferably used; and a solvent containing at least one selected from the group consisting of acetonitrile, dimethylacetamide, 1,3-dimethyl-2-imidazolidinone, dimethyl sulfoxide, methanol, and 2-methyltetrahydrofuran are more preferably used.

Steps (3) and (3') can be carried out in the presence of an additive. In some embodiments, steps (3) and (3') are performed without additives. As the additive, a base can be used. For example, a tertiary amine such as triethylamine, N,N-diisopropylethylamine, and 2,6-lutidine is preferably used, and triethylamine is more preferably used.

The amount of additive used is not particularly limited, but for example, preferably 1 molar equivalent or more and 20 molar equivalents or less, more preferably 1 molar equivalent or more and 10 molar equivalents or less, per (i).

Steps (3) and (3') can be carried out in the presence or absence of water, but preferably are carried out in the presence of water. The amount of water used is not particularly limited, but for example, preferably 1 molar equivalent or more and 300 molar equivalents or less, more preferably 1 molar equivalent or more and 100 molar equivalents or less, per (i). Preferably, the water is not water contained in an organic solvent.

In an aspect, the following step (4) may be included after one or more of step (1), (1'), (1"), (1‴), (1⁗), (3), or (3').

Step (4)

Step (4) is a step of washing a mixture after one or more of steps (1), (1'), (1"), (1‴), (1""), (3), or (3') with a washing solution. This step can efficiently remove dibenzofulvene or a dibenzofulvene derivative (preferably (9H-fluoren-9-yl) methanesulfonic acid or a salt thereof or a (9H-fluoren-9-yl)methanesulfonic acid derivative or a salt thereof) in the mixture from an organic solution that is a mixture after one or more steps of the step (1), (1'), (1"), (1‴), (1⁗), (3), or (3'). As used herein, "washing" means removing a substance that may be an impurity, other than an object of interest, from a solution containing the object of interest, using a solution not containing the object of interest, by a partitioning operation. The object of interest is usually present in the organic layer, and in this case, a substance that may be an impurity can be extracted into an aqueous layer to remove by washing the organic layer with an aqueous solution.

As the washing solution, it is preferable to use a basic solution (pH 10 to 14), usually a basic aqueous solution of pH 10 to 14. The washing solution is preferably at least one selected from the group consisting of an aqueous ammonia solution, an aqueous carbonate solution, and an aqueous phosphate solution, and more preferably an aqueous ammonia solution. The carbonate solution may be, for example, an aqueous sodium carbonate solution. The aqueous phosphate solution may be, for example, an aqueous potassium phosphate solution.

In step (4), it is preferable to use an extraction solvent to efficiently extract from the mixture an amino group-containing compound in which a protecting group having a Fmoc skeleton is deprotected. Examples of such an extraction solvent include at least one selected from the group consisting of a benzene-based solvent, an ester-based solvent, and an ether-based solvent. Among them, a solvent containing at least one selected from the group consisting of benzene, toluene, xylene, fluorobenzene, chlorobenzene, 1,2-dichlorobenzene, bromobenzene, anisol, ethylbenzene, nitrobenzene, cumen, benzotrifluoride, methyl acetate, ethyl acetate, n-propyl acetate, isopropyl acetate, n-butyl acetate, isobutyl acetate, 2-methyltetrahydrofuran, cyclopentylmethyl ether, and t-butyl methyl ether is preferred, and a solvent containing at least one selected from the group consisting of toluene, isopropyl acetate, and 2-methyltetrahydrofuran is more preferred.

(Production method of compound)

In one aspect, the present invention provides a method for producing a first compound, comprising a method for removing dibenzofulvene or a dibenzofulvene derivative described above. The first compound produced by the method is not particularly limited, and examples thereof include an amino acid and a peptide. In an aspect, (9H-fluoren-9-yl) methanesulfonic acid or a salt thereof or a (9H-fluoren-9-yl)methanesulfonic acid derivative or a salt thereof can be produced by the production method of the present invention.

In one aspect, the present invention provides a method for producing a peptide compound, comprising the following steps of:

1) treating a protecting-group peptide protected by a protecting group having a Fmoc skeleton with a deprotecting agent capable of deprotecting a protecting group having a Fmoc skeleton in the presence of a sulfite ion or a bisulfite ion to obtain a deprotected peptide in which the protecting group is removed; and
2) optionally extending the deprotected peptide with one or more amino acids to obtain an extended peptide.

In one aspect, the present invention provides a method for producing a peptide compound, comprising the

following steps of:

1) treating a protected peptide in which an amino group is protected by a protecting group having a Fmoc skeleton with a deprotecting agent capable of deprotecting a protecting group having a Fmoc skeleton to obtain a first mixture of (a) dibenzofulvene or a dibenzofulvene derivative and (b) a deprotected peptide in which the protecting group is removed;
2) treating the first mixture with a sulfite ion or a bisulfite ion to remove the dibenzofulvene or the dibenzofulvene derivative to obtain a second mixture containing the deprotected peptide; and
3) optionally extending the deprotected peptide with one or more amino acids to obtain an extended peptide.

In the production method of the present invention, the deprotected peptide or the extended peptide has an amino acid residue having one reaction site on the C-terminal side and an amino acid residue having another reaction site on the N-terminal side, and the production method may further comprise a step of binding the one reaction site with another reaction site to form a cyclic peptide compound.

The production method according to the present invention may be either a liquid phase synthesis method or a solid phase synthesis method, and is not limited to these, but the liquid phase synthesis method is preferred. The liquid-phase synthesis and solid-phase synthesis can be performed by methods well known to those skilled in the art.

The solid phase synthesis method is a method in which a compound is bound to a solid phase (resin for synthesis), and the compound and a reagent are chemically reacted on the solid phase to synthesize a compound of interest.

The solid phase synthesis method of a peptide is a method of synthesizing a peptide on a solid phase by binding a desired amino acid or peptide to a solid phase; and sequentially linking a desired amino acid or peptide to the amino acid or peptide bound to the solid phase to extend a peptide chain. A peptide of interest can be obtained by separating the peptide bound to the solid phase from the solid phase.

The liquid phase synthesis method is a method of synthesizing a compound of interest by performing a chemical reaction of a compound in a liquid phase (in a solution). The liquid phase synthesis method of a peptide encompasses a homogeneous reaction in which all reagents including an amino acid and a peptide are dissolved in a solvent, as well as a non-homogeneous reaction in which all or part of the reagents are not dissolved in a solvent but dispersed or suspended.

The production method according to the present invention may further comprise, before one or more of steps (2), (3), or (3') of the removing method described above, a step of condensing a second amino group-containing compound in which an amino group is protected by a protecting group having a Fmoc skeleton and a third amino group-containing compound to obtain the first amino group-containing compound in which an amino group is protected by a protecting group having a Fmoc skeleton.

The "protecting group having a Fmoc skeleton" in the second amino group-containing compound in which the amino group is protected by a protecting group having a Fmoc skeleton is preferably a 9-fluorenylmethyloxycarbonyl (Fmoc) group. In one embodiment of the "second amino group-containing compound" in a second amino group-containing compound in which an amino group is protected by a protecting group having a Fmoc skeleton is preferably an amino group-containing compound having a carboxyl group. In another embodiment of the "second amino group-containing compound" in a second amino group-containing compound in which an amino group is protected by a protecting group having a Fmoc skeleton is preferably a peptide or an amino acid, and more preferably an amino acid.

In one embodiment of the third amino group-containing compound, it is preferred that the third amino group-containing compound is an amino group-containing compound that is not bound to a carrier for liquid phase peptide synthesis, and more preferably the third amino group-containing compound is an amino group-containing compound that is not bound to a carrier for liquid phase peptide synthesis, and the carrier for liquid phase peptide synthesis is a compound that binds to the third amino group-containing compound directly or via a linker to make the third amino group-containing compound more soluble in an organic solvent and more insoluble in water. Examples of the above-described carrier for liquid phase peptide synthesis include the compounds described in Patent Literature 4. In another embodiment of the third amino group-containing compound is preferably a peptide, an amino acid, or an amino acid amide.

The step of obtaining the first amino group-containing compound in which an amino group is protected by a protecting group having a Fmoc skeleton can be carried out in the presence or absence of a solvent, in the presence or absence of a condensing agent, preferably at a temperature ranging from -50°C to a temperature near the boiling point of the solvent, more preferably in the range of -20°C to 80°C, preferably for 0.1 hours to 48 hours, more preferably for 0.5 hours to 24 hours.

Examples of the solvent include acetonitrile, 2-MeTHF, THF, dichloromethane, toluene, ethyl acetate, isopropyl acetate, DMF, DMA, and NMP.

Examples of the condensing agent that can be used include HATU (1-[bis(dimethylamino)methylene]-1H-1,2,3-triazolo[4,5-b]pyridinium 3-oxide hexafluorophosphate, CAS RN: 148893-10-1), T3P (2,4,6-tripropyl-1,3,5,2,4,6-trioxatriphosphorinane-2,4,6-trioxide (50% ethyl acetate solution, about 1.7 mol/L), CAS RN: 68957-94-8), HBTU (1-[bis(dimethylamino)methylene]-1H-benzotriazolium 3-oxide hexafluorophosphate, CAS RN: 94790-37-1), COMU ((1-cyano-2-ethoxy-2-oxoethylideneaminooxy)dimethylaminomorpholinocarbenium hexafluorophosphate, CAS RN: 1075198-30-9), BEP (2-bromo-1-ethylpyridinium tetrafluoroborate, CAS RN: 878-23-9), PyBOP (1H-benzotriazol-1-yloxytripyrrolidinophosphonium hexafluorophosphate, CAS RN: 128625-52-5), DMT-MM (4-(4,6-dimethoxy-1,3,5-triazin-2-yl)-4-methylmorpholinium chloride, CAS RN: 3945-69-5), PyOxim ([ethylcyano(hydroxyimino)acetato-O2]tri-1-pyrrolidinylphosphonium hexafluorophosphate, CAS RN: 153433-21-7), TATU (1-[bis(dimethylamino)methylene]-1H-1,2,3-triazolo[4,5-b] pyridinium 3-oxide tetrafluoroborate, CAS RN: 873798-09-5), TBTU (1-[bis(dimethylamino)methylene]-1H-benzotriazolium 3-oxide tetrafluoroborate, CAS RN: 125700-67-6), TOTU (O-[(ethoxycarbonyl)cyanomethyleneamino]-N,N,N',N'-tetramethyluronium tetrafluoroborate, CAS RN: 136849-72-4), EDCI (1-(3-dimethylaminopropyl)-3-ethylcarbodiimide, CAS RN: 1892-57-5), DIC (N,N'-diisopropylcarbodiimide, CAS RN: 693-13-0), and DCC (N,N'-dicyclohexylcarbodiimide, CAS RN: 538-75-0). The step of obtaining the first amino group-containing compound by such a condensation reaction can be performed with reference to the method described in the literature. For example, references can be made to Amino Acids, 2018, 50, 39-68; Solid phase peptide synthesis (published by Bachem Holding AG) [searched September 18, 2022], Internet <URL: https://www.bachem.com/wpfd_file/solid-phase-peptide-synthesis/>; Amino Acids, Peptides and Proteins in Organic Chemistry: Building Blocks, Catalysis and Coupling Chemistry, Volume 3, 2011; J. Peptide Res., 2005, 65, 153-166; Org. Process Res. Dev., 2018, 22, 760-772; Fundamentals and experiments of peptide synthesis, written by Nobuo Izumiya, published by Maruzen (1985); and the like.

The production method according to the present invention may include repeating the above-described removing method of dibenzofulvene or a dibenzofulvene derivative 2 times or more or 3 times or more.

One specific order of the method of the present invention includes performing step (2), followed by step (1), (1'), (1"), (1‴) or (1⁗), followed by (4). For example, a second amino group-containing compound in which an amino group is protected by a protecting group having a Fmoc skeleton and a third amino group-containing compound are condensed in a reaction vessel to obtain a first amino group-containing compound in which an amino group is protected by a protecting group having a Fmoc skeleton. Subsequently, a first amino group-containing compound in which an amino group is protected by a protecting group having a Fmoc skeleton is mixed with a deprotecting agent to deprotect the protecting group having a Fmoc skeleton. At this time, dibenzofulvene or a dibenzofulvene derivative is generated. Subsequently, a sulfite ion or a bisulfite ion or a compound that produces a sulfite ion or a bisulfite ion is added to the reaction vessel and mixed with the dibenzofulvene or the dibenzofulvene derivative to form (9H-fluoren-9-yl)methanesulfonic acid or a salt thereof or a (9H-fluoren-9-yl)methanesulfonic acid derivative or a salt thereof. Subsequently, an organic solvent is added to the reaction vessel, and the organic layer is washed with a washing solution.

One specific order of the method of the present invention includes performing step (3) followed by (4). For example, a second amino group-containing compound in which an amino group is protected by a protecting group having a Fmoc skeleton and a third amino group-containing compound are condensed in a reaction vessel to obtain the first amino group-containing compound in which an amino group is protected by a protecting group having a Fmoc skeleton. Subsequently, (i) a first amino group-containing compound in which an amino group is protected by a protecting group having a Fmoc skeleton in the reaction vessel is mixed with (ii) a deprotecting agent, and (iii) a sulfite ion or a bisulfite ion or a compound that produces a sulfite ion or a bisulfite ion to form (9H-fluoren-9-yl)methanesulfonic acid or a salt thereof or a (9H-fluoren-9-yl)methanesulfonic acid derivative or a salt thereof. Subsequently, an organic solvent is added to the reaction vessel and the organic layer is washed with a washing solution. In this method, deprotection of a protecting group having a Fmoc skeleton and formation of (9H-fluoren-9-yl)methanesulfonic acid or a salt thereof or a (9H-fluoren-9-yl) methanesulfonic acid derivative or a salt thereof is carried out in one step.

(Deprotection method)

In one aspect, the present invention provides a method for deprotecting a protecting group having a Fmoc skeleton, comprising a step of treating a first amino group-containing compound protected by a protecting group having a Fmoc skeleton with a deprotecting agent capable of deprotecting a protecting group having a Fmoc skeleton in the presence of a sulfite ion or a bisulfite ion.

(Use)

Another aspect of the present invention is the use of the following (a) and/or (b) for removing dibenzofulvene or a dibenzofulvene derivative:

(a) a sulfite ion or a bisulfite ion or a compound that produces a sulfite ion or a bisulfite ion,
(b) at least one compound selected from the group consisting of bisulfite, disulfurous acid and a salt thereof, sulfurous acid and a salt thereof, dithionous acid and a salt thereof, and a solvate of these.

(Combination use)

Another aspect of the present invention is the combination use of the following (a) and/or (b) with a deprotecting agent capable of deprotecting a protecting group having a Fmoc skeleton, for deprotecting a protecting group having a Fmoc skeleton:

(a) a sulfite ion or a bisulfite ion or a compound that produces a sulfite ion or a bisulfite ion,
(b) at least one compound selected from the group consisting of bisulfite, disulfurous acid and a salt thereof, sulfurous acid and a salt thereof, dithionous acid and a salt thereof, and a solvate of these.

(Composition)

The present invention provides a composition comprising: an amino group-containing compound; and (9H-fluoren-9-yl)methanesulfonic acid or a salt thereof or a (9H-fluoren-9-yl)methanesulfonic acid derivative or a salt thereof, in which a value represented by the following formula A, calculated from a UVarea value at 210 nm by HPLC analysis is 1% or less:

area value of (9H-fluoren-9-yl)methanesulfonic acid or salt thereof or (9H-fluoren-9-yl)methanesulfonic acid derivative or salt thereof/(area value of amino group-containing compound + area value of (9H-fluoren-9-yl)methanesulfonic acid or salt thereof or (9H-fluoren-9-yl)methanesulfonic acid derivative or salt thereof) $\times$ 100 (%). (formula A):

In an embodiment, the value represented by formula A is 1% or less, 0.5% or less, or undetectable.
The present invention provides a composition comprising an amino group-containing compound, and (9H-fluoren-9-yl)methanesulfonic acid or a salt thereof or a (9H-fluoren-9-yl)methanesulfonic acid derivative or a salt thereof, wherein a content ratio of the (9H-fluoren-9-yl)methanesulfonic acid or a salt thereof or the (9H-fluoren-9-yl)methanesulfonic acid derivative or a salt thereof to the amino group-containing compound, and the (9H-fluoren-9-yl)methanesulfonic acid or a salt thereof or the (9H-fluoren-9-yl)methanesulfonic acid derivative or a salt thereof is 0.01 or less. In an embodiment, the content ratio is calculated by measuring a UVarea value at 210 nm by HPLC analysis. In an embodiment, the content ratio is 0.01 or less, 0.005 or less, or undetectable.
In an aspect, the amino group-containing compound contained in the composition of the present invention is a peptide, an amino acid, or an amino acid amide. In an aspect, the (9H-fluoren-9-yl)methanesulfonic acid or a salt thereof or the (9H-fluoren-9-yl)methanesulfonic acid derivative or a salt thereof contained in the composition of the present invention is (9H-fluoren-9-yl)methanesulfonic acid or a salt thereof.
All of the prior art cited herein are incorporated herein by reference. This application claims priority to Japanese Patent Application No. 2022-175592, filed November 1, 2022, the contents of which are incorporated herein by reference in their entirety. Also, references cited herein, including the following references, inclusive of patent applications and publications, are incorporated herein by reference in their entirety: International Publication Nos. WO 2013100132, WO 2018225851, WO 2018225864, WO 2019117274, WO 2020111238, WO 2020122182, WO 2021075478, WO 2021090856, WO 2021132545, WO 2021246471, WO 2022097540, WO 2022138891, WO 2022145444, WO 2022234864, and WO 2023127869.

Examples

Hereinafter, the present disclosure will be described in more detail based on Examples, but the present disclosure is not limited to the following Examples.
The abbreviations used herein are listed below.

2-MeTHF: 2-methyltetrahydrofuran
CPME: cyclopentylmethyl ether
Cy: cyclohexyl
DIPEA: N,N-diisopropylethylamine
DMA: dimethylacetamide
DMF: N,N-dimethylformamide
DMI: 1,3-dimethyl-2-imidazolidinone
DMSO: dimethylsulfoxide
DBF: dibenzofulvene

DBU: diazabicycloundecene
Et: ethyl
EtOAc: ethyl acetate
Fmoc: 9-fluorenylmethoxycarbonyl
FMSA: 9H-fluorene-9-yl methanesulfonic acid
FMSA Salt: 9H-fluoren-9-yl methanesulfonate
HATU: O-(7-azabenzotriazol-1-yl)-N,N,N',N'-tetramethyluronium hexafluorophosphate
HMDS: 1,1,1,3,3,3-hexamethyldisilazane
IPAc: isopropyl acetate
Me: methyl
MeCN: acetonitrile
MeOH: methanol
NMM: N-methylmorpholine
NMP: N-methyl-2-pyrrolidone
$^{n}$Pr: n-propyl
T3P: propylphosphonic acid anhydride
TFA: trifluoroacetic acid

The contents of the present invention are further described by the following Examples, but the present invention is not limited to these contents. Except as specifically described, starting substances, starting materials, solvents, and reagents were obtained from commercial suppliers, or synthesized using known methods.
HPLC analysis was performed using H-Class system manufactured by Waters Corporation, by measuring with a PDA detector, and analyzing at a wavelength of 210 nm. Analysis conditions are shown below.

HPLC analysis conditions method 1

Apparatus: Waters(TM) ACQUITY UPLC H-Class
Column: CAPCELL CORE ADME (OSAKA SODA), 2.1 mm ID × 50 mm, 2.7 μm
Mobile phase: 0.05% TFA/water (A), 0.05% TFA/MeCN (B)
Elution method: B): 5%(0 min) → 100% (5 min) → 5% (5.1 min) → 5% (7 min)
Flow rate: 0.5 mL/min
Column temperature: 35°C
Detection wavelength: 210 nm (PDA)

HPLC analysis conditions method 2

Apparatus: Waters(TM) ACQUITY UPLC H-Class
Column: Ascentis Express C18 (Sigma-Aldrich(TM)), 4.6 mm ID × 50 mm, 2.7 μm
Mobile phase: 0.05% TFA/water (A), 0.05% TFA/MeCN (B)
Elution method: B) 5%(0 min)→100%(6 min)→100%(7 min)->5%(7.1 min)→5%(9 min)
Flow rate: 0.8 mL/min
Column temperature: 35°C
Detection wavelength: 210 nm (PDA)

[Table 1]

| Example No. (compound No.) | HPLC analysis conditions | Retention Time (min) |
|---|---|---|
| Example 1-(i) (compound 3) | Method 1 | 4.43 |
| Example 1-(ii) (compound 4) | Method 1 | 2.29 |
| Example 1-(iii) (compound 4) | Method 2 | 3.00 |
| Example 2 (FMSA or FMSA Salt) | Method 2 | 2.73 |

(continued)

| Example No. (compound No.) | HPLC analysis conditions | Retention Time (min) |
|---|---|---|
| Example 3 (compound 6) | Method 2 | 6.25 |
| Example 4-(i) (compound 8) | Method 1 | 3.50 |
| Example 4-(ii) (compound 9) | Method 1 | 1.61 |
| Example 4-(iii) (compound 11) | Method 1 | 4.37 |
| Example 4-(iv) (compound 12) | Method 1 | 2.20 |
| Example 4-(v) (compound 14) | Method 1 | 6.20 |
| Example 4-(vi) (compound 15) | Method 1 | 2.76 |
| Example 4-(vii) (compound 17) | Method 1 | 4.23 |
| Example 4-(viii) (compound 18) | Method 1 | 2.84 |
| Example 4-(ix) (compound 20) | Method 1 | 4.52 |
| Example 4-(x) (compound 21) | Method 1 | 2.58 |
| Example 5-(i) (compound 23) | Method 1 | 3.99 |
| Example 5-(ii) (compound 25) | Method 1 | 5.67 |
| Example 5-(iii) (compound 26) | Method 1 | 4.33 |
| Example 8-(i) (compound 33) | Method 1 | 4.48 |
| Example 8-(ii) (compound 34) | Method 1 | 2.37 |
| Example 11 (compound 35) | Method 1 | 2.08 |

LCMS analysis was performed using H-Class system manufactured by Waters Corporation. MS analysis was performed using a QDa detector or an SQD2 detector. Analysis conditions are shown below.

LCMS analysis conditions method 1

Apparatus: Waters(TM) ACQUITY UPLC H-Class + ACQUITY QDA
Column: CAPCELL CORE ADME manufactured (OSAKA SODA), 2.1 mm ID × 50 mm, 2.7 μm
Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min
Column temperature: 35°C
Detection wavelength: 210 nm (PDA)

LCMS analysis conditions method 2

Apparatus: Waters(TM) ACQUITY UPLC H-Class + SQD2
Column: Ascentis Express C18 (Sigma-Aldrich Co. LLC), 4.6 mm ID × 50 mm, 2.7 μm
Mobile phase: 0.05% TFA/water (A), 0.05% TFA/MeCN (B)
Elution method: B) 5% (0 min) → 100% (6 min) → 100% (7 min) → 5% (7.1 min) → 5% (9 min)
Flow rate: 0.8 mL/min
Column temperature: 35°C
Detection wavelength: 210 nm (PDA)

LCMS analysis conditions method 3

Apparatus: Waters(TM) ACQUITY UPLC H-Class + SQD2
Column: ACQUITY UPLC CSH C18 (Waters corporation), 2.1 mm ID × 150 mm, 1.7 μm
Mobile phase: 0.05% TFA/water (A), 0.05% TFA/MeCN (B)
Elution method: B) 20% (0 min) → 100% (24 min) → 100% (29 min) → 20% (29.1 min) → 20% (34 min)
Flow rate: 0.3 mL/min
Column temperature: 50°C
Detection wavelength: 220 nm (PDA)

[Table 2]

| Example No. (compound No.) | HPLC analysis conditions | Retention Time (min) | MS Found(m/z) | MS polarity |
|---|---|---|---|---|
| Example 1-(i) (compound 3) | Method 1 | 4.43 | 563.43 | $[M+Na]^+$ |
| Example 1-(ii) (compound 4) | Method 1 | 2.24 | 319.36 | $[M+H]^+$ |
| Example 1-(iii) (compound 4) | Method 2 | 3.00 | 319.749 | $[M+H]^+$ |
| Example 2 (FMSA or FMSA Salt) | Method 2 | 2.73 | 259.385 | $[M-H]^-$ |
| Example 3 (compound 6) | Method 2 | 6.25 | 702.968 | $[M+H]^+$ |
| Example 4-(ix) (compound 20) | Method 1 | 4.52 | 907.73 | $[M+H]^+$ |
| Example 4-(x) (compound 21) | Method 1 | 2.53 | 663.69 | $[M+H]^+$ |
| Example 5-(ii) (compound 25) | Method 3 | 23.54 | 1869.95 | $[M+Na]^+$ |
| Example 5-(iii) (compound 26) | Method 3 | 15.50 | 1625.65 | $[M+H]^+$ |
| Example 6-(i) (compound 28) | Method 1 | 4.72 | 591.96 | $[M+Na]^+$ |
| Example 6-(ii) (compound 29) | Method 1 | 2.60 | 347.89 | $[M+H]^+$ |
| Example 7 (compound 31) | Method 1 | 1.83 | 190.98 | $[M+H]^+$ |
| Example 8-(i) (compound 33) | Method 1 | 4.48 | 563.43 | $[M+Na]^+$ |
| Example 8-(ii) (compound 34) | Method 1 | 2.31 | 319.37 | $[M+H]^+$ |
| Example 11 (compound 35) | Method 1 | 2.01 | 266.29 | $[M+H]^+$ |

The $^1$H-NMR spectrum and $^{19}$F-NMR spectrum was measured with a nuclear magnetic resonance device JNM-ECZ500R (manufactured by JEOL Ltd.). The deuterium lock signal from a sample solvent was used as a reference. As the sample solvent, commercially available deuterated solvents were used, depending on the purpose of measurement. The chemical shift of tetramethylsilane used as an internal standard was set to 0 ppm, and the chemical shift of the signal of the compound to be analyzed was expressed in ppm. Abbreviations for splitting of a signal were expressed as follows: s = singlet, brs = broad singlet, d = doublet, t = triplet, q = quartet, dd = double doublet, and m = multiplet. The splitting width of a signal was expressed as a J value (Hz). The integral value of the signal was calculated based on a ratio of the signal area intensity of each signal.

The measuring method by qNMR was performed by dissolving a residue containing the compound of interest and an internal standard in DMSO-$d_6$ and subjecting to the following analysis conditions. The yield was calculated by the following formula using the value of the content of the compound of interest in the residue calculated by qNMR.

$$\text{Yield (\%)} = [\text{weight of residue (mg)} \times \text{content (\%)}]/\text{theoretical yield (mg)} \times 100$$

Measuring apparatus: JNM-ECZ500R
Internal standard: 3,5-bis(trifluoromethyl) benzoic acid
Measurement conditions ($^1$H-NMR): DMSO-$d_6$ 24.3°C; pulse angle 90°; digital resolution 0.25 Hz; relaxation time 60 seconds; no spin; cumulative number 8 times
Measurement conditions ($^{19}$F-NMR): DMSO-$d_6$, 24.3°C; pulse angle 90°; digital resolution 0.22 Hz; relaxation time 60 seconds; no spin; cumulative number 8 times

The measuring method by HPLC and LCMS was performed by preparing a mixed solution containing the compound of interest as a sample according to any of the following methods and subjecting it to the analysis conditions described above.

Sample preparation method 1: a mixed solution containing the compound of interest was diluted with MeCN.
Sample preparation method 2: a mixed solution containing the compound of interest was diluted with a mixed solution of MeCN and $H_2N^nPr$ in a ratio of 9 : 1.
Sample preparation method 3: a mixed solution containing the compound of interest was diluted with a mixed solution of MeOH and water in a ratio of 4 : 1.

The reaction conversion rate was calculated by any of the following formulas using the area value of the starting material and the area value of the compound of interest, or the area value of the starting material, the area value of the propylamide compound of the starting material and the area value of the compound of interest calculated by HPLC analysis.

Calculation formula 1 of reaction conversion rate: reaction conversion rate (%) = area value of compound of interest /(area value of starting material + area value of compound of interest) × 100

Calculation formula 2 of reaction conversion rate: reaction conversion rate (%) = area value of compound of interest /(area value of starting material + area value of propylamide compound of starting material + area value of compound of interest) × 100

The Fmoc group-deprotection reaction conversion rate was calculated by the following formula using the area value of the Fmoc-protected compound and the area value of the Fmoc-deprotected compound calculated by HPLC analysis.

Calculation formula of Fmoc group-deprotection reaction conversion rate: Fmoc group-deprotection reaction conversion rate (%) = area value of Fmoc-deprotected compound/(area value of Fmoc-protected compound + area value of Fmoc-deprotected compound) × 100

The DBF capture reaction conversion rate was calculated by the following formula using the area value of DBF and the area value of FMSA or FMSA salt, or the area value of compound 35, or the area value of compound 36, or the area value of compound 37 calculated by HPLC analysis.

Calculation formula 1 of DBF capture reaction conversion rate: DBF capture reaction conversion rate (%) = area value of FMSA or FMSA salt/(area value of DBF + area value of FMSA or FMSA salt) × 100

Calculation formula 2 of DBF capture reaction conversion rate: DBF capture reaction conversion rate (%) = area value of FMSA or FMSA salt/(area value of DBF + area value of compound 35 + area value of FMSA or FMSA salt) × 100

Calculation formula 3 of DBF capture reaction conversion rate: DBF capture reaction conversion rate (%) = area value of compound 36/(area value of DBF + area value of compound 36) × 100

Calculation formula 4 of DBF capture reaction conversion rate: DBF capture reaction conversion rate (%) = area value of compound 37/(area value of DBF + area value of compound 37) × 100

The FMSA or FMSA salt residual rate was obtained by the following formula using the Fmoc-deprotected compound and the area value of FMSA or FMSA salt calculated by HPLC analysis.

Calculation formula of FMSA or FMSA salt residual rate: FMSA or FMSA salt residual rate (%) = area value of FMSA or FMSA salt/(area value of Fmoc-deprotected compound + area value of FMSA or FMSA salt) × 100

<u>Example 1</u>

<u>(i) Synthesis of compound 3: (2S)-2-[[(1S)-1-benzyl-2-tert-butoxy-2-oxoethyl]carbamoyl]pyrrolidin-1-carboxylate 9H-fluoren-9-ylmethyl</u>

[Formula 13]

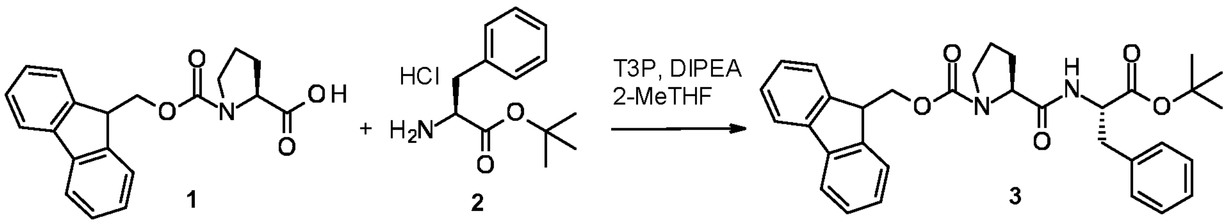


1 2 3

Compound 1 (0.964 g) and compound 2 (0.813 g, 1.1 eq.) were added to the reaction vessel, and the reaction vessel was subjected to nitrogen replacement. 2-MeTHF (12 mL, 12 v/w) and DIPEA (1.70 mL, 3.4 eq.) were then sequentially added at room temperature. T3P (50 w/w% 2-MeTHF solution, 2.68 mL, 1.5 eq.) was added while stirring the reaction mixture, then the mixture was stirred at room temperature for 1 hour. DIPEA (1.70 mL, 3.4 eq.) and T3P (50 w/w % 2-MeTHF solution, 2.68 mL, 1.5 eq.) were then added with stirring at room temperature, and the mixture was further stirred for 0.5 hours. The reaction mixture was sampled and prepared as a sample (sample preparation method 1), and it was confirmed by HPLC analysis that the reaction conversion rate was 99.9% (calculation formula 1 of reaction conversion rate). To the reaction vessel, 5% aqueous sodium carbonate solution (15 mL) was added, and the mixture was stirred for 10 minutes. After the aqueous layer was drained, the obtained organic layer was washed with 5% aqueous sodium bisulfate monohydrate solution (15 mL × 2), 5% aqueous sodium carbonate solution (15 mL), and 5% aqueous sodium chloride solution (15 mL). The obtained organic layer was dehydrated with sodium sulfate, and then sodium sulfate was removed by filtration. After concentration under reduced pressure at the external temperature of 40°C, a residue (1.43 g) containing compound 3 was obtained.

LC purity of compound 3: 99.0% (HPLC analysis conditions: method 1)
Content: 98.8 wt% (The obtained residue and 3,5-bis(trifluoromethyl)benzoic acid were dissolved in DMSO-$d_6$ and subjected to qNMR analysis.)

Examples using sodium bisulfite as DBF capture agent

<u>(ii) Synthesis of compound 4: tert-butyl (2S)-3-phenyl-2-[[(2S)-pyrrolidin-2-carbonyl]amino]propanoate</u>

[Formula 14]

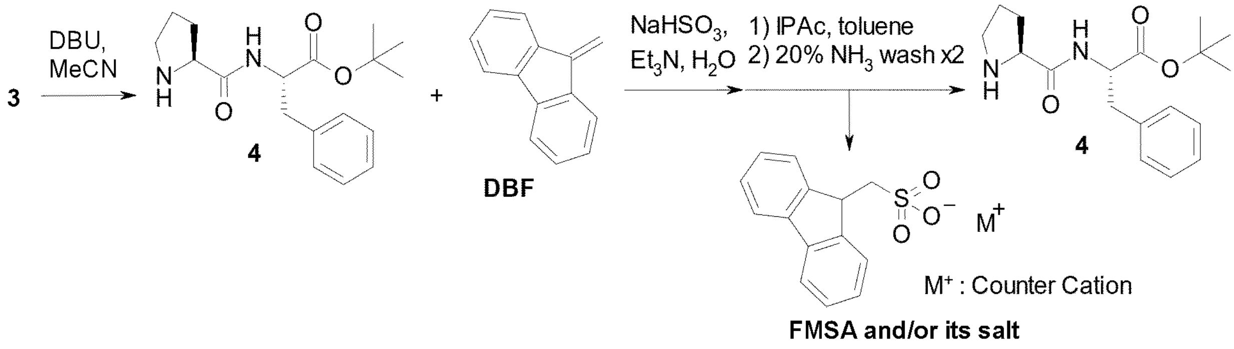


Compound 3 (95.0 mg, content 98.8 wt%, actual amount 94 mg) and MeCN (0.27 mL, 3.0 v/w) were added to the reaction vessel, and the reaction vessel was subjected to nitrogen replacement. DBU (25.9 μL, 1.0 eq.) was added at room temperature while stirring the reaction mixture, then the mixture was stirred at room temperature for 30 minutes. The reaction mixture was sampled and prepared as a sample (sample preparation method 1). The Fmoc group-deprotection reaction was evaluated by HPLC analysis, and it was confirmed that the reaction conversion rate proceeded to 99.9% or more (calculation formula of Fmoc group-deprotection reaction conversion rate). Triethylamine (96.0 μL, 4.0 eq.), water (31.3 μL, 10 eq.), and sodium bisulfite (45.6 mg, 2.5 eq.) were then sequentially added to the reaction mixture at room temperature. After stirring for 1 hour, the reaction mixture was sampled and prepared as a sample (sample preparation method 1). The DBF capture reaction was evaluated by HPLC analysis, and it was confirmed that the reaction conversion rate was 99.9% or more (calculation formula 1 of DBF capture reaction conversion rate). IPAc (0.50 mL), toluene (0.50 mL), and 20% aqueous ammonia solution (1.0 mL) were added to the reaction vessel, and the mixture was stirred for 5 minutes. The aqueous layer was drained, and then the obtained organic layer was washed again with 20% aqueous ammonia solution (1.0 mL). Sampling was performed and a sample was prepared (sample preparation method 1), and it was confirmed by HPLC analysis that the FMSA or FMSA salt residual rate was 0.39% (calculation formula of FMSA or FMSA salt residual rate). The obtained organic layer was concentrated under reduced pressure at the external temperature of 40°C. MeCN (50 μL) was added, and the mixture was subjected to the following analysis as a MeCN solution (124 mg) containing compound 4.

LC purity of compound 4: 96.2% (HPLC analysis conditions: method 1)
Content: 43.2 wt% (The obtained residue and 3,5-bis(trifluoromethyl)benzoic acid were dissolved in DMSO-$d_6$ and subjected to qNMR analysis.)
Yield: 96.7%

Examples using sodium metabisulfite ($Na_2S_2O_5$) as DBF capture agent

<u>(iii) Synthesis of compound 4: tert-butyl (2S)-3-phenyl-2-[[(2S)-pyrrolidin-2-carbonyl]amino]propanoate</u>

[Formula 15]

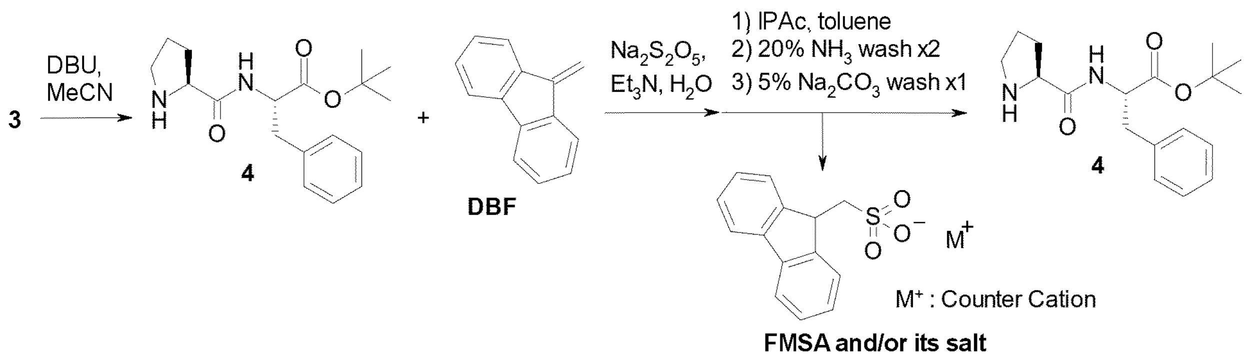


Compound 3 (200 mg, content 98.8 wt%, actual amount 198 mg) was suspended in 0.94 mL of acetonitrile, and DBU (55 μL, 1.0 eq.) was added. The reaction mixture was stirred at room temperature for 30 minutes, then the completion of the Fmoc deprotection reaction was confirmed by HPLC (calculation formula of Fmoc group-deprotection reaction

conversion rate). $Na_2S_2O_5$ (88 mg, 1.25 eq.), triethylamine (206 μL, 4.0 eq.), and water (67 μL, 10.0 eq.) were then added, and the mixture was stirred for 1 hour, and then the completion of the dibenzofulvene capture reaction was confirmed by HPLC (calculation formula 1 of DBF capture reaction conversion rate). IPAc (0.94 mL) and toluene (0.94 mL) were added to the reaction mixture. To this, 20% aqueous ammonia solution (2.0 mL) was added. The mixture was stirred for 10 minutes, then allowed to stand still, and then the aqueous layer was removed. The obtained organic layer was washed again with 20% aqueous ammonia solution. To this, 5% aqueous sodium carbonate solution (2.0 mL) was added. The mixture was stirred for 10 minutes, then allowed to stand still, and then the aqueous layer was removed. The obtained organic layer was concentrated under reduced pressure to obtain compound 4.
LC purity of compound 4: 89.5% (HPLC analysis conditions: method 2)

Example 2

Structural identification of (9H-fluoren-9-yl)methanesulfonic acid (FMSA or FMSA salt)

[Formula 16]

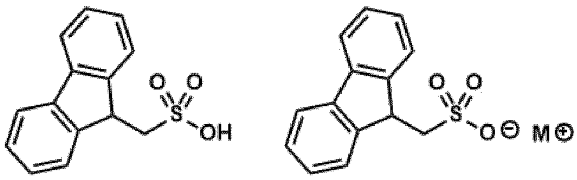

(9*H*-fluoren-9-yl)methanesulfonic acid (FMSA) and/or its salt

The aqueous layer obtained in the liquid separation washing step with 20% aqueous ammonia solution in Example 1-(iii) was lyophilized under reduced pressure to remove water and other volatile components to obtain a 1 : 1 mixture of FMSA and DBU.

LC purity (excluding DBU peak): 98.3% (HPLC analysis conditions: method 2)
1H-NMR (500MHz, D2O, excluding DBU signals) δ: 3.04 (2H, d, J=5.7 Hz), 4.03 (1H, t, J=5.7 Hz), 7.01-7.08 (4H, m), 7.24-7.26 (2H, d, J=7.4 Hz), 7.52-7.54 (2H, d, J=7.4 Hz)

Example 3

Synthesis of compound 6: tert-butyl (2S)-2-[[(2S)-1-[(2S)-2-(9H-fluoren-9-ylmethoxycarbonylamino)-3-(p-tolyl)propanoyl]pyrrolidin-2-carbonyl]amino]-3-phenyl-propanoate

[Formula 17]

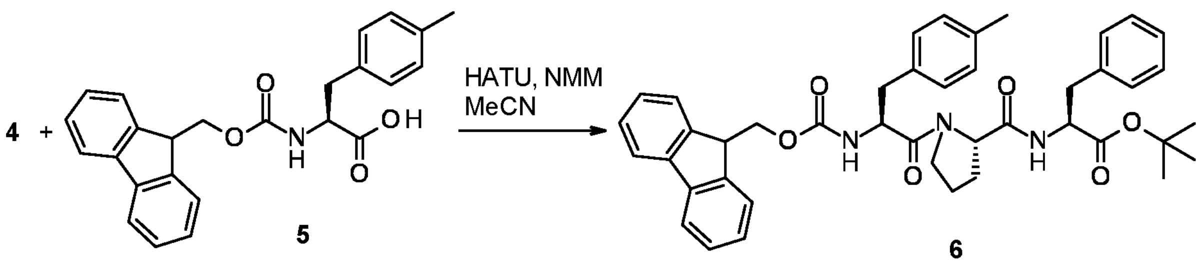


5

6

Compound 4 (177 mg) obtained in Example 1-(iii) and compound 5 (245 mg, 1.1 eq.) were dissolved in MeCN (1.0 mL) in a flask, and the reaction vessel was subjected to nitrogen replacement. NMM (183 μL, 3.0 eq.) and HATU (317 mg, 1.5 eq.) were sequentially added. After the reaction mixture was stirred at room temperature for 1 hour, the completion of the reaction was confirmed by HPLC analysis (calculation formula 1 of reaction conversion rate). Cyclopentylmethyl ether (2.0 mL), 5% aqueous sodium carbonate solution (1.0 mL), and N-methylimidazole (44.5 μL, 1.0 eq.) were sequentially

added. The reaction mixture was stirred for 1 hour, then allowed to stand still, and then the aqueous layer was removed. To the reaction vessel, 20% aqueous ammonia solution (1.0 mL) was added. After stirring for 5 minutes, the mixture was allowed to stand still, and then the aqueous layer was removed. To this, 10% aqueous sodium bisulfate solution (1.0 mL) was added. The mixture was stirred for 5 minutes, then allowed to stand still, and then the aqueous layer was removed. To this, 5% aqueous sodium carbonate solution (1.0 mL) was added. The mixture was stirred for 5 minutes, then allowed to stand still, and then the aqueous layer was removed. The obtained organic layer was concentrated under reduced pressure to obtain compound 6.
LC purity of compound 6: 92.0% (HPLC analysis conditions: method 2)

Example 4

(i) Synthesis of compound 8: (tert-butyl (3S)-3-[benzyloxycarbonyl(methyl)amino]-4-(dimethylamino)-4-oxo-butanoate)

[Formula 18]

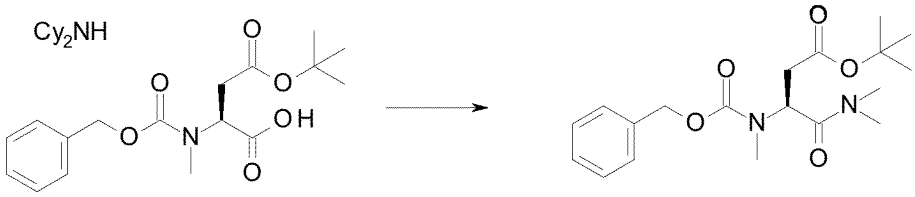

7 8

To a reaction kettle after nitrogen replacement, compound 7 (62.8 kg) and 2-MeTHF (310 kg) were sequentially added at room temperature. The external temperature of the reaction kettle was set to 10°C, and while the reaction mixture was stirred, DIPEA (89.5 kg) and a solution of dimethylamine-THF (2 M, THF solution, 71.4 kg) were added sequentially, then the mixture was stirred for 30 minutes. After T3P (50% w/w, 2-MeTHF solution, 151 kg) was added, the external temperature of the reaction kettle was set to 25°C, and the mixture was stirred for 2 hours. The reaction mixture was sampled and prepared as a sample (sample preparation method 2), and it was confirmed by HPLC analysis that the reaction conversion rate was 90.2% (calculation formula 2 of reaction conversion rate). The external temperature of the reaction kettle was set to 10 °C, and a solution of dimethylamine-THF (2 M, THF solution, 9.80 kg) was added while stirring. The external temperature of the reaction kettle was set to 25°C, and the mixture was stirred for 20 minutes. After stopping the stirring and allowing the reaction mixture to stand still for 10 hours, the reaction mixture was sampled and prepared as a sample (sample preparation method 2), and it was confirmed by HPLC analysis that the reaction conversion rate was 99.4% (calculation formula 2 of reaction conversion rate). The external temperature of the reaction kettle was set to 10°C, and 10% aqueous citric acid monohydrate solution (380 kg) was added to the reaction mixture. The external temperature of the reaction kettle was set to 25 °C, and the mixture was stirred for 10 minutes, then the stirring was stopped, and the aqueous layer was drained from the reaction kettle. The obtained organic layer was washed with 10% aqueous citric acid monohydrate solution (380 kg × 2) and 5% aqueous sodium carbonate solution (380 kg × 2). The obtained organic layer was concentrated under reduced pressure at the external temperature of 55°C until the liquid volume of the obtained organic layer reached about 220 L. 2-MeTHF (85 kg) was added, and the mixture was concentrated under reduced pressure again at the external temperature of 55°C until the liquid volume reached about 100 L to obtain a solution containing compound 8 (95.6 kg).

(ii) Synthesis of compound 9: (tert-butyl (3S)-4-(dimethylamino)-3-(methylamino)-4-oxo-butanoate)

[Formula 19]

8

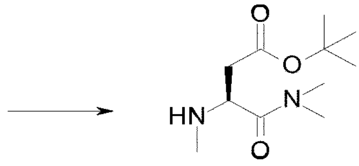

9

To a reaction kettle after nitrogen replacement, 5% Pd/C (17.9 kg, 50% aqueous content) and 2-MeTHF (85 kg) were added sequentially at room temperature. After nitrogen replacement, the reaction kettle was pressurized to 0.45 MPaG (5.5 atm) with hydrogen, and the internal temperature of the reaction kettle was set to 25°C. The mixture was stirred for 2 hours while maintaining the internal pressure pressurized to 0.40 MPaG (5.0 atm). Then, a solution (94.6 kg) containing compound 8 obtained in Example 4-(i) and 2-MeTHF (174 kg) were sequentially added at room temperature. The reaction kettle was pressurized with hydrogen until the internal pressure of the reaction kettle reached 0.18 MPaG (2.8 atm). After stirring for 1.5 hours, it was confirmed that there was no fluctuation in the internal pressure. The reaction kettle was then pressurized to 0.18 MPaG (2.8 atm) with hydrogen, and the mixture was stirred for another 0.5 hours. The reaction mixture was sampled and prepared as a sample (sample preparation method 1), and it was confirmed by HPLC analysis that the reaction conversion rate was 99.9% (calculation formula 1 of reaction conversion rate). After replacing the internal air of the reaction kettle with nitrogen, the reaction mixture was pressurized and filtered. After washing the inside of the reaction kettle and the filter with 2-MeTHF (85 kg × 2), the filtrate and the washing solution were added to the reaction kettle after nitrogen replacement. The mixture was concentrated under reduced pressure while stirring at the external temperature of 40°C until the liquid volume reached about 100 L. This concentrate solution and the washing solution obtained by washing the reaction kettle with 2-MeTHF (43 kg) were combined to obtain a solution containing compound 9 (132 kg).

(iii) Synthesis of compound 11: (tert-butyl (3S)-3-[[(2S)-2-[benzyloxycarbonyl(methyl)amino]-2-cyclopentyl-acetyl]-methyl-amino]-4-(dimethylamino)-4-oxo-butanoate)

[Formula 20]

9 +

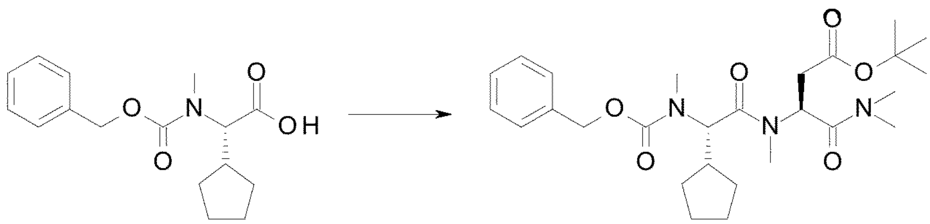

10 11

To a reaction kettle after nitrogen replacement, a solution (132 kg) containing compound 9 obtained in Example 4-(ii) was added at room temperature. The external temperature of the reaction kettle was cooled to 10°C, and acetonitrile (38 kg), compound 10 (41.5 kg), 2-MeTHF (16 kg), and DIPEA (67.7 kg) were sequentially added. HATU (67.7 kg) was then added, and then the external temperature was raised to 25°C. After the reaction mixture was stirred at 25°C for 3 hours, the reaction mixture was sampled and prepared as a sample (sample preparation method 2), and it was confirmed by HPLC analysis that the reaction conversion rate was 98.5% (calculation formula 2 of reaction conversion rate). To the reaction kettle, CPME (54 kg), 5% aqueous potassium carbonate solution (48 kg), and N-methylimidazole (9.70 kg) were sequentially added, and the mixture was stirred for 30 minutes. Then, 2.5% aqueous ammonia solution (190 kg) and 2-MeTHF (55 kg) were added, and the mixture was stirred for 10 minutes, and then the aqueous layer was drained. The obtained organic layer was washed with 2.5% aqueous ammonia solution (240 kg), 10% aqueous sodium bisulfate monohydrate solution (240 kg × 2), and 5% aqueous potassium carbonate solution (240 kg × 2). 2-MeTHF (86 kg) was added to the obtained organic layer, and the mixture was concentrated under reduced pressure while stirring at the external temperature of 60°C until the liquid volume reached about 100 L to obtain a solution containing compound 11 (98.1 kg).

(iv) Synthesis of compound 12: (tert-butyl (3S)-3-[[(2S)-2-cyclopentyl-2-(methylamino)acetyl]-methyl-amino]-4-(dimethylamino)-4-oxo-butanoate)

[Formula 21]

11

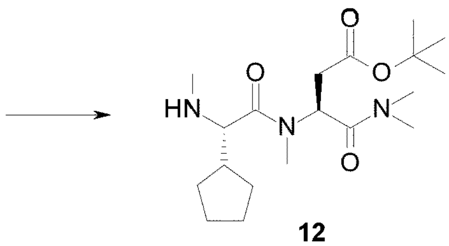

12

To a reaction kettle after nitrogen replacement, 5% Pd/C (17.5 kg, 50% aqueous content) and 2-MeTHF (85 kg) were added sequentially at room temperature. After nitrogen replacement, the reaction kettle was pressurized to 0.45 MPaG (5.5 atm) with hydrogen, and the internal temperature of the reaction kettle was set to 25°C. The mixture was stirred for 2 hours while maintaining the internal pressure pressurized to 0.40 MPaG (5.0 atm). Then, a solution (97.1 kg) containing compound 11 obtained in Example 4-(iii) and 2-MeTHF (160 kg) were sequentially added at room temperature. The external temperature of the reaction kettle was set to 25°C, and the reaction kettle was pressurized with hydrogen until the internal pressure of the reaction vessel reached 0.18 MPaG (2.8 atm). After stirring for 2 hours, it was confirmed that there was no fluctuation in the internal pressure. The reaction kettle was then pressurized to 0.18 MPaG (2.8 atm) with hydrogen, and the reaction mixture was stirred for another 1 hour. The reaction mixture was sampled and prepared as a sample (sample preparation method 1), and it was confirmed by HPLC analysis that the reaction conversion rate was 99.9% (calculation formula 1 of reaction conversion rate). The reaction kettle was then pressurized to 0.18 MPaG (2.8 atm) with hydrogen, and the reaction mixture was stirred for 1 hour. The reaction mixture was sampled and prepared as a sample (sample preparation method 1), and it was confirmed by HPLC analysis that the reaction conversion rate was 99.9% or more (calculation formula 1 of reaction conversion rate). After replacing the internal air of the reaction kettle with nitrogen, the reaction mixture was pressurized and filtered. The inside of the reaction kettle and the filter were washed with 2-MeTHF (85 kg × 2), then the obtained filtrate and the washing solution were combined and concentrated under reduced pressure while stirring at the external temperature of 60°C until the liquid volume reached about 100 L. The concentrate solution and the washing solution obtained by washing the reaction kettle with 2-MeTHF (43 kg) were combined to obtain a solution containing compound 12 (133 kg).

(v) Synthesis of compound 14: (tert-butyl (3S)-3-[[(2S) -2-cyclopentyl-2-[methyl-[1-[(2,2,2-trifluoroacetyl)amino]cyclopentanecarbonyl]amino]acetyl]-methyl-amino]-4- (dimethylamino)-4-oxo-butanoate)

[Formula 22]

12 +

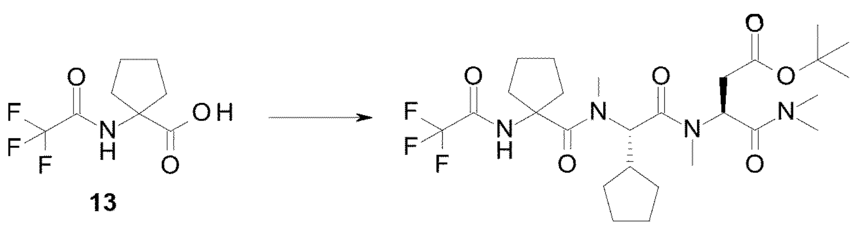

13

14

To a reaction kettle after nitrogen replacement, compound 13 (51.8 kg) and 2-MeTHF (210 kg) were sequentially added at room temperature. The external temperature of the reaction kettle was cooled to 10°C, and DIPEA (77.1 kg), a solution containing compound 12 obtained in Example 4-(iv) (133 kg), T3P (50% w/w, 2-MeTHF solution, 194 kg), and DMAP (28.1 kg) were sequentially added. The external temperature of the reaction vessel was set to 50°C, and the mixture was stirred for 5 hours. The reaction mixture was sampled and prepared as a sample (sample preparation method 2), and it was confirmed by HPLC analysis that the reaction conversion rate was 99.3% (calculation formula 2 of reaction conversion

rate). The external temperature of the reaction vessel was set to 10°C, and 5% aqueous sodium carbonate solution (350 kg) was added. The external temperature of the reaction vessel was set to 25 °C, and the mixture was stirred for 30 minutes, then the stirring was stopped, and the aqueous layer was drained from the reaction vessel. Then, 5% aqueous sodium bisulfate monohydrate solution (350 L) was added, and the mixture was stirred for 10 minutes, then the stirring was stopped and the aqueous layer was drained from the reaction vessel. The obtained organic layer was washed with 5% aqueous sodium bisulfate monohydrate solution (350 kg), and 5% aqueous sodium carbonate solution (350 kg). After the reaction mixture was transferred to the concentration kettle, the inside of the reaction kettle was washed with 2-MeTHF (86 kg). Then the reaction mixture and the washing solution were combined, and the mixture was concentrated under reduced pressure while stirring at the external temperature of 60°C until the liquid volume reached about 200 L. 2-MeTHF (63 kg) was then added, and the mixture was concentrated under reduced pressure while stirring at the external temperature of 60°C until the liquid volume reached about 200 L. Methanol (29 kg) was added to obtain a solution containing compound 14 (223 kg).

(vi) Synthesis of compound 15: (tert-butyl (3S)-3-[[(2S)-2-[(1-aminocyclopentanecarbonyl)-methyl-amino]-2-cyclopentyl-acetyl]-methyl-amino]-4-(dimethylamino)-4-oxo-butanoate)

[Formula 23]

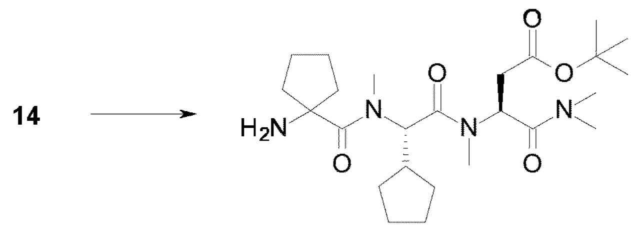

15

To a reaction vessel after nitrogen replacement, a solution (223 kg) containing compound 14 and 2-MeTHF (110 kg) were sequentially added at room temperature. The external temperature of the reaction vessel was set to -20°C. $LiBH_4$ (4 M, THF solution, 51.5 kg) was added while stirring, and then the mixture was stirred for 3 hours. $LiBH_4$ (4 M, THF solution, 0.6 kg) was added while stirring, then the reaction mixture was sampled and prepared as a sample (sample preparation method 3), and it was confirmed by HPLC analysis that the reaction conversion rate was 99.5% (calculation formula 1 of reaction conversion rate). After TFE (228 kg) was added, the external temperature of the reaction kettle was raised to 0°C over 1 hour, and the mixture was stirred at 0°C for 1 hour. Then, 20% aqueous ammonium chloride solution (200 kg) was added dropwise over 2 hours. The mixture was stirred at the external temperature of 25°C for 30 minutes, then the stirring was stopped, and the aqueous layer was drained from the reaction vessel. The external temperature of the reaction vessel was set to 10°C, and trifluoroacetic acid (26.0 kg) was added. The external temperature of the reaction vessel was set to 25°C, and the mixture was stirred for 1 hour. The obtained reaction mixture and the washing solution obtained by washing the reaction kettle with 2-MeTHF (84.7 kg × 2) were combined to obtain a mixture. To another reaction kettle after nitrogen replacement, 2M aqueous sodium hydroxide solution (630 kg) was added at room temperature, and the external temperature of the reaction vessel was set to 10°C. To this, the above-described mixture was added dropwise over 1.5 hours, and then the external temperature of the reaction vessel was set to 25°C. The mixture was stirred for 10 minutes, then the stirring was stopped, and the aqueous layer was drained from the reaction vessel. The obtained organic layer was washed with 2M aqueous sodium hydroxide solution (630 kg × 3) and 10% aqueous dipotassium hydrogen phosphate solution (330 kg). The obtained organic layer was concentrated under reduced pressure at the external temperature of 40°C until the liquid volume reached about 220 L. Again, 2-MeTHF (85 kg) was added, and then the mixture was concentrated under reduced pressure at the external temperature of 40°C until the liquid volume reached about 100 L. This concentration procedure was repeated 10 times, and the concentrate solution and the washing solution obtained by washing the reaction kettle with acetonitrile (56.2 kg) were combined to obtain a solution containing compound 15 (151 kg).

(vii) Synthesis of compound 17: (benzyl (2S)-2-[[1-[[(1S)-2-[[(1S)-3-tert-butoxy-1-(dimethylcarbamoyl)-3-oxo-propyl]-methyl-amino]-1-cyclopentyl-2-oxo-ethyl]-methylcarbamoyl]cyclopentyl]carbamoyl]pyrrolidine-1-carboxylate)

[Formula 24]

15 +

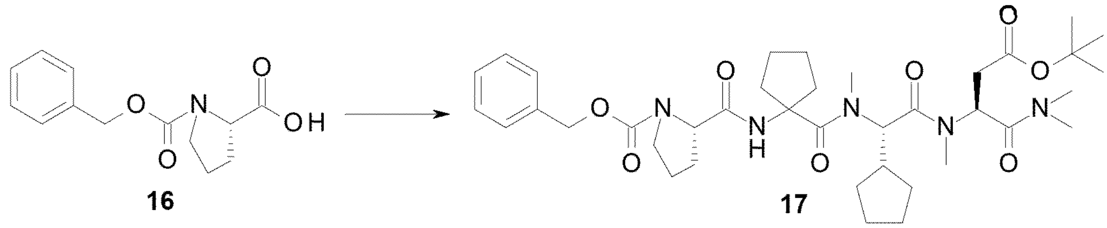

16

17

The external temperature of the reaction vessel after nitrogen replacement was set to 10°C, then a solution containing compound 15 obtained in Example 4-(vi) (94.8 kg), compound 16 (36.6 kg), and acetonitrile (157 kg) were sequentially added. Then, DIPEA (43.8 kg) and 2-bromo-1-ethylpyridinium tetrafluoroborate (47.1 kg) were sequentially added. The external temperature of the reaction vessel was set to 25°C, and the mixture was stirred for 1 hour. The reaction mixture was sampled and prepared as a sample (sample preparation method 1), and it was confirmed by HPLC analysis that the reaction conversion rate was 99.6% (calculation formula 2 of reaction conversion rate). The external temperature of the reaction vessel was set to 10°C, and CPME (470 kg), 5% aqueous potassium carbonate solution (320 kg), and N-methylimidazole (9.40 kg) were sequentially added. The external temperature of the reaction vessel was set to 25°C, and the mixture was stirred for 40 minutes, then the aqueous layer was drained from the reaction vessel. The obtained organic layer was washed with 5% aqueous sodium bisulfate monohydrate solution (320 kg×2), and 5% aqueous sodium carbonate solution (320 kg×2). The obtained organic layer was concentrated under reduced pressure at the external temperature of 60°C until the liquid volume reached about 500 L. Again, 2-MeTHF (86 kg) was added, and the mixture was concentrated under reduced pressure at the external temperature of 40°C until the liquid volume reached about 200 L to obtain a solution containing compound 17 (176 kg).

(viii) Synthesis of compound 18: (tert-butyl (3S)-3-[[(2S)-2-cyclopentyl-2-[methyl-[1-[[(2S)-pyrrolidin-2-carbonyl]amino]cyclopentanecarbonyl]amino]acetyl]-methyl-amino]-4-(dimethylamino)-4-oxo-butanoate)

[Formula 25]

17

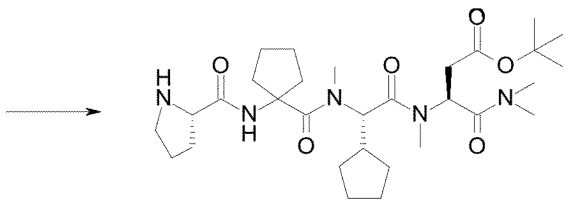

18

To a reaction kettle after nitrogen replacement, 5% Pd/C (16.7 kg, 50% aqueous content) and THF(90 kg) were added sequentially at room temperature. After nitrogen replacement, the reaction kettle was pressurized to 0.45 MPaG (5.5 atm) with hydrogen, and the internal temperature of the reaction kettle was set to 25°C. The mixture was stirred for 2 hours while maintaining the internal pressure pressurized to 0.40 MPaG (5.0 atm). A solution (176 kg) containing compound 17 obtained in Example 4-(vii) and THF(230 kg) were sequentially added at room temperature. The external temperature of the reaction vessel was set to 25°C, and the reaction vessel was pressurized with hydrogen until the internal pressure thereof reached 0.18 MPaG (2.8 atm). After 4 hours, it was confirmed that there was no fluctuation in the internal pressure. Then, the reaction vessel after nitrogen replacement was pressurized to 0.18 MPaG (2.8 atm) with hydrogen, and the reaction mixture was stirred for another 1 hour. The reaction mixture was sampled and prepared as a sample (sample preparation method 1), and it was confirmed by HPLC analysis that the reaction conversion rate was 99.6% (calculation formula 1 of reaction conversion rate). After replacing the internal air of the reaction vessel with nitrogen, the reaction mixture was pressurized and filtered. The reaction vessel and the filter were washed with 2-MeTHF (85 kg × 2). The obtained filtrate and the washing solution were concentrated under reduced pressure at the external temperature of 50°C until the liquid volume reached about 600 L. Again, 2-MeTHF (160 kg) was added, and then the mixture was concentrated under reduced pressure at the external temperature of 50°C until the liquid volume reached about 230 L. This concentration procedure was repeated 3 times. Then, the external temperature of the reaction kettle was

set to 50°C. Heptane (100 kg) was added, and then the seed crystal (116 g) of compound 18 and heptane (5.3 kg) were sequentially added. The mixture was stirred for 2 hours, then heptane (85 L) was added dropwise over 15 minutes, and the mixture was stirred again for 2 hours. Heptane (470 L) was further added dropwise over 75 minutes and the mixture was stirred for 17 hours. The external temperature of the reaction kettle was cooled to 22°C over 2 hours, and the mixture was stirred for 20 hours. The internal temperature of the reaction kettle was cooled to 10°C over 1 hour, and the mixture was stirred for 1 hour. The obtained slurry was filtered, and the obtained crystal was washed with a mixed solution of heptane (170 kg) and 2-MeTHF (17 kg). Further, the crystal was washed with heptane (180 kg), and the obtained crystal was dried under reduced pressure at 40°C for 3 hours to obtain compound 18 (34.5 kg). The seed crystal of compound 18 was obtained according to the method described in Example 70 of International Publication No. WO 2023/127869.

(ix) Synthesis of compound 20: tert-butyl (3S)-3-[[(2S)-2-cyclopentyl-2-[[1-[[(2S)-1-[(2S)-2-[9H-fluoren-9-ylmethoxycarbonyl(methyl)amino]propanoyl]pyrrolidin-2-carbonyl]amino]cyclopentanecarbonyl]-methyl-amino]acetyl]-methylamino]-4-(dimethylamino)-4-oxo-butanoate

[Formula 26]

18 +

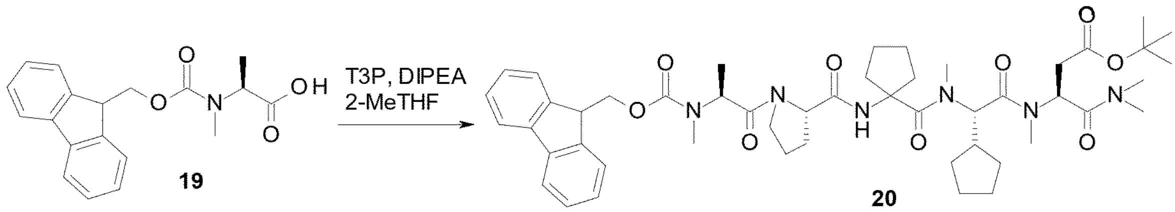


19

20

Compound 19 (0.931 g) and compound 18 (1.82 g, 1.1 eq.) were added to the reaction vessel, and the reaction vessel was subjected to nitrogen replacement. 2-MeTHF (12 mL, 12 v/w) and DIPEA (1.70 mL, 3.4 eq.) were then sequentially added at room temperature. T3P (50 w/w% 2-MeTHF solution, 2.68 mL, 1.5 eq.) was added while stirring the reaction mixture, then the mixture was stirred at room temperature for 1 hour. The reaction mixture was sampled and prepared as a sample (sample preparation method 1), and it was confirmed by HPLC analysis that the reaction conversion rate was 99.9% or more (calculation formula 1 of reaction conversion rate). To the reaction vessel, 5% aqueous sodium carbonate solution (15 mL) was added, and the mixture was stirred for 5 minutes. After draining the aqueous layer, the obtained organic layer was washed with 5% aqueous sodium bisulfate monohydrate solution (15 mL × 4), 5% aqueous sodium carbonate solution (15 mL), and 5% aqueous sodium chloride solution (15 mL). The obtained organic layer was dehydrated with sodium sulfate, and then sodium sulfate was removed by filtration. After concentration under reduced pressure at the external temperature of 40°C, a residue (2.42 g) containing compound 20 was obtained.

LC purity of compound 20: 99.2% (HPLC analysis conditions: method 1)
Content: 91.7 wt% (The obtained residue and 3,5-bis(trifluoromethyl)benzoic acid were dissolved in DMSO-$d_6$ and subjected to qNMR analysis.)

(x) Synthesis of compound 21: tert-butyl (3S)-3-[[(2S)-2-cyclopentyl-2-[methyl-[1-[[(2S)-1-[(2S)-2-(methylamino)propanoyl]pyrrolidin-2-carbonyl]amino]cyclopentanecarbonyl]amino]acetyl]-methyl-amino]-4-(dimethylamino)-4-oxo-butanoate

[Formula 27]

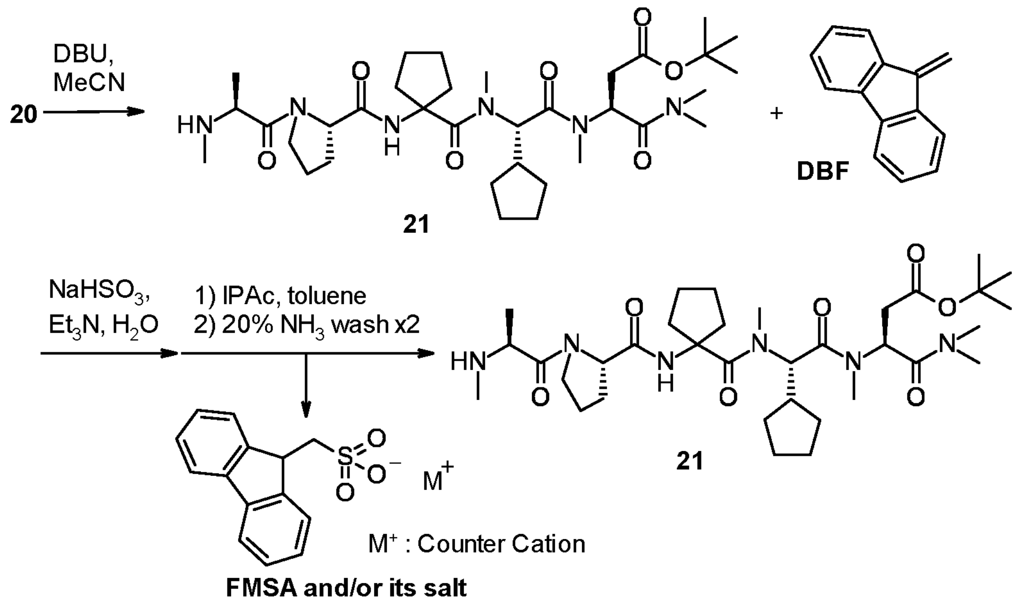


Compound 20 (168 mg, content 91.7 wt%, actual amount 154 mg) and MeCN (0.31 mL, 2.0 v/w) were added to the reaction vessel, and the reaction vessel was subjected to nitrogen replacement. DBU (25.9 μL, 1.0 eq.) was added at room temperature while stirring the reaction mixture, then the mixture was stirred at room temperature for 30 minutes. The reaction mixture was sampled and prepared as a sample (sample preparation method 1). The Fmoc group-deprotection reaction was evaluated by HPLC analysis, and it was confirmed that the reaction conversion rate proceeded to 99.9% or more (calculation formula of Fmoc group-deprotection reaction conversion rate). Triethylamine (96.0 μL, 4.0 eq.), water (31.3 μL, 10 eq.), and sodium bisulfite (44.5 mg, 2.5 eq.) were then sequentially added to the reaction mixture at room temperature. After stirring for 1 hour, the reaction mixture was sampled and prepared as a sample (sample preparation method 1). The DBF capture reaction was evaluated by HPLC analysis, and it was confirmed that the reaction conversion rate was 99.9% or more (calculation formula 1 of DBF capture reaction conversion rate). IPAc (0.75 mL), toluene (0.75 mL), and 20% aqueous ammonia solution (1.5 mL) were added to the reaction vessel, and the mixture was stirred for 5 minutes. The aqueous layer was drained, and then the obtained organic layer was washed again with 20% aqueous ammonia solution (1.5 mL). The layer was sampled and prepared as a sample (sample preparation method 1), and it was confirmed by HPLC analysis that the FMSA or FMSA salt residual rate was 0.33% (calculation formula of FMSA or FMSA salt residual rate). The obtained organic layer was concentrated under reduced pressure at the external temperature of 40°C. MeCN (100 μL) was added, and the mixture was subjected to the following analysis as a MeCN solution (211 mg) containing compound 21.

LC purity of compound 21: 97.9% (HPLC analysis conditions: method 1)
Content: 40.6 wt% (The obtained residue and 3,5-bis(trifluoromethyl)benzoic acid were dissolved in DMSO-$d_6$ and subjected to qNMR analysis.)
Yield: 74.3%

Example 5

(i) Synthesis of compound 23: tert-butyl N-((S)-2-((S)-1-(N-N-((S)-3-((S)-2-(1-((S)-1-((S)-2-amino-4-(3,5-difluoro-4-(trifluoromethyl)phenyl)butanoyl)pyrrolidine-2-carboxamide)-N-methylcyclopentane-1-carboxamide)-2-cyclopentyl-N-methylacetamide)-4-(dimethylamino)-4-oxobutanoyl)-N-methyl-L-leucyl-L-isoleucyl-N-methyl-L-alanyl)-N-ethylazetidine-2-carboxamide)-3-(p-tolyl)propanoyl)-N-methylglycinate

[Formula 28]

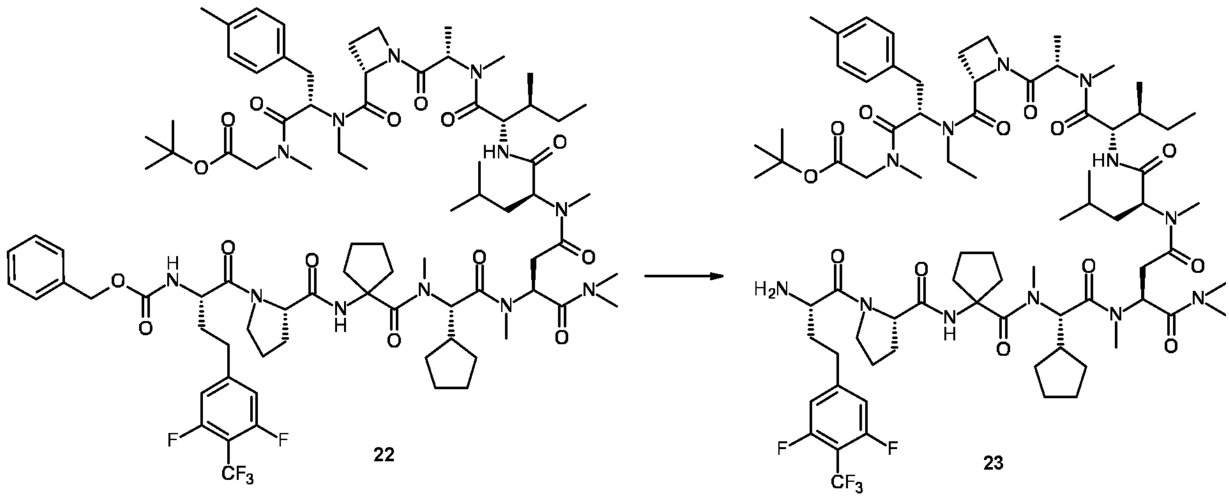

22

23

To a reaction vessel after nitrogen replacement, 10% Pd/C (425.1 mg, 50% aqueous content) and 2-MeTHF (6.0 mL) were added sequentially at room temperature. After nitrogen replacement, the reaction vessel was pressurized to 0.40 MPaG (5.0 atm) with hydrogen, and the internal temperature of the reaction vessel was set to 25°C. The mixture was stirred for 30 minutes while maintaining the internal pressure pressurized to 0.40 MPaG (5.0 atm). A solution containing compound 22 synthesized according to the method described in Example 21 of International Publication No. WO 2022/234864 (7.22 g, content 42.4 wt%, actual amount 3.06 g) and 2-MeTHF (6.0 mL) were added sequentially at room temperature. The external temperature of the reaction vessel was set to 25°C, and the reaction vessel was pressurized with hydrogen until the internal pressure thereof reached 0.20 MPaG (3.0 atm). After 1 hour and 20 minutes, it was confirmed that there was no fluctuation in the internal pressure. Then, the reaction vessel after nitrogen replacement was pressurized to 0.20 MPaG (3.0 atm) with hydrogen, and the reaction mixture was stirred for another 2 hours and 30 minutes. The reaction mixture was sampled and prepared as a sample (sample preparation method 1), and it was confirmed by HPLC analysis that the reaction conversion rate was 97.3% (calculation formula 1 of reaction conversion rate). After replacing the internal air of the reaction vessel with nitrogen, the reaction mixture was pressurized and filtered. The reaction vessel and the filter were washed with 2-MeTHF (12 mL × 3). The resulting filtrate and the washing solution were concentrated under reduced pressure at the external temperature of 30°C, and the resulting residue was purified by silica gel chromatography (eluent: MeOH/dichloromethane 0 : 100 to 10 : 90). The eluate containing compound 23 was concentrated under reduced pressure at 30°C to obtain compound 23 (2.49 g). LC purity of compound 23: 98.5% (HPLC analysis conditions: method 1)

(ii) Synthesis of compound 25: tert-butyl N-((S)-2-((S)-1-(N-N-((S)-3-((S)-2-(1-((S)-1-((S)-2-((S)-2-((((9H-fluoren-9-yl) methoxy)carbonyl)(methyl)amino)-3-methylbutanamide)-4-(3,5-difluoro-4-(trifluoromethyl)phenyl)butanoyl)pyrrolidine-2-carboxamide)-N-methylcyclopentane 1-carboxamide)-2-cyclopentyl-N-methylacetamide)-4-(dimethylamino)-4-oxobutanoyl)-N-methyl-L-leucyl-L-isoleucyl-N-methyl-L-alanyl)-N-ethylazetidine-2-carboxamide)-3-(p-tolyl)propanoyl)-N-methylglycinate

[Formula 29]

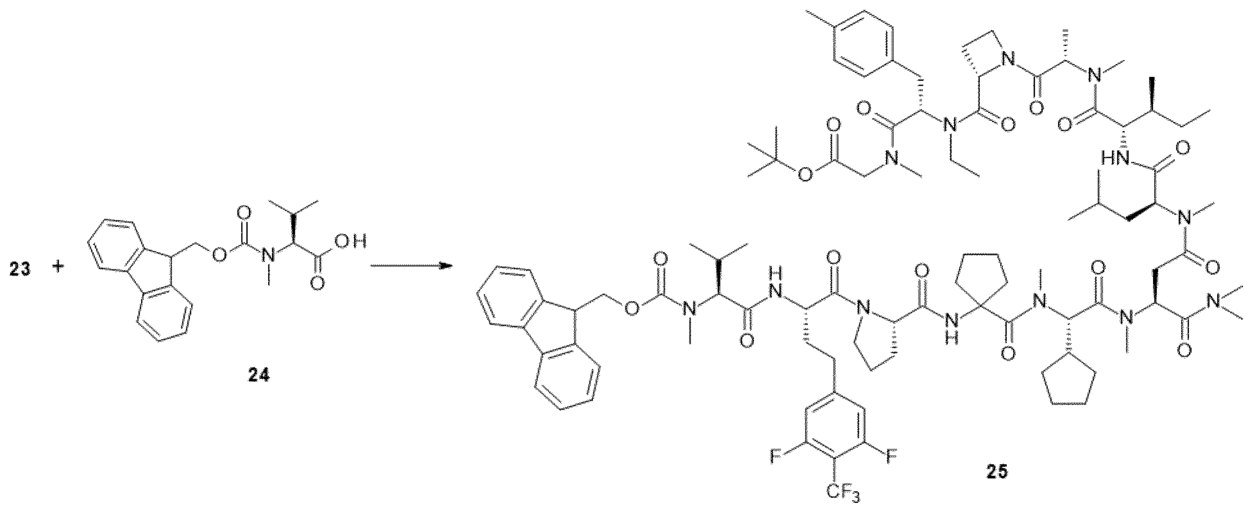

Compound 23 (1.00 g) obtained in Example 5-(i), compound 24 (281 mg), 2-MeTHF (3.0 mL), acetonitrile (3.0 mL), and DIPEA (0.578 mL) were added sequentially. The reaction vessel was then subjected to nitrogen replacement, and HATU (378 mg) was added. After the external temperature was set to 25°C and the reaction mixture was stirred for 3 hours, the reaction mixture was sampled and prepared as a sample (sample preparation method 1), and it was confirmed by HPLC analysis that the reaction conversion rate was 96.1% (calculation formula 1 of reaction conversion rate). 2-MeTHF (3.0 mL), 5% aqueous potassium carbonate solution (6.0 mL), and N-methylimidazole (52.9 μL) were sequentially added, and the mixture was stirred for 30 minutes, and then the aqueous layer was drained. The obtained organic layer was washed with 10% aqueous ammonia solution (6.0 mL × 2), 5% aqueous sulfuric acid solution (6.0 mL), and 5% aqueous potassium carbonate solution (6.0 mL). The obtained organic layer was concentrated under reduced pressure at the external temperature of 40°C, and the obtained residue was purified by silica gel chromatography (eluent: MeOH/ethyl acetate 0 : 100 to 8 : 92). The eluate containing compound 25 was concentrated under reduced pressure at 30°C to obtain compound 25 (1.04 g).

LC purity of compound 25: 95.6% (HPLC analysis conditions: method 1)
Content: 95.8 wt% (The obtained residue and 3,5-bis(trifluoromethyl)benzoic acid were dissolved in DMSO-$d_6$ and subjected to qNMR analysis.)

(iii) Synthesis of compound 26: tert-butyl N-((S)-2-((S)-1-(N-N-((S)-3-((S)-2-cyclopentyl-2-(1-((S)-1-((S)-4-(3,5-difluoro-4-(trifluoromethyl)phenyl)-2-((S)-3-methyl-2-methylamino)butanamide)butanoyl)pyrrolidine-2-carboxamide)-N-methylcyclopentane-1-carboxamide)-N-methylacetamide)-4-(dimethylamino)-4-oxobutanoyl)-N-methyl-L-leucyl-L-isoleucyl-N-methyl-L-alanyl)-N-ethylazetidine-2-carboxamide)-3-(p-tolyl)propanoyl)-N-methylglycinate

[Formula 30]

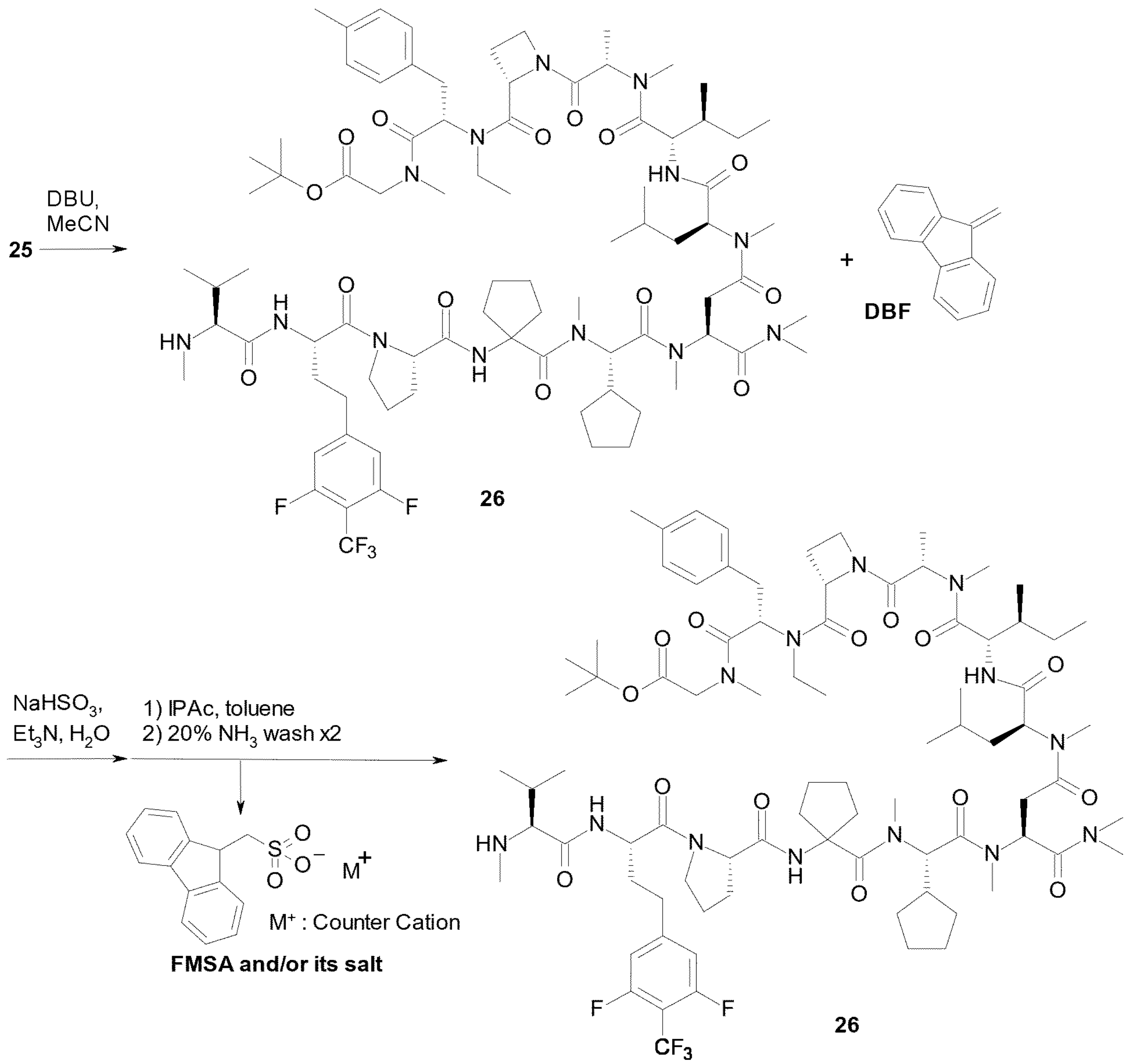


Compound 25 (300 mg, content 95.8 wt%, actual amount 287 mg) and MeCN (0.6 mL, 2.0 v/w) were added to the reaction vessel, and the reaction vessel was subjected to nitrogen replacement. DBU (22.8 μL, 1.0 eq.) was added at room temperature while stirring the reaction mixture, then the mixture was stirred at room temperature for 30 minutes. The reaction mixture was sampled and prepared as a sample (sample preparation method 1). The Fmoc group-deprotection reaction was evaluated by HPLC analysis, and it was confirmed that the reaction conversion rate was completed (the completion means that the peak of Fmoc-deprotected compound is confirmed while the peak of Fmoc-protected compound is not confirmed. The same applies hereinafter.) (calculation formula of Fmoc group-deprotection reaction conversion rate). Triethylamine (106 **μL,** 5.0 eq.), water (27.5 **μL,** 10 eq.), and sodium bisulfite (55.5 mg, 3.5 eq.) were then sequentially added to the reaction mixture at room temperature. After stirring for 1 hour and 30 minutes, the reaction mixture was sampled and prepared as a sample (sample preparation method 1). The DBF capture reaction was evaluated by HPLC analysis, and it was confirmed that the reaction conversion rate was 99.9% or more (calculation formula 1 of DBF capture reaction conversion rate). IPAc (1.5 mL), toluene (1.5 mL), and 20% aqueous ammonia solution (3.0 mL) were added to the reaction vessel, and the mixture was stirred for 5 minutes. The aqueous layer was drained, and then the obtained organic layer was washed again with 20% aqueous ammonia solution (3.0 mL). The layer was sampled and prepared as a sample (sample preparation method 1), and it was confirmed by HPLC analysis that the FMSA or FMSA salt residual rate was 0.11% (calculation formula of FMSA or FMSA salt residual rate). The obtained organic layer was concentrated under reduced pressure at the external temperature of 40°C to obtain a residue (261 mg) containing compound 26, which was subjected to the following analysis.

LC purity of compound 26: 98.5% (HPLC analysis conditions: method 1)
Content: 95.7 wt% (The obtained residue and 3,5-bis(trifluoromethyl)benzoic acid were dissolved in DMSO-$d_6$ and subjected to qNMR analysis.)
Yield: 98.9%

Example 6

(i) Synthesis of compound 28: tert-butyl (S)-2-((((9H-fluoren-9-yl)methoxy)carbonyl)amino)-3-(4-iodophenyl)propanoate

[Formula 31]

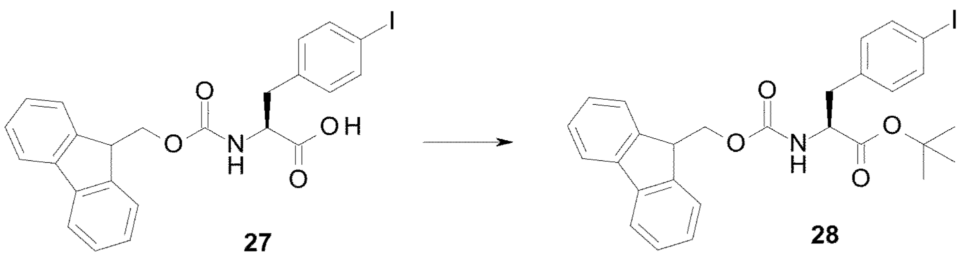

27

28

The internal air of a reaction vessel was replaced with nitrogen, and compound 27 (2.00 g) was added to the reaction vessel. Cyclohexane (10 mL), dichloromethane (4 mL), and tert-butyl 2,2,2-trichloroacetimidate (1.75 mL) were then added to the reaction vessel. Boron trifluoride diethyl ether complex (98.8 μL) was added and the mixture was stirred at room temperature for 45 minutes. Then, boron trifluoride diethyl ether complex (98.8 μL) was added, and then the mixture was stirred at room temperature for 1 hour and 5 minutes. Furthermore, tert-butyl 2,2,2-trichloroacetimidate (1.05 mL) was added, and then the mixture was stirred at room temperature for 1 hour. The reaction mixture was sampled and prepared as a sample (sample preparation method 1), and it was confirmed by HPLC analysis that the reaction conversion rate was 81.7% (calculation formula 1 of reaction conversion rate). Triethylamine (0.217 mL) was added, and the resulting slurry was filtered and the solid residue was washed with cyclohexane (20 mL × 3). The resulting filtrate was concentrated under reduced pressure at the external temperature of 30°C, and the resulting residue was purified by silica gel chromatography (eluent: ethyl acetate/heptane 10 : 90 to 39 : 61). The eluate containing compound 28 was concentrated under reduced pressure at 30°C to obtain compound 28 (1.60 g).

LC purity of compound 28: 99.6% (HPLC analysis conditions: method 1)
Content: 96.9 wt% (The obtained residue and 3,5-bis(trifluoromethyl)benzoic acid were dissolved in DMSO-$d_6$ and subjected to qNMR analysis.)
Yield: 70.1%

(ii) Synthesis of compound 29: tert-butyl (S)-2-amino-3-(4-iodophenyl)propanoate

[Formula 32]

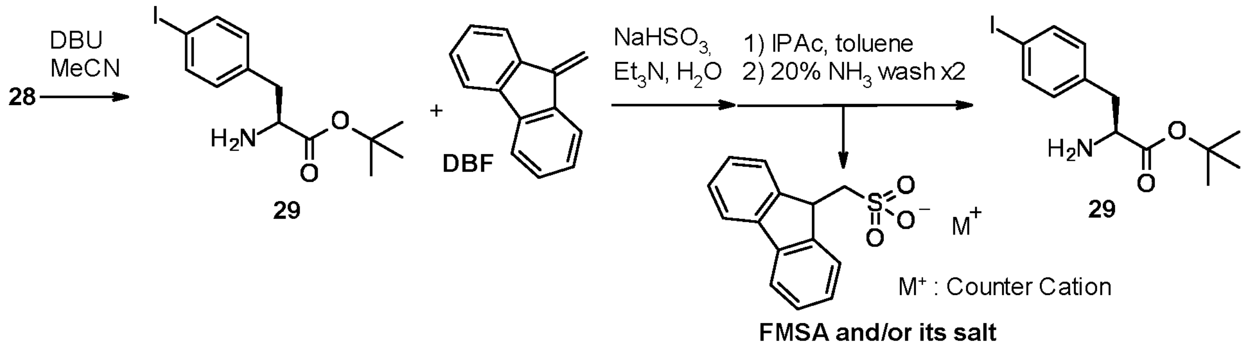


Compound 28 (300 mg, content 96.9 wt%, actual amount 291 mg) and MeCN (0.57 mL, 2 v/w) were added to a reaction vessel, and the reaction vessel was subjected to nitrogen replacement. DBU (74.8 μL, 1.0 eq.) was added at room temperature while stirring the reaction mixture, then the mixture was stirred at room temperature for 30 minutes. The reaction mixture was sampled and prepared as a sample (sample preparation method 1). The Fmoc group-deprotection reaction was evaluated by HPLC analysis, and it was confirmed that the reaction was completed (calculation formula of Fmoc group-deprotection reaction conversion rate). Triethylamine (279 μL, 4.0 eq.), water (90.1 μL, 10 eq.), and sodium bisulfite (130 mg, 2.5 eq.) were then sequentially added to the reaction mixture at room temperature. After stirring for 1 hour, the reaction mixture was sampled and prepared as a sample (sample preparation method 1) The DBF capture reaction was evaluated by HPLC analysis, and it was confirmed that the reaction conversion rate was 99.9% (calculation formula 1 of DBF capture reaction conversion rate). IPAc (1.5 mL), toluene (1.5 mL), and 20% aqueous ammonia solution (3.0 mL) were added to the reaction vessel, and the mixture was stirred for 5 minutes. The aqueous layer was drained, and then the obtained organic layer was washed again with 20% aqueous ammonia solution (3.0 mL). The layer was sampled and prepared as a sample (sample preparation method 1), and it was confirmed by HPLC analysis that the FMSA or FMSA salt residual rate was 1.09% (calculation formula of FMSA or FMSA salt residual rate). The obtained organic layer was concentrated under reduced pressure at the external temperature of 40°C to obtain a residue (175 mg) containing compound 29, which was subjected to analysis.

LC purity of compound 29: 93.3% (HPLC analysis conditions: method 1)
Content: 93.7 wt% (The obtained residue and 3,5-bis(trifluoromethyl)benzoic acid were dissolved in DMSO-$d_6$ and subjected to qNMR analysis.)
Yield: 92.7%

Example 7

Synthesis of compound 31: 5-methyl-4-phenylthiazol-2-amine

[Formula 33]

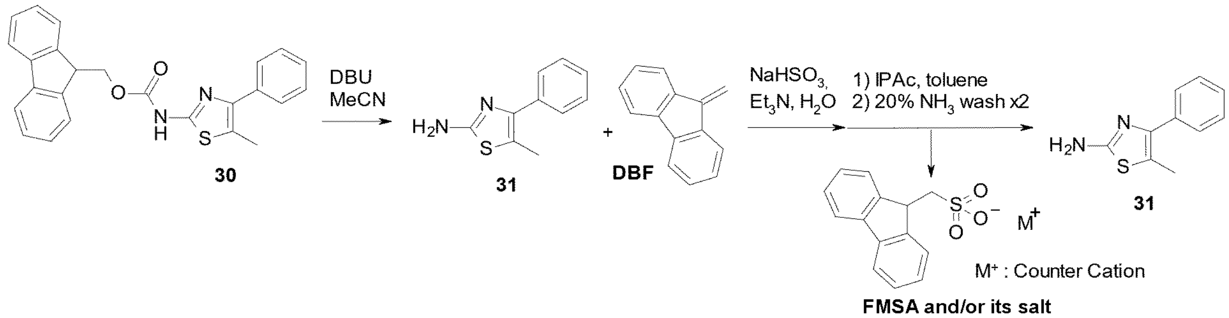


Compound 30 (300 mg) and MeCN (0.60 mL, 2 v/w) were added to a reaction vessel, and the reaction vessel was subjected to nitrogen replacement. DBU (109 μL, 1.0 eq.) was added at room temperature while stirring the reaction mixture, then the mixture was stirred at room temperature for 30 minutes. The reaction mixture was sampled and prepared

as a sample (sample preparation method 1). The Fmoc group-deprotection reaction was evaluated by HPLC analysis, and it was confirmed that the reaction was completed (calculation formula of Fmoc group-deprotection reaction conversion rate). Triethylamine (406 μL, 4.0 eq.), water (131 μL, 10 eq.), and sodium bisulfite (190 mg, 2.5 eq.) were then sequentially added to the reaction mixture at room temperature. After stirring for 1 hour, the reaction mixture was sampled and prepared as a sample (sample preparation method 1). The DBF capture reaction was evaluated by HPLC analysis, and it was confirmed that the reaction conversion rate was 99.8% (calculation formula 1 of DBF capture reaction conversion rate). IPAc (1.50 mL), toluene (1.50 mL), and 20% aqueous ammonia solution (3.0 mL) were added to the reaction vessel, and the mixture was stirred for 5 minutes. The aqueous layer was drained, and then the obtained organic layer was washed again with 20% aqueous ammonia solution (1.0 mL). The layer was sampled and prepared as a sample (sample preparation method 1), and it was confirmed by HPLC analysis that the FMSA or FMSA salt residual rate was 0.65% (calculation formula of FMSA or FMSA salt residual rate). The obtained organic layer was concentrated under reduced pressure at the external temperature of 40°C to obtain a residue (130 mg) containing compound 31, which was subjected to analysis.

LC purity of compound 31: 95.1% (HPLC analysis conditions: method 1)
Content: 80.4 wt% (The obtained residue and 3,5-bis(trifluoromethyl)benzoic acid were dissolved in DMSO-$d_6$ and subjected to qNMR analysis.)
Yield: 75.7%

Example 8

(i) Synthesis of compound 33: tert-butyl (2S)-2-[[(2S)-2(9H-fluoren-9-ylmethoxycarbonylamino)pent-4-enoyl]amino]-3-phenyl-propanoate

[Formula 34]

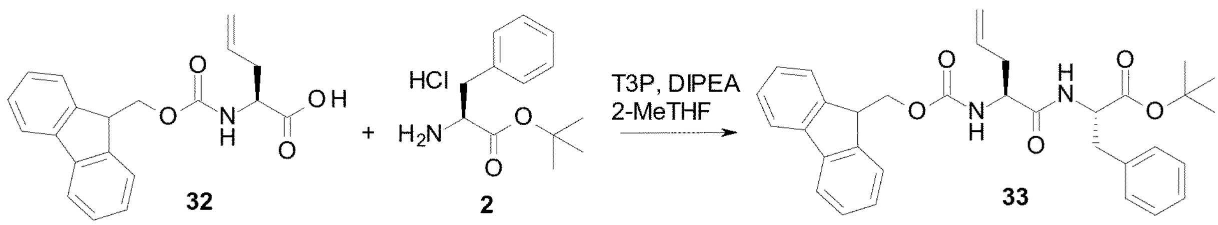


Compound 32 (0.964 g) and compound 2 (0.809 g, 1.1 eq.) were added to the reaction vessel, and the reaction vessel was subjected to nitrogen replacement. To the reaction mixture, 2-MeTHF (12 mL, 12 v/w) and DIPEA (1.70 mL, 3.4 eq.) were then sequentially added at room temperature. T3P (50 w/w% 2-MeTHF solution, 2.68 mL, 1.5 eq.) was added while stirring, then the mixture was stirred at room temperature for 1 hour. The reaction mixture was sampled and prepared as a sample (sample preparation method 1), and it was confirmed by HPLC analysis that the reaction conversion rate was 99.9% (calculation formula 1 of reaction conversion rate). To the reaction vessel, 5% aqueous sodium carbonate solution (15 mL) was added, and the mixture was stirred for 5 minutes. After draining the aqueous layer, the obtained organic layer was washed with 5% aqueous sodium bisulfate monohydrate solution (15 mL × 2), 5% aqueous sodium carbonate solution (15 mL), and 5% aqueous sodium chloride solution (15 mL). The obtained organic layer was dehydrated with sodium sulfate, and then sodium sulfate was removed by filtration. After concentration under reduced pressure at the external temperature of 40°C, a residue (0.794 g) containing compound 33 was obtained.

LC purity of compound 33: 99.3% (HPLC analysis conditions: method 1)
Content: 93.8 wt% (The obtained residue and 3,5-bis(trifluoromethyl)benzoic acid were dissolved in DMSO-$d_6$ and subjected to qNMR analysis.)

(ii) Synthesis of compound 34: tert-butyl (2S)-2-[[(2S)-2-aminopent-4-enoyl]amino]-3-phenyl-propanoate

[Formula 35]

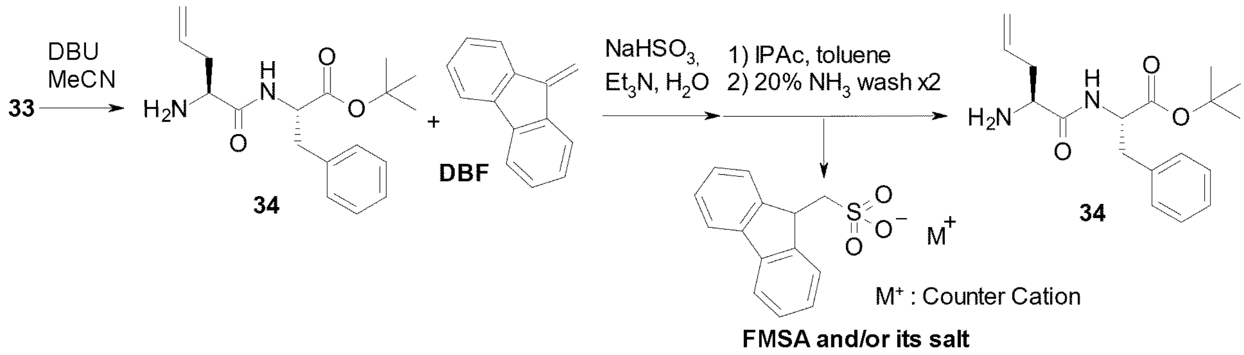


33, 34, DBF, 34

FMSA and/or its salt

Compound 33 (100 mg, content 93.8%, actual amount 94 mg) and MeCN (0.18 mL, 2 v/w) were added to the reaction vessel, and the reaction vessel was subjected to nitrogen replacement. DBU (25.9 μL, 1.0 eq.) was added at room temperature while stirring the reaction mixture, then the mixture was stirred at room temperature for 30 minutes. The reaction mixture was sampled and prepared as a sample (sample preparation method 1). The Fmoc group-deprotection reaction was evaluated by HPLC analysis, and it was confirmed that the reaction conversion rate proceeded to 99.9% or more (calculation formula of Fmoc group-deprotection reaction conversion rate). Triethylamine (96.0 μL, 4.0 eq.), water (31.3 μL, 10 eq.), and sodium bisulfite (45.4 mg, 2.5 eq.) were then sequentially added to the reaction mixture at room temperature. After stirring for 1 hour, the reaction mixture was sampled and prepared as a sample (sample preparation method 1). The DBF capture reaction was evaluated by HPLC analysis, and it was confirmed that the reaction conversion rate was 99.9% or more (calculation formula 1 of DBF capture reaction conversion rate). IPAc (0.50 mL), toluene (0.50 mL), and 20% aqueous ammonia solution (1.0 mL) were added to the reaction vessel, and the mixture was stirred for 5 minutes. The aqueous layer was drained, and then the obtained organic layer was washed again with 20% aqueous ammonia solution (1.0 mL). The layer was sampled and prepared as a sample (sample preparation method 1), and it was confirmed by HPLC analysis that the FMSA or FMSA salt residual rate was 0.30% (calculation formula of FMSA or FMSA salt residual rate). The obtained organic layer was concentrated under reduced pressure at the external temperature of 40°C. MeCN (50 μL) was added, and the mixture was subjected to the following analysis as a MeCN solution (121 mg) containing compound 34.

LC purity of compound 34: 99.1% (HPLC analysis conditions: method 1)
Content: 44.9 wt% (The obtained residue and 3,5-bis(trifluoromethyl)benzoic acid were dissolved in DMSO-$d_6$ and subjected to qNMR analysis.)
Yield: 98.6%

Example 9

Examination of reaction conditions

Using compound 33, Fmoc group-deprotection and subsequent DBF capture reaction were carried out under the reaction conditions shown in the table below in the same procedure as in Examples 8-(ii), and the Fmoc group-deprotection rate and the DBF capture rate were measured (calculation formula of Fmoc group-deprotection reaction conversion rate, calculation formula 1 of DBF capture reaction conversion rate).

Example 9-1 was performed with increase of the molar equivalent of each reagent, and it was confirmed that the reaction conversion rate of DBF capture reaction was 99.9% or more.

In Example 9-2 where triethylamine was not added, the reaction conversion rate of the DBF capture reaction was decreased to 15.9%. However, in Example 9-3 where the reaction temperature was raised to 50°C, the conversion rate was increased to 88.9% after 6 hours from the start of the reaction, and in Example 9-4 where the temperature was raised to 80°C, the conversion rate of 100% was confirmed after 6 hours from the start of the reaction.

Examples 9-5, 9-6, 9-7, 9-8, 9-9, and 9-10 were studied using potassium sulfite, potassium bisulfite, ammonium sulfite monohydrate, calcium sulfite hemihydrate, and sodium dithionite as capture agents, respectively, and it was confirmed that capturing DBF as FMSA or FMSA salt proceeded in all of the conditions.

Examples 9-11 and 9-12 were studied using an amide-based solvent DMA and an alcohol-based solvent MeOH as solvents, and it was confirmed that the conversion rate of DBF capture reaction was 99.1% and 99.9% or more, respectively.

Examples 9-13 and 9-14 were studied using DIPEA and 2,6-lutidine as bases to be added in DBF capturing conditions, and it was confirmed that the conversion rate of DBF capture reaction was 99.8% and 67.4%, respectively.

[Table 3]

| Example | Fmoc group-deprotection reaction conditions | Fmoc group-deprotection reaction conversion rate (%) | DBF capture conditions | DBF capture rate (%) |
|---|---|---|---|---|
| 9-1 | DBU (1 eq.)<br>MeCN (20 v/w)<br>room temperature, 1 h | 99.4% | $NaHSO_3$ (5.6 eq.)<br>$NEt_3$ (10 eq.)<br>water (100 eq.)<br>room temperature, 4 h | 99.9% or more |
| 9-2 | DBU (1 eq.)<br>MeCN (5 v/w)<br>room temperature, 0.5 h | 99.5% | $NaHSO_3$ (5.6 eq.)<br>water (10 eq.)<br>room temperature, 1 h | 15.9% |
| 9-3 | DBU (1 eq.)<br>MeCN (5 v/w)<br>room temperature, 0.5 h | 100% | NaHSOs (5.6 eq.)<br>water (10 eq.)<br>50°C, 6 h | 88.9% |
| 9-4 | DBU (1 eq.)<br>MeCN (5 v/w)<br>room temperature, 0.5 h | 100% | NaHSOs (5.6 eq.)<br>water (10 eq.)<br>80°C, 6 h | 100% |
| 9-5 | DBU (1 eq.)<br>MeCN (9 v/w)<br>25°C, 0.5 h | 100% | $K_2SO_3$ (2.5 eq.)<br>water (10 eq.)<br>25°C, 2 h | 47.0% |
| 9-6 | DBU (1 eq.)<br>MeCN (5 v/w)<br>room temperature, 0.5 h | 99.7% | $KHSO_3$ (2 eq.)<br>$NEt_3$ (4 eq.)<br>water (10 eq.)<br>room temperature, 3 h | 94.4% |
| 9-7 | DBU (1.0 eq.)<br>MeCN (5 v/w)<br>room temperature, 0.5 h | 99.6% | $(NH_4)_2SO_3 \cdot H_2O$ (2.0 eq.)<br>NEt3 (4.0 eq.)<br>water (10 eq.)<br>room temperature, 3 h | 52.3% |
| 9-8 | DBU (1.0 eq.)<br>MeCN (5 v/w)<br>room temperature, 0.5 h | 99.6% | $CaSO_3 \cdot 0.5H_2O$ (2.0 eq.)<br>$NEt_3$ (4.0 eq.)<br>water (4 v/w)<br>DMF (4 v/w)<br>room temperature, 2 h | 39.4% |
| 9-9 | DBU (1.0 eq.)<br>MeCN (5 v/w)<br>room temperature, 0.5 h | 100% | $CaSO_3 \cdot 0.5H_2O$ (2.0 eq.)<br>$NEt_3$ (4.0 eq.)<br>water (4 v/w)<br>DMF (4 v/w)<br>50°C, 2 h | 91.8% |
| 9-10 | DBU (1.0 eq.)<br>MeCN (5 v/w)<br>room temperature, 0.5 h | 99.7% | $Na_2S_2O_4$ (2.0 eq.)<br>$NEt_3$ (4.0 eq.)<br>water (10 eq.)<br>room temperature, 3 h | 98.7% |

(continued)

| Example | Fmoc group-deprotection reaction conditions | Fmoc group-deprotection reaction conversion rate (%) | DBF capture conditions | DBF capture rate (%) |
|---|---|---|---|---|
| 9-11 | DBU (1.0 eq.)<br>DMA (5 v/w)<br>room temperature, 0.5 h | 99.8% | $NaHSO_3$ (5.6 eq.)<br>$NEt_3$ (4.0 eq.)<br>water (10 eq.)<br>room temperature, 2.5 h | 99.1% |
| 9-12 | DBU (1.0 eq.)<br>MeOH (5 v/w) room temperature, 2 h<br>(15 h under DBF capture conditions) | 62.7%<br>(98.6%) | $NaHSO_3$ (5.6 eq.)<br>$Et_3$ (4.0 eq.)<br>water (10 eq.)<br>room temperature, 15 h | 99.9% or more |
| 9-13 | DBU (1.0 eq.)<br>MeCN (5 v/w)<br>room temperature, 0.5 h | 100% | $NaHSO_3$ (5.6 eq.)<br>DIPEA (4.0 eq.)<br>water (10 eq.)<br>room temperature, 1 h | 99.8% |
| 9-14 | DBU (1.0 eq.)<br>MeCN (5 v/w)<br>room temperature, 0.5 h | 100% | $NaHSO_3$ (5.7 eq.)<br>2,6-lutidine (4.0 eq.)<br>water (10 eq.)<br>room temperature, 19 h | 67.4% |

Example 10

Examination of washing conditions

[Formula 36]

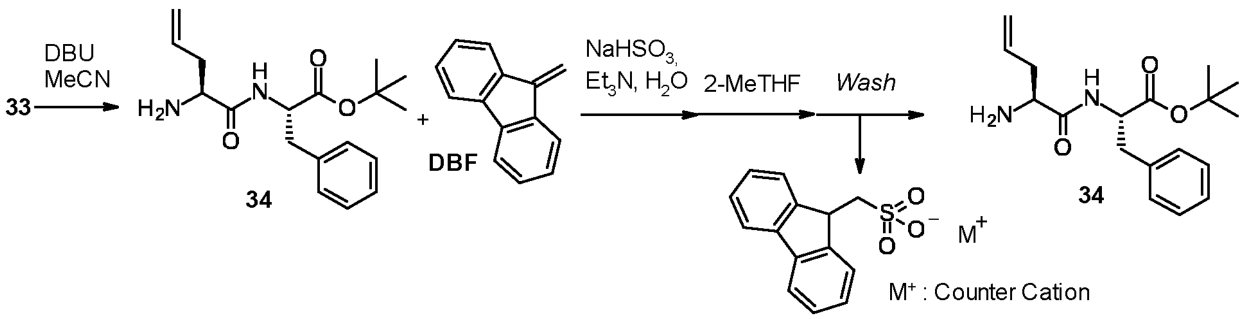


FMSA and/or its salt

Compound 33 (32.0 mg, content 79.4%, actual amount 25.4 mg) and MeCN (540 μL, 21 v/w) were added to the reaction vessel, and the reaction vessel was subjected to nitrogen replacement. DBU (7.0 μL, 1.0 eq.) was added at room temperature while stirring the reaction mixture, then the mixture was stirred at room temperature for 1 hour. The reaction mixture was sampled and prepared as a sample (sample preparation method 1). The Fmoc group-deprotection reaction was evaluated by HPLC analysis, and it was confirmed that the reaction conversion rate was 99.3% (calculation formula of Fmoc group-deprotection reaction conversion rate). Triethylamine (65.5 μL, 10 eq.), water (16.9 μL, 20 eq.), and sodium bisulfite (69.1 mg, 14 eq.) were then sequentially added to the reaction mixture at room temperature. After stirring for 3 hours, the reaction mixture was sampled and prepared as a sample (sample preparation method 1). The DBF capture reaction was evaluated by HPLC analysis, and it was confirmed that the reaction conversion rate was 99.8% (calculation formula 1 of DBF capture reaction conversion rate). The reaction solution was divided into three, and 2-MeTHF (300 μL) was added to each, and the reaction solution was washed under the conditions shown in the table below, and the FMSA or

FMSA salt residual rate was confirmed by HPLC analysis (calculation formula of FMSA or FMSA salt residual rate).

[Table 4]

| Example | Washing conditions | FMSA or FMSA salt residual rate (%) |
|---|---|---|
| | Before washing | 77.9% |
| 10-1 | 5% aqueous sodium carbonate solution (0.5 mL)<br>Number of washing 2 times | 65.9% |
| 10-2 | 5% aqueous potassium phosphate solution (0.5 mL)<br>Number of washing 2 times | 62.0% |
| 10-3 | 2.5% aqueous ammonia solution (0.5 mL)<br>Number of washing 2 times | 18.9% |

Example 11

Examples of using morpholine as deprotecting agent

[Formula 37]

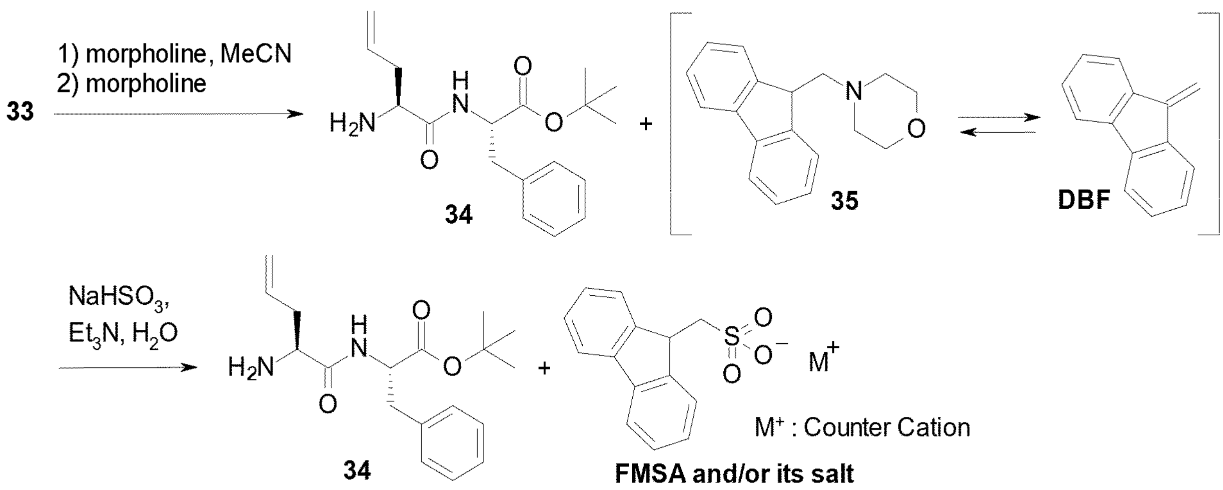

33
34
35
DBF
34
FMSA and/or its salt

Compound 33 (32.5 mg, content 79.4%, actual amount 25.8 mg) and MeCN (0.127 mL, 5 v/w) were added to the reaction vessel, and the reaction vessel was subjected to nitrogen replacement. Morpholine (20.2 μL, 5.0 eq.) was added at room temperature while stirring the reaction mixture, then the mixture was stirred at room temperature for 30 minutes. Then, morpholine (20.2 μL, 5.0 eq.) was added at room temperature while the reaction mixture was stirred, and the mixture was stirred for another 1 hour. The reaction mixture was sampled and prepared as a sample (sample preparation method 1). The Fmoc group-deprotection reaction was evaluated by HPLC analysis, and it was confirmed that the reaction conversion rate was 91.8% (calculation formula of Fmoc group-deprotection reaction conversion rate). At this time, DBF and a morpholine adduct of DBF, compound 35, were observed, and the ratio of DBF to compound 35 was 6.5 : 93.5. Then, triethylamine (26.1 μL, 4.0 eq.), water (8.4 μL, 10 eq.), and sodium bisulfite (27.5 mg, 5.6 eq.) were sequentially added to the reaction mixture at room temperature. After stirring for 15 hours, the reaction mixture was sampled and prepared as a sample (sample preparation method 1). The Fmoc group-deprotection reaction and DBF capture reaction were evaluated by HPLC analysis and it was confirmed that the reaction conversion rate of Fmoc group-deprotection reaction was 100% and the reaction conversion rate of DBF capture reaction was 15.6% (calculation formula of Fmoc group-deprotection reaction conversion rate, and calculation formula 2 of DBF capture reaction conversion rate). The external temperature of the reaction vessel was warmed to 50°C. After stirring for 2 hours, the reaction mixture was sampled and prepared as a sample (sample preparation method 1). The DBF capture reaction was evaluated by HPLC analysis, and it was confirmed that the reaction conversion rate was 44.3% (calculation formula 2 of DBF capture reaction conversion rate). Further, the external temperature of the reaction vessel was warmed to 78°C, and after stirred for 2 hours, the reaction mixture was

sampled and prepared as a sample (sample preparation method 1). The DBF capture reaction was evaluated by HPLC analysis, and it was confirmed that the reaction conversion rate was 95.7% (calculation formula 2 of DBF capture reaction conversion rate).

Example 12

Examples of using primary or secondary amine as deprotecting agent

Using compound 33, Fmoc group-deprotection reaction and subsequent DBF capture reaction were carried out using amines shown in the table below in the same deprotection reaction of the Fmoc group as in Examples 11, and the Fmoc group-deprotection rate and the DBF capture rate were measured (calculation formula of Fmoc group-deprotection reaction conversion rate, calculation formula 1 of DBF capture reaction conversion rate).

In Examples 12-1, 12-2, 12-3, and 12-4, no adduct compounds of corresponding amine to DBF were observed unlike the study of Example 11 where morpholine was added, but it was confirmed that DBF was captured as FMSA or an FMSA salt. It was also confirmed that the deprotection reaction of the Fmoc group proceeds after the reaction mixture being subjected to the capture condition of DBF, and the deprotection reaction and the DBF capture reaction proceed simultaneously.

[Table 5]

| Example | Fmoc group-deprotection reaction conditions | Fmoc group-deprotection reaction conversion rate (%) | DBF capture conditions | Fmoc group-deprotection reaction conversion rate (%) | DBF capture rate (%) |
|---|---|---|---|---|---|
| 12-1 | $H_2N^nPr$ (2 eq.)<br>MeCN (5 v/w)<br>room temperature, 1 h | 16.5% | $NaHSO_3$ (5.6 eq.)<br>$NEt_3$ (4 eq.)<br>water (10 eq.)<br>room temperature, 2 h<br>Then, 80°C, 1 h | 47.2%<br>100% | 98.9%<br>100% |
| 12-2 | $HNEt_2$ (2 eq.)<br>MeCN (5 v/w)<br>room temperature, 1 h | 69.0% | $NaHSO_3$ (5.6 eq.)<br>$NEt_3$ (4 eq.)<br>water (10 eq.)<br>room temperature, 2 h | 97.9% | 100% |
| 12-3 | $HNCy_2$ (2 eq.)<br>MeCN (5 v/w)<br>room temperature, 1 h | 7.3% | NaHSOs (5.6 eq.)<br>$NEt_3$ (4 eq.)<br>water (10 eq.)<br>room temperature, 2 h | 52.4% | 99.6% |
| | | | Then, 80°C, 1 h | 100% | 100% |
| 12-4 | HMDS (2 eq.)<br>MeCN (5 v/w)<br>room temperature, 1 h | Not detected | NaHSOs (5.6 eq.)<br>$NEt_3$ (4 eq.)<br>water (10 eq.)<br>room temperature, 2 h | 16.8% | 94.6% |
| | | | Then, 80°C, 1 h | 99.7% | 99.4% |

Example 13

Examples of simultaneous deprotection and DBF capture

[Formula 38]

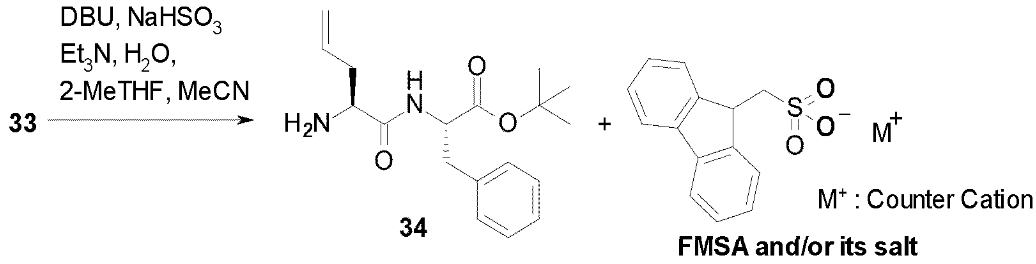


Compound 33 (32.0 mg, content 79.4%, actual amount 25.4 mg), MeCN (110 μL, 4.3 v/w), and 2-MeTHF (110 μL, 4.3 v/w) were added to the reaction vessel, and the reaction vessel was subjected to nitrogen replacement. To the reaction mixture with stirring, triethylamine (65.2 μL, 10 eq.), water (8.4 μL, 10 eq.), sodium bisulfite (16.5 mg, 3.4 eq.), and DBU (20.9 μL, 3.0 eq.) were sequentially added at room temperature, and the mixture was stirred for 1 hour. The reaction mixture was sampled and prepared as a sample (sample preparation method 1). It was confirmed by HPLC analysis that the reaction conversion rate of Fmoc group-deprotection reaction was 100% (calculation formula of Fmoc group-deprotection reaction conversion rate) and the reaction conversion rate of dibenzofulvene capture reaction was 99.9% or more (calculation formula 1 of DBF capture reaction conversion rate).

Example 14

Examination of reaction conditions

Using compound 33, Fmoc group-deprotection and DBF capture were carried out at the same time under the reaction conditions shown in the table bdelow in the same procedure as in Examples 13, and the Fmoc group-deprotection rate and the DBF capture rate were measured (calculation formula of Fmoc group-deprotection reaction conversion rate, calculation formula 1 of DBF capture reaction conversion rate).

In Examples 14-1, 14-2, 14-3, and 14-4 where the reaction was performed with a MeOH solvent, the deprotection reaction and the DBF capture reaction proceeded at 25°C regardless of the presence or absence of water, and the completion of the deprotection reaction and the DBF capture reaction was confirmed in 1 hour when heated to 50°C.

In Example 14-5 where a DMI solvent was used, the deprotection reaction and the DBF capture reaction were completed in 1 hour even at a reaction temperature of 25°C. In Example 14-6 where the reaction was performed under conditions of not adding of water in the presence of a DMI solvent, a delay in the DBF capture reaction was confirmed. In Example 14-17 where the reaction was performed under the conditions of not adding triethylamine, it was confirmed that the deprotection reaction and the DBF capture reaction were completed at 50°C for 1 hour.

In Examples 14-8 and 14-9, a DMSO solvent was used, and both Examples were carried out under the conditions of not adding triethylamine. In Example 14-8 where water was added, the deprotection reaction and the DBF capture reaction were completed in 1 hour, and also in Example 14-9 where water was not added, the completion of both reactions was confirmed by extending the reaction time to 4 hours.

[Table 6]

| Example | Reaction conditions | Fmoc group-deprotection reaction conversion rate (%) | DBF capture rate (%) |
|---|---|---|---|
| 14-1 | DBU (3 eq.)<br>$NaHSO_3$ (3.4 eq.)<br>$NEt_3$ (10 eq.)<br>water (1 v/w)<br>MeOH (4 v/w)<br>room temperature, 6 h | 100% | 100% |
| 14-2 | DBU (3 eq.)<br>$NaHSO_3$ (3.4 eq.)<br>$NEt_3$ (10 eq.)<br>water (1 v/w)<br>MeOH (4 v/w)<br>50°C, 1 h | 100% | 100% |

(continued)

| Example | Reaction conditions | Fmoc group-deprotection reaction conversion rate (%) | DBF capture rate (%) |
|---|---|---|---|
| 14-3 | DBU (3 eq.)<br>$NaHSO_3$ (3.4 eq.)<br>$NEt_3$ (10 eq.)<br>MeOH (4 v/w)<br>room temperature, 6 h | 100% | 98.4% |
| 14-4 | DBU (3 eq.)<br>$NaHSO_3$ (3.4 eq.)<br>$NEt_3$ (10 eq.)<br>MeOH (4 v/w)<br>50°C, 4 h | 100% | 100% |
| 14-5 | DBU (3 eq.)<br>$NaHSO_3$ (3.4 eq.)<br>$NEt_3$ (10 eq.)<br>water (1 v/w)<br>DMI (4 v/w)<br>25°C, 1 h | 100% | 100% |
| 14-6 | DBU (3 eq.)<br>$NaHSO_3$ (3.4 eq.)<br>$NEt_3$ (10 eq.)<br>DMI (4 v/w)<br>50°C, 6 h | 100% | 32.5% |
| 14-7 | DBU (3 eq.)<br>$NaHSO_3$ (3.4 eq.)<br>water (1 v/w)<br>DMI (4 v/w)<br>50°C, 1 h | 100% | 100% |
| 14-8 | DBU (3 eq.)<br>$NaHSO_3$ (3.4 eq.)<br>water (1 v/w)<br>DMSO (4 v/w)<br>50°C, 1 h | 100% | 100% |
| 14-9 | DBU (3 eq.)<br>$NaHSO_3$ (3.4 eq.)<br>DMSO (4 v/w)<br>50°C, 4 h | 100% | 100% |

Example 15

Confirmation of presence or absence of regenerated DBF during wash removal with base when using sodium bisulfite as capture agent

[Formula 39]

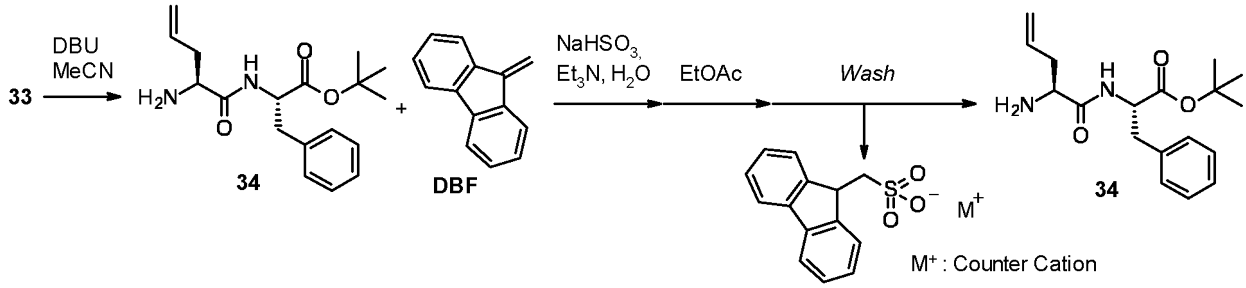


33
34
DBF
34
FMSA and/or its salt

Compound 33 (32.0 mg, content 79.4%, actual amount 25.4 mg) and MeCN (540 μL, 21 v/w) were added to the reaction vessel, and the reaction vessel was subjected to nitrogen replacement. DBU (7.0 μL, 1.0 eq.) was added at room temperature while stirring the reaction mixture, then the mixture was stirred at room temperature for 1 hour. The reaction mixture was sampled and prepared as a sample (sample preparation method 1). The Fmoc group-deprotection reaction was evaluated by HPLC analysis, and it was confirmed that the reaction conversion rate was 99.4% (calculation formula of Fmoc group-deprotection reaction conversion rate). Triethylamine (65.5 μL, 10 eq.), water (85 μL, 100 eq.), and sodium bisulfite (27.6 mg, 5.6 eq.) were then sequentially added to the reaction mixture at room temperature. After stirring for 4 hours, the reaction mixture was sampled and prepared as a sample (sample preparation method 1). The DBF capture reaction was evaluated by HPLC analysis, and it was confirmed that the reaction conversion rate was 99.9% or more (calculation formula 1 of DBF capture reaction conversion rate). Ethyl acetate (0.5 mL) was added to the reaction mixture, then the mixture was washed four times with 2.5% aqueous ammonia solution (0.5 mL). Almost no presence of compound 34 was confirmed in each aqueous layer after washing, then the area ratio of FMSA or FMSA Salt to compound 34 and the area ratio of DBF to compound 34 were measured by HPLC analysis using compound 34 as an internal standard. The results are shown in Table 7. The area ratio of FMSA or FMSA salt gradually decreased as the number of washes increased, while there was no change in the area ratio of DBF, which confirmed that there was no regeneration of DBF from the FMSA or FMSA salt.

[Table 7]

| Washing conditions | Number of washing | LC area ratio FMSA or FMSA salt:compound 34 | LC area ratio DBF:compound 34 |
|---|---|---|---|
| 2.5% aqueous ammonia solution (0.5 mL) | Before washing | 79.7:20.3 | 0:100 |
| | 1 time | 78.3:21.7 | 0:100 |
| | 2 times | 64.0:36.0 | 0:100 |
| | 3 times | 37.3:62.7 | 0:100 |
| | 4 times | 12.1:87.9 | 0:100 |

Comparative Example 1

Confirmation of presence or absence of regenerated DBF during wash removal with base when using sodium 2-mercaptoethanesulfonate as capture agent

[Formula 40]

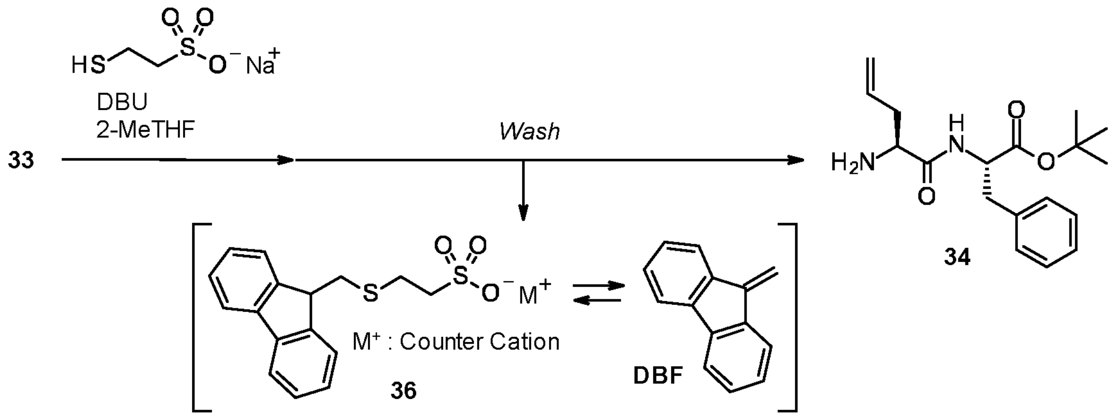


Compound 33 (60.0 mg, content 79.4%, actual amount 47.6 mg), sodium 2-mercaptoethanesulfonate(21.6 mg, 1.5 eq.), and 2-MeTHF(1.1 mL, 23 v/w) were added to the reaction vessel, and the reaction vessel was subjected to nitrogen replacement. DBU (13.1 μL, 1.0 eq.) was added at room temperature while stirring the reaction mixture, then the mixture was stirred at the external temperature of 50°C for 3 hours. The reaction mixture was sampled and prepared as a sample (sample preparation method 1). It was confirmed by HPLC analysis that the reaction conversion rate of Fmoc group-deprotection reaction was 100% (calculation formula of Fmoc group-deprotection reaction conversion rate) and the reaction conversion rate of DBF capture reaction was 84.8% (calculation formula 3 of DBF capture reaction conversion rate). The reaction mixture was then washed four times with 5% aqueous tripotassium phosphate solution (1 mL). Almost no presence of compound 34 was confirmed in each aqueous layer after washing, then the area ratio of compound 36 to compound 34 and the area ratio of DBF to compound 34 were measured by HPLC analysis using compound 34 as an internal standard. The results are shown in Table 8. As the number of washes increased, the area ratio of compound 36 gradually decreased while the area ratio of DBF gradually increased, which confirmed that DBF is regenerated from compound 36.

[Table 8]

| Washing conditions | Number of washing | LC area ratio compound 36:compound 34 | LC area ratio DBF:compound 34 |
|---|---|---|---|
| 5% aqueous tripotassium phosphate solution (1 mL) | Before washing | 72.1:27.9 | 31.6:68.4 |
| | 1 time | 43.4:56.6 | 39.3:60.7 |
| | 2 times | 24.9:75.1 | 48.2:51.8 |
| | 3 times | 11.1:88.9 | 48.3:51.7 |
| | 4 times | 3.5:96.5 | 48.7:51.3 |

Comparative Example 2

Confirmation of presence or absence of regenerated DBF during wash removal with base when using sodium 2-mercaptoethanesulfonate as capture agent

[Formula 41]

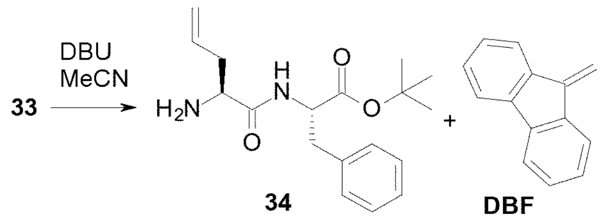


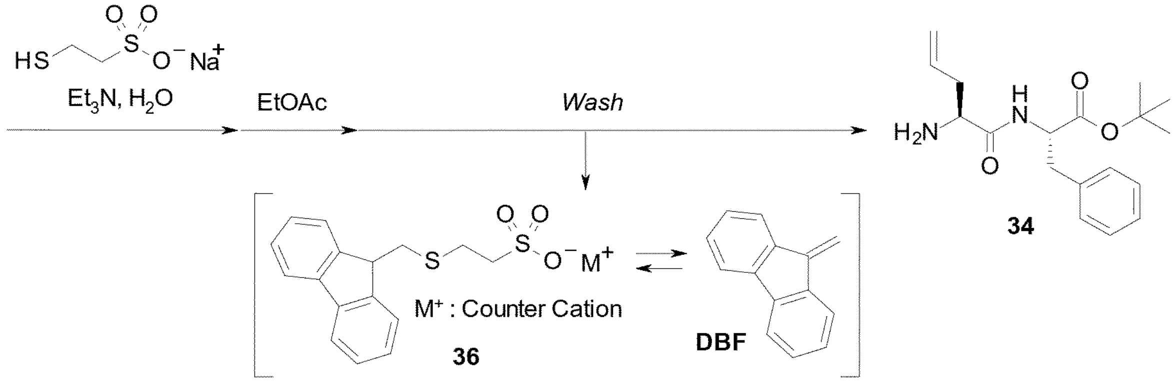


Compound 33 (94.0 mg, content 92.9%, actual amount 87.3 mg) and MeCN (2.0 mL, 21 v/w) were added to the reaction vessel, and the reaction vessel was subjected to nitrogen replacement. DBU (25.9 μL, 1.0 eq.) was added at room temperature while stirring the reaction mixture, then the mixture was stirred at room temperature for 1 hour. The reaction mixture was sampled and prepared as a sample (sample preparation method 1). The Fmoc group-deprotection reaction was evaluated by HPLC analysis, and it was confirmed that the reaction was completed (calculation formula of Fmoc group-deprotection reaction conversion rate). Triethylamine (240 μL, 10 eq.), water (310 μL, 100 eq.), and sodium 2-mercaptoethanesulfonate (160 mg, 5.6 eq.) were then sequentially added to the reaction mixture at room temperature. After stirring for 1 hour, the reaction mixture was sampled and prepared as a sample (sample preparation method 1). The DBF capture reaction was evaluated by HPLC analysis, and it was confirmed that the reaction conversion rate was 100% (calculation formula 3 of DBF capture reaction conversion rate). Ethyl acetate (2.0 mL) was added to the reaction mixture, then the mixture was washed four times with 2.5% aqueous ammonia solution (2.0 mL). Almost no presence of compound 34 was confirmed in each aqueous layer after washing, then the area ratio of FMSA or FMSA salt to compound 34 and the area ratio of DBF to compound 34 were measured by HPLC analysis using compound 34 as an internal standard. The results are shown in Table 9. As the number of washes increased, the area ratio of compound 36 gradually decreased while the area ratio of DBF gradually increased, which confirmed that DBF is regenerated from compound 36.

[Table 9]

| Washing conditions | Number of washing | LC area ratio compound 36:compound 34 | LC area ratio DBF:compound 34 |
|---|---|---|---|
| 2.5% aqueous ammonia sloution (2 mL) | Before washing | 80.5:19.5 | 0:100 |
| | 1 time | 79.1:20.9 | 0:100 |
| | 2 times | 66.1:33.9 | 3.3:96.7 |
| | 3 times | 38.6:61.4 | 4.4:95.6 |
| | 4 times | 11.7:88.3 | 5.2:94.8 |

Comparative Example 3

Confirmation of presence or absence of regenerated DBF during wash removal with base when using (3-mercaptopropyl)phosphonic acid as capture agent

[Formula 42]

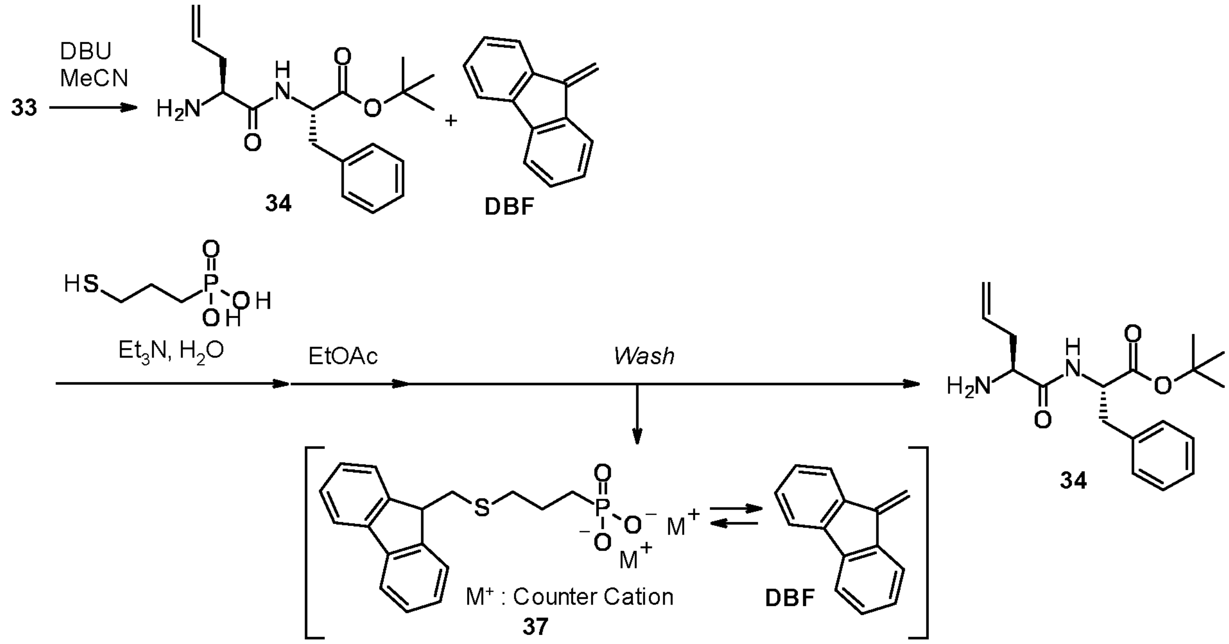


Compound 33 (29.1 mg, content 92.9%, actual amount 27.0 mg) and MeCN (611 μL, 21 v/w) were added to the reaction vessel, and the reaction vessel was subjected to nitrogen replacement. DBU (7.48 μL, 1.0 eq.) was added at room temperature while stirring the reaction mixture, then the mixture was stirred at room temperature for 30 minutes. The reaction mixture was sampled and prepared as a sample (sample preparation method 1). The Fmoc group-deprotection reaction was evaluated by HPLC analysis, and it was confirmed that the reaction was completed (calculation formula of Fmoc group-deprotection reaction conversion rate). Triethylamine (69.7 μL, 10 eq.), water (90 μL, 100 eq.), and (3-mercaptopropyl)phosphonic acid (43.7 mg, 5.6 eq.) synthesized according to the method of Synthesis Example 1 described in Patent Literature 4 were then sequentially added to the reaction mixture at room temperature. After the mixture was stirred for 2 hours, (3-mercaptopropyl)phosphonic acid (43.7 mg, 5.6 eq.) and triethylamine (69.7 μL, 10 eq.) were added. After the mixture was stirred for 1 hour and 30 minutes, triethylamine (69.7 μL, 10 eq.) was further added. After stirring for another 1 hour, the reaction mixture was sampled and prepared as a sample (sample preparation method 1). The DBF capture reaction was evaluated by HPLC analysis, and it was confirmed that the reaction conversion rate was 97.6% (calculation formula 4 of DBF capture reaction conversion rate). Ethyl acetate (0.6 mL) was added to the reaction mixture, then the mixture was washed four times with 2.5% aqueous ammonia solution (0.6 mL). Almost no presence of compound 34 was confirmed in each aqueous layer after washing, then the area ratio of compound 37 to compound 34 and the area ratio of DBF to compound 34 were measured by HPLC analysis using compound 34 as an internal standard. The results are shown in Table 10. As the number of washes increased, the area ratio of compound 37 gradually decreased while the area ratio of DBF gradually increased, which confirmed that DBF is regenerated from compound 37.

[Table 10]

| Washing conditions | Number of washing | LC area ratio compound 37:compound 34 | LC area ratio DBF:compound 34 |
|---|---|---|---|
| 2.5% aqueous ammonia solution (1 mL) | Before washing | 52.1:47.9 | 2.6:97.4 |
| | 1 time | 44.6:55.4 | 4.1:95.9 |
| | 2 times | 27.3:72.7 | 7.2:92.8 |
| | 3 times | 18.8:81.2 | 7.7:92.3 |
| | 4 times | 10.1:89.9 | 8.4:91.6 |

Industrial Applicability

The present invention provides a new method for removing dibenzofulvene, the method being capable of

capturing dibenzofulvene generated in a step of deprotecting a protecting group having a Fmoc skeleton, and removing dibenzofulvene without regeneration.

## Claims

1. A method for removing dibenzofulvene or a dibenzofulvene derivative, comprising the following step (1), (1'), or (1"):

   (1) mixing the following (i) and (ii):

      (i) dibenzofulvene or a dibenzofulvene derivative;
      (ii) a sulfite ion or a bisulfite ion or a compound that produces a sulfite ion or a bisulfite ion,

   (1') mixing the following (i) and (ii):

      (i) dibenzofulvene or a dibenzofulvene derivative;
      (ii) at least one selected from the group consisting of bisulfite, disulfurous acid and a salt thereof, sulfurous acid and a salt thereof, dithionous acid and a salt thereof, and a solvate of these,

   (1") forming (9H-fluoren-9-yl)methanesulfonic acid or a salt thereof or a (9H-fluoren-9-yl)methanesulfonic acid derivative or a salt thereof.

2. The method according to claim 1, wherein, in the step (1) or (1'), (9H-fluoren-9-yl)methanesulfonic acid or a salt thereof or a (9H-fluoren-9-yl)methanesulfonic acid derivative or a salt thereof is formed.

3. The method according to claim 1 or 2, comprising, before the step (1), (1') or (1"), step (2) of mixing a first amino group-containing compound in which an amino group is protected by a protecting group having a Fmoc skeleton with a deprotecting agent capable of deprotecting a protecting group having a Fmoc skeleton.

4. A method for removing dibenzofulvene or a dibenzofulvene derivative, comprising the following step (3) or (3'):

   (3) mixing the following (i) to (iii):

      (i) a first amino group-containing compound in which an amino group is protected by a protecting group having a Fmoc skeleton;
      (ii) a deprotecting agent capable of deprotecting a protecting group having a Fmoc skeleton;
      (iii) a sulfite ion or a bisulfite ion or a compound that produces a sulfite ion or a bisulfite ion,

   (3') mixing the following (i) to (iii):

      (i) a first amino group-containing compound in which an amino group is protected by a protecting group having a Fmoc skeleton;
      (ii) a deprotecting agent capable of deprotecting a protecting group having a Fmoc skeleton;
      (iii) at least one selected from the group consisting of bisulfite, disulfurous acid and a salt thereof, sulfurous acid and a salt thereof, dithionous acid and a salt thereof, and a solvate of these.

5. A method for deprotecting a protecting group having a Fmoc skeleton, comprising the following step (3) or (3'):

   (3) mixing the following (i) to (iii):

      (i) a first amino group-containing compound in which an amino group is protected by a protecting group having a Fmoc skeleton;
      (ii) a deprotecting agent capable of deprotecting a protecting group having a Fmoc skeleton;
      (iii) a sulfite ion or a bisulfite ion or a compound that produces a sulfite ion or a bisulfite ion,

   (3') mixing the following (i) to (iii):

      (i) a first amino group-containing compound in which an amino group is protected by a protecting group

having a Fmoc skeleton;
(ii) a deprotecting agent capable of deprotecting a protecting group having a Fmoc skeleton;
(iii) at least one selected from the group consisting of bisulfite, disulfurous acid and a salt thereof, sulfurous acid and a salt thereof, dithionous acid and a salt thereof, and a solvate of these.

**6.** The method according to claim 4 or 5, wherein, in the step (3) or (3'), (9H-fluoren-9-yl)methanesulfonic acid or a salt thereof or a (9H-fluoren-9-yl)methanesulfonic acid derivative or a salt thereof is formed.

**7.** The method according to any one of claims 4 to 6, wherein dibenzofulvene or a dibenzofulvene derivative generated by the step of mixing a first amino group-containing compound in which an amino group is protected by a protecting group having a Fmoc skeleton with a deprotecting agent capable of deprotecting a protecting group having a Fmoc skeleton is removed from a mixture generated by the step (3) or (3').

**8.** The method according to any one of claims 1 to 7, further comprising step (4) of washing a mixture after one or more steps of the step (1), (1'), (1"), (3), or (3') with a washing solution.

**9.** The method according to any one of claims 1 to 8, wherein an additive is further mixed in one or more steps of the step (1), (1'), (1"), (3), or (3').

**10.** The method according to any one of claims 3 to 9, wherein the deprotecting agent capable of deprotecting a protecting group having a Fmoc skeleton is a second base.

**11.** A method for producing a first compound, comprising the method according to any one of claims 1 to 10.

**12.** The production method according to claim 11, wherein the first compound is an amino acid or a peptide.

**13.** A method for producing a peptide compound, comprising the following steps of:

1) treating a protecting-group peptide protected by a protecting group having a Fmoc skeleton with a deprotecting agent capable of deprotecting a protecting group having a Fmoc skeleton in the presence of a sulfite ion or a bisulfite ion to obtain a deprotected peptide in which the protecting group is removed; and
2) optionally extending the deprotected peptide with one or more amino acids to obtain an extended peptide.

**14.** Use of the following (a) or (b) for removing dibenzofulvene or a dibenzofulvene derivative:

(a) a sulfite ion or a bisulfite ion or a compound that produces a sulfite ion or a bisulfite ion, and/or
(b) at least one compound selected from the group consisting of bisulfite, disulfurous acid and a salt thereof, sulfurous acid and a salt thereof, dithionous acid and a salt thereof, and a solvate of these.

**15.** Combination use of the following (a) and/or (b) with a deprotecting agent capable of deprotecting a protecting group having a Fmoc skeleton, for deprotecting a protecting group having a Fmoc skeleton:

(a) a sulfite ion or a bisulfite ion or a compound that produces a sulfite ion or a bisulfite ion,
(b) at least one compound selected from the group consisting of bisulfite, disulfurous acid and a salt thereof, sulfurous acid and a salt thereof, dithionous acid and a salt thereof, and a solvate of these.

**16.** A composition comprising an amino group-containing compound, and (9H-fluoren-9-yl)methanesulfonic acid or a salt thereof or a (9H-fluoren-9-yl)methanesulfonic acid derivative or a salt thereof, wherein a content ratio of the (9H-fluoren-9-yl)methanesulfonic acid or a salt thereof or the (9H-fluoren-9-yl)methanesulfonic acid derivative or a salt thereof to the amino group-containing compound, and the (9H-fluoren-9-yl)methanesulfonic acid or a salt thereof or the (9H-fluoren-9-yl)methanesulfonic acid derivative or a salt thereof is 0.01 or less.

## INTERNATIONAL SEARCH REPORT

International application No.
**PCT/JP2023/039324**

**A. CLASSIFICATION OF SUBJECT MATTER**

***C07K 1/06***(2006.01)i
FI: C07K1/06

According to International Patent Classification (IPC) or to both national classification and IPC

**B. FIELDS SEARCHED**

Minimum documentation searched (classification system followed by classification symbols)

C07K1/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 7063409 B1 (PEPTISTAR INC.) 25 April 2022 (2022-04-25)<br>claims, paragraphs [0006], [0011], tables 1-4 | 1-16 |
| A | WO 2010/016551 A1 (AJINOMOTO CO., INC.) 11 February 2010 (2010-02-11)<br>claims, paragraphs [0008], [0011], tables 1-4 | 1-16 |
| A | WO 09/014177 A1 (AJINOMOTO CO., INC.) 29 January 2009 (2009-01-29)<br>claims, paragraphs [0008], [0010], examples 1-8 | 1-16 |
| A | LEGGIO, A. et al., Lewis acid catalyzed methylation of N-(9H-fluoren-9-yl)methanesulfonyl(Fms) protected lipophilic α-amino acid methyl esters, Journal of Peptide Science, 2015, vol. 21, no. 8, pp. 644-650<br>abstract, schemes 1, 3 | 1-16 |
| A | ISHIBASHI, Y. et al., (9H-Fluoren-9-yl)methanesulfonyl (Fms): An Amino Protecting Group Complementary to Fmoc, European Journal of Organic Chemistry, 2010, vol. 22, pp. 4201-4204<br>Abstract | 1-16 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 December 2023** | **19 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**
**Information on patent family members**

International application No.
**PCT/JP2023/039324**

| Patent document cited in search report | Publication date (day/month/year) | Patent family member(s) | Publication date (day/month/year) |
|---|---|---|---|
| JP 7063409 B1 | 25 April 2022 | (Family: none) | |
| WO 2010/016551 A1 | 11 February 2010 | US 2011/0190475 A1<br>claims, paragraphs [0012], [0050], [0051], examples 1-4<br>EP 2322498 A1 | |
| WO 09/014177 A1 | 29 January 2009 | US 2010/0184952 A1<br>claims, paragraphs [0012], [0030], [0031], examples 1-8<br>EP 2181983 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009014177 A
- WO 2010016551 A
- WO 2013089241 A
- JP 7063409 B
- WO 5113118 A
- WO 5929756 A
- WO 6092513 A
- WO 5768712 A
- WO 5803674 A
- WO 6116782 A
- WO 6201076 A
- WO 6283774 A
- WO 6283775 A
- WO 6322350 A
- WO 6393857 A
- WO 6531235 A
- WO 2020175472 A
- WO 2020175473 A
- JP 2022175592 A
- WO 2013100132 A
- WO 2018225851 A
- WO 2018225864 A
- WO 2019117274 A
- WO 2020111238 A
- WO 2020122182 A
- WO 2021075478 A
- WO 2021090856 A
- WO 2021132545 A
- WO 2021246471 A
- WO 2022097540 A
- WO 2022138891 A
- WO 2022145444 A
- WO 2022234864 A
- WO 2023127869 A


### Non-patent literature cited in the description


- **J. E. SHEPPECK II et al.** *Tetrahedron Lett.*, 2000, vol. 28 (41), 5329-5333
- *CHEMICAL ABSTRACTS*, 148893-10-1
- *CHEMICAL ABSTRACTS*, 68957-94-8
- *CHEMICAL ABSTRACTS*, 94790-37-1
- *CHEMICAL ABSTRACTS*, 1075198-30-9
- *CHEMICAL ABSTRACTS*, 878-23-9
- *CHEMICAL ABSTRACTS*, 128625-52-5
- *CHEMICAL ABSTRACTS*, 3945-69-5
- *CHEMICAL ABSTRACTS*, 153433-21-7
- *CHEMICAL ABSTRACTS*, 873798-09-5
- *CHEMICAL ABSTRACTS*, 125700-67-6
- *CHEMICAL ABSTRACTS*, 136849-72-4
- *CHEMICAL ABSTRACTS*, 1892-57-5
- *CHEMICAL ABSTRACTS*, 693-13-0
- *CHEMICAL ABSTRACTS*, 538-75-0
- Amino Acids. Bachem Holding AG, 2018, vol. 50, 39-68
- *Amino Acids, Peptides and Proteins in Organic Chemistry: Building Blocks, Catalysis and Coupling Chemistry*, 2011, vol. 3
- *J. Peptide Res.*, 2005, vol. 65, 153-166
- *Org. Process Res. Dev.*, 2018, vol. 22, 760-772
- Fundamentals and experiments of peptide synthesis. Maruzen, 1985